(19) Europäisches Patentamt
European Patent Office
Office européen des brevets

(11) EP 2 054 516 B1

(12) **EUROPEAN PATENT SPECIFICATION**

(45) Date of publication and mention of the grant of the patent:
**08.01.2014 Bulletin 2014/02**

(21) Application number: **07788181.1**

(22) Date of filing: **02.08.2007**

(51) Int Cl.:
***C12N 15/82** (2006.01)* ***A01H 1/00** (2006.01)*
***C07K 14/415** (2006.01)*

(86) International application number:
**PCT/EP2007/058043**

(87) International publication number:
**WO 2008/015263 (07.02.2008 Gazette 2008/06)**

(54) **PLANTS HAVING IMPROVED CHARACTERISTICS AND A METHOD FOR MAKING THE SAME**

PFLANZEN MIT VERBESSERTEN EIGENSCHAFTEN SOWIE VERFAHREN ZUR HERSTELLUNG DAVON

INSTALLATIONS DOTÉES DE CARACTÉRISTIQUES DE RENDEMENT AMÉLIORÉES ET PROCÉDÉ DE FABRICATION DE CELLES-CI

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**02.08.2006 EP 06118347**
**10.08.2006 US 836804 P**
**20.09.2006 EP 06120994**
**05.10.2006 EP 06121856**
**06.10.2006 EP 06121928**
**12.10.2006 US 851265 P**
**12.10.2006 US 851258 P**
**12.10.2006 US 851250 P**
**27.10.2006 EP 06123066**
**31.10.2006 EP 06123237**
**17.11.2006 US 859717 P**
**01.12.2006 US 868095 P**

(43) Date of publication of application:
**06.05.2009 Bulletin 2009/19**

(60) Divisional application:
**09178579.0 / 2 199 394**
**09178586.5 / 2 199 395**
**09178588.1 / 2 199 396**
**09178590.7 / 2 189 533**
**09178866.1 / 2 189 534**
**12185022.6 / 2 543 732**
**12185025.9 / 2 546 262**
**12185028.3 / 2 540 832**
**12185029.1 / 2 543 733**

(73) Proprietor: **CropDesign N.V.**
**9052 Zwijnaarde (BE)**

(72) Inventors:
- **FRANKARD, Valerie**
  **1410 Waterloo (BE)**
- **MIRONOV, Vladimir**
  **9000 Gent (BE)**
- **REUZEAU, Christophe**
  **24350 La Chapelle Gonaguet (FR)**
- **SANZ MOLINERO, Ana Isabel**
  **9050 Gentbrugge (BE)**

(74) Representative: **Drohmann, Christian et al**
**BASF SE**
**Global Intellectual Property**
**GVX/A - C6**
**67056 Ludwigshafen (DE)**

(56) References cited:
**WO-A-2006/008271** **US-A1- 2004 034 888**
**US-A1- 2004 045 049** **US-A1- 2004 072 289**

- **DATABASE EMBL [Online] 29 April 2002 (2002-04-29), "Arabidopsis thaliana At2g41900/T6D20.20 mRNA, complete cds." XP002445984 retrieved from EBI accession no. EMBL:AY093957 Database accession no. AY093957**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 054 516 B1

- BECERRA C ET AL: "Ankyrin repeat-containing proteins in Arabidopsis: characterization of a novel and abundant group of genes coding ankyrin-transmembrane proteins" GENE: AN INTERNATIONAL JOURNAL ON GENES AND GENOMES, ELSEVIER, AMSTERDAM, NL, vol. 340, no. 1, 29 September 2004 (2004-09-29), pages 111-121, XP004649119 ISSN: 0378-1119
- BROWN R S: "Zinc finger proteins: getting a grip on RNA" CURRENT OPINION IN STRUCTURAL BIOLOGY, CURRENT OPINION IN STRUCTURAL BIOLOGY, GB, vol. 15, no. 1, February 2005 (2005-02), pages 94-98, XP004755671 ISSN: 0959-440X
- KURIYAMA H ET AL: "Characterization and chromosomal mapping of a novel human gene, ANKHZN" GENE, ELSEVIER, AMSTERDAM, NL, vol. 253, no. 2, 8 August 2000 (2000-08-08), pages 151-160, XP004215718 ISSN: 0378-1119
- BORK PEER: "Hundreds of ankyrin-like repeats in functionally diverse proteins: Mobile modules that cross phyla horizontally?" PROTEINS STRUCTURE FUNCTION AND GENETICS, vol. 17, no. 4, 1993, pages 363-374, XP009088008 ISSN: 0887-3585
- LI ZHONGSEN ET AL: "PEI1, an embryo-specific zinc finger protein gene required for heart-stage embryo formation in arabidopsis" PLANT CELL, AMERICAN SOCIETY OF PLANT PHYSIOLOGISTS, ROCKVILLE, MD, US, vol. 10, no. 3, March 1998 (1998-03), pages 383-398, XP002302043 ISSN: 1040-4651 cited in the application
- SCHENA M ET AL: "THE HAT4 GENE OF ARABIDOPSIS ENCODES A DEVELOPMENTAL REGULATOR" GENES AND DEVELOPMENT, COLD SPRING HARBOR LABORATORY PRESS, PLAINVIEW, NY, US, vol. 7, no. 3, 1993, pages 367-379, XP009033912 ISSN: 0890-9369

## Description

The present invention relates generally to the field of molecular biology and concerns a method for improving various plant characteristics by modulating expression in a plant of a nucleic acid encoding a GRP (Growth Related Protein). The present invention also concerns plants having modulated expression of a nucleic acid encoding a GRP polypeptide, which plants have improved characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various characteristics may be improved in plants by modulating expression in a plant of a nucleic acid encoding a GRP polypeptide in a plant. The GRP polypeptide as described herein is an Ankyrin - Zinc finger polypeptide (AZ). The improved characteristics comprise yield related traits, such as enhanced yield and/or enhanced growth, or modified content of storage compounds.

## Background

Ankyrin - Zinc finger polypeptide

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Transcription is performed by RNA polymerases. These polymerases are usually associated with other proteins (transcription factors) that determine the specificity of the transcription process. The transcription factors bind to cis-regulatory elements of the gene and may also mediate binding of other regulatory proteins. Stegmaier et al. proposed a classification of transcription factors based on their DNA-binding domains. 5 superclasses were discriminated, based on the presence of: 1) basic domains, 2) zinc-coordinating domains, 3) helix-turn-helix domains, 4) beta scaffold domains with minor groove contacts and 0) other domains (Stegmaier et al., Genome informatics 15, 276-286, 2004). The group of transcription factors comprising zinc-coordinating domains is quite divers and may be further classified according to their conserved cysteine and histidine residues, including the WRKY domains, C6 zinc clusters, DM and GCM domains.

Besides DNA binding motifs, transcription factors may also comprise protein-protein interaction motifs. One such motif is the ankyrin motif. It is present in very diverse families of proteins, usually as a repeat of 2 to over 20 units. Each unit contains two antiparallel helices and a beta-hairpin.

Although many plant proteins with zinc finger domains are well characterised, little is known about plant proteins comprising the C3H1 zinc finger motif. PEI1, a transcription factor that reportedly plays a role in embryo development, has a zinc finger motif that resembles the C3H1 motif but lacks an ankyrin motif (Li and Thomas, Plant Cell 10, 383-398, 1998). WO 02/44389 describes AtSIZ, a transcription factor isolated from *Arabidopsis.* Expression of *AtSIZ* under control of the CaMV35S promoter was found to promote transcription of stress-induced genes, and plants with increased expression of *AtSIZ* reportedly had a higher survival rate under salt stress than control plants, but no analysis was provided with respect to seed yield. It was postulated that AtSIZ could be used for increasing resistance in plants to osmotic stress.

## Summary

Surprisingly, it has now been found that modulating expression of a nucleic acid encoding a GRP polypeptide gives plants having improved characteristics relative to control plants.

The present specification describes that modulating expression in a plant of a nucleic acid encoding the AZ polypeptide from *Arabidopsis* (AtAZ) or a homologue thereof gives plants having increased yield relative to control plants.

The specification describes a method for increasing plant yield, comprising modulating expression in a plant of a nucleic acid encoding the AZ polypeptide or a homologue thereof. Advantageously, performance of the methods according to the present invention results in plants having increased yield, particularly seed yield, relative to corresponding wild type plants. The present specification also describes nucleic acid sequences and constructs useful in performing such methods.

The present invention provides subject matter as set forth in any one and all of items (1) to (22) below:

1. Method for increasing seed yield of plants relative to corresponding wild type plants, comprising introducing and expressing in a plant an AZ nucleic acid or a variant thereof, wherein said AZ nucleic acid encodes an AZ polypeptide or a homologue thereof, wherein the homologue gives plants having increased yield, and wherein said AZ polypeptide or the homologue thereof comprises two ankyrin repeats and two C3H1 Zinc finger domains, and wherein said ankyrin repeats are located upstream of the C3H1 Zinc fingec domains.
2. Method according to item 1, wherein the AZ polypeptide comprises at least one of the following motifs:

   (P/A)CSRAY(S/T)HDWTEC (motif 1, SEQ ID NO: 3)
   HPGENARRRDPR (motif 2, SEQ ID: NO: 4)
   HG(V/I)FE(C/S)WLHP(A/S)QY(R/K)TRLCK (motif 3, SEQ ID NO: 5)
   CFFAH (motif 4, SEQ ID NO: 6).

3. Method according to item 1 or 2, wherein said AZ nucleic acid encodes an AZ polypeptide of SEQ ID NO: 2 or a homologue thereof.
4. Method according to any one of items 1 to 3, wherein said variant is a portion of an AZ nucleic acid or a sequence capable of hybridising to an AZ nucleic acid, which portion or hybridising sequence encodes an AZ polypeptide comprising two ankyrin repeats and two C3H1 Zinc finger domains, and wherein said ankyrin repeats are located upstream of the C3H1 Zinc finger domains
5. Method according to any one of items 1 to 4, wherein said AZ nucleic acid or variant thereof is overexpressed in a plant.
6. Method according to any one of items 1 to 5, wherein said AZ nucleic acid or variant thereof is of plant origin, preferably from a dicotyledonous plant, more preferably from the family Brassicaceae, most preferably the nucleic acid is from Arabidopsis thaliana.
7. Method according to any one of items 1 to 6, wherein said AZ nucleic acid or variant thereof is operably linked to a seed specific promoter.
8. Method according to item 7, wherein said seed-specific promoter is a WSI18 promoter.
9. Method according to any one of items 1 to 8, wherein said increased seed yield comprises increased thousand kernel weight.
10. Construct comprising;

   (i) an *AZ* nucleic acid or a variant thereof as defined in any of items 1 to 4 and 6;
   (ii) a seed-specific WSI18 promoter or a constitutive GOS2 promoter operably linked to the nucleic acid sequence of (i)

11. Construct according to item 10, wherein said WSI18 promoter is as represented by SEQ ID NO: 55 or SEQ ID NO: 9.
12. Construct according to item 10, wherein said GOS2 promoter is as represented by SEQ ID NO: 56 or SEQ ID NO: 54.
13. Plant transformed with a construct according to any one of items 10 to 12.
14. Method for the production of a transgenic plant having increased seed yield compared to corresponding wild type plants, which method comprises:

   (i) introducing and expressing in a plant or plant cell an *AZ* nucleic acid or variant thereof as defined in any of items 1 to 4 and 6; and
   (ii) cultivating the plant cell under conditions promoting plant growth and development,

15. Transgenic plant according to item 13, wherein said plant is a monocotyledonous plant such as sugar cane or wherein the plant is a cereal, such as rice, maize, wheat, barley, millet, rye, oats or sorghum.
16. Harvestable parts of a plant according to any one of items 13 or 15, wherein said harvestable parts comprise a construct according to any one of items 10 to 12.
17. Harvestable parts of a plant according to item 16 wherein said harvestable parts are seeds.

18. Products directly derived from a plant according to item 13 or 15 and/or from harvestable parts of a plant according to items 16 or 17, wherein said products comprise a construct according to any one of items 10 to 12.
19. Use of an AZ nucleic acid or variant thereof as defined in any of items 1 to 4 and 6, or use of an AZ polypeptide or a homologue thereof, in increasing seed yield, relative to corresponding wild type plants.
20. Use according to item 19, wherein said increased seed yield comprises increased thousand kernel weight.
21. Use of an AZ nucleic acid or variant thereof as defined in any of items 1 to 4 and 6, or use of an AZ polypeptide or a homologue thereof, as a molecular marker.
22. Use of a construct according to any of items 10 to 12 in a method for making plants having increased seed yield relative to control plants.

**Definitions**

Polypeptide (s)/Protein(s)

The terms "polypeptide" and "protein" are used interchangeably herein and refer to amino acids in a polymeric form of any length, linked together by peptide bonds.

Polynucleotide(s)/Nudeic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag·100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |

(continued)

| Residue | Conservative Substitutions | Residue | Conservative Substitutions |
|---|---|---|---|
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1 °C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| **Gene Source** | **Reference** |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |

(continued)

| Gene Source | Reference |
|---|---|
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are shown in Tables 2b to 2e below. Further examples of seed-specific promoters are given in Qing Qu and Takaiwa (Plant Biotechnol. J. 2, 113-125, 2004), which disclosure is incorporated by reference herein as if fully set forth.

**Table 2b:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |

(continued)

| Gene source | Reference |
|---|---|
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2c:** examples of endosperm-specific promoters

| **Gene source** | **Reference** |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2d:** Examples of embryo specific promoters:

| **Gene source** | **Reference** |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2e:** Examples of aleurone-specific promoters:

| Gene source | Reference |
|---|---|
| $\alpha$-amylase (Amy32b) | Lanahan et al, Plant Cell 4:203-211, 1992; Skriver et al, Proc Natl Acad Sci USA 88: 7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38, 1998 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene as described herein so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and technique. A method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods as described herein does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence as described herein may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods as described herein (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

As described herein, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct as described herein. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides as described herein or used in the methods as described herein, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process as described herein for introducing the nucleic acids advantageously employs techniques which enable the removal or excision of these marker genes. One such a method is what is known as co-transformation. The co-transformation method employs two vectors simultaneously for the transformation, one vector bearing the nucleic acid as described herein and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the IoxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences as described herein is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors as described herein, all those constructions brought about by recombinant methods in which either

(a) the nucleic acid sequences encoding proteins useful in the methods as described herein, or
(b) genetic control sequence(s) which is operably linked with the nucleic acid sequence as described herein, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods as described herein, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant as described herein is thus understood as meaning, as above, that the nucleic acids used in the method as described herein are not at their natural locus in the genome of said plant, it being possible for the nucleic acids to be expressed homologously or heterologously. However, as mentioned, transgenic also means that, while the nucleic acids as described herein or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids as described herein at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct as described herein and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient as described herein to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

<u>T-DNA activation tagging</u>

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

<u>TILLING</u>

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description**

**Detailed description for the Ankyrin - Zn finger polypeptide**

Surprisingly, the specificiation describes that modulating expression in a plant of a nucleic acid encoding the AZ polypeptide from *Arabidopsis* (AtAZ) or a homologue thereof gives plants having increased yield relative to control plants. According to one embodiment of the present invention, the specificiation describes a method for increasing plant yield, comprising modulating expression in a plant of a nucleic acid encoding the AZ polypeptide or a homologue thereof. Advantageously, performance of the methods according to the present specification results in plants having increased yield, particularly seed yield, relative to corresponding wild type plants.

A "reference", "reference plant", "control", "control plant", "wild type" or "wild type plant" is in particular a cell, a tissue, an organ, a plant, or a part thereof, which was not produced according to the method as described herein. Accordingly, the terms "wild type", "control" or "reference" are exchangeable and can be a cell or a part of the plant such as an organelle or tissue, or a plant, which was not modified or treated according to the herein described method. Accordingly, the cell or a part of the plant such as an organelle or a plant used as wild type, control or reference corresponds to the cell, plant or part thereof as much as possible and is in any other property but in the result of the process as described herein as identical to the subject matter as described herein as possible. Thus, the wild type, control or reference is treated identically or as identical as possible, saying that only conditions or properties might be different which do not influence the quality of the tested property. That means in other words that the wild type denotes (1) a plant, which carries the unaltered or not modulated form of a gene or allele or (2) the starting material/plant from which the plants produced by the process or method as described herein are derived.

Preferably, any comparison between the wild type plants and the plants produced by the method of the invention is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, a plant, which was not modulated, modified or treated according to the herein described process as described herein and is in any other property as similar to the subject matter as described herein as possible. The reference, control or wild type is in its genome, transcriptome, proteome or metabolome as similar as possible to the subject as described herein. Preferably, the term "reference-" "control-" or "wild type-"-organelle, -cell, -tissue or plant, relates to an organelle, cell, tissue or plant, which is nearly genetically identical to the organelle, cell, tissue or plant, as described herein or a part thereof preferably 95%, more preferred are 98%, even more preferred are 99,00%, in particular 99,10%, 99,30%, 99,50%, 99,70%, 99,90%, 99,99%, 99, 999% or more. Most preferably the "reference", "control", or "wild type" is a subject, e.g. an organelle, a cell, a tissue, a plant, which is genetically identical to the plant, cell organelle used according to the method as described herein except that nucleic acid molecules or the gene product encoded by them are changed, modulated or modified according to the inventive method.

The term "expression" or "gene expression" is as defined herein, preferably it results in the appearance of a phenotypic trait as a consequence of the transcription of a specific gene or specific genes.

The increase referring to the activity of the polypeptide amounts in a cell, a tissue, a organelle, an organ or an organism or a part thereof preferably to at least 5%, preferably to at least 10% or at to least 15%, especially preferably to at least 20%, 25%, 30% or more, very especially preferably are to at least 40%, 50% or 60%, most preferably are to at least 70% or more in comparison to the control, reference or wild type.

The term "increased yield" as defined herein is taken to mean an increase in biomass (weight) of one or more parts of a plant, which may include aboveground (harvestable) parts and/or (harvestable) parts below ground. In a preferred embodiment, the increased yield is increased seed yield.

Therefore, such harvestable parts are preferably seeds, and performance of the methods of the invention results in plants having increased seed yield relative to the seed yield of control plants.

An increase in seed yield may also be manifested as an increase in seed size and/or seed volume, which may also influence the composition of seeds (including oil, protein and carbohydrate total content and composition).

Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in seed filling rate (which is the number of filled seeds divided by the total number of seeds and multiplied by 100), among others. Taking rice as an example, a yield increase may be manifested by an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate, (which is the number of filled seeds divided by the total number of seeds and multiplied by 100), increase in thousand kernel weight, among others. An increase in yield may also result in modified architecture, or may occur as a result of modified architecture.

According to the specification, performance of the methods of the invention result in plants having increased yield, particularly seed yield. Therefore, according to the present specification, there is provided a method for increasing plant yield, which method comprises modulating expression in a plant of a nucleic acid encoding the AZ polypeptide or a homologue thereof.

Since the transgenic plants as described herein have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the planting and harvesting of corn plants followed by, for example, the planting and optional harvesting of soy bean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present specification, performance of the methods of the invention gives plants having an increased growth rate or increased yield in comparison to control plants. Therefore, according to the present specification, there is provided a method for increasing yield and/or growth rate in plants, which method comprises modulating expression in a plant of a nucleic acid encoding the AZ polypeptide or a homologue thereof.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress as described herein leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods as described herein may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that do not impose stress, such as the stresses described above, on plants. Non-stress conditions allow optimal growth of plants. Persons skilled in the art are aware of normal soil conditions and climatic conditions for a given location.

Performance of the methods as described herein gives plants grown under non-stress conditions or under mild drought conditions increased yield relative to control plants grown under comparable conditions. Therefore, according to the present specification, there is provided a method for increasing yield in plants grown under non-stress conditions or under mild drought conditions, which method comprises increasing expression in a plant of a nucleic acid encoding a AZ polypeptide.

As described herein, the increase in yield and/or growth rate occurs according to the methods of the present specification under non-stress conditions.

Performance of the methods as described herein gives plants grown under conditions of nutrient deficiency, particularly under conditions of nitrogen deficiency, increased yield relative to control plants grown under comparable conditions. Therefore, according to the present specification, there is provided a method for increasing yield in plants grown under conditions of nutrient deficiency, which method comprises increasing expression in a plant of a nucleic acid encoding a AZ polypeptide. Nutrient deficiency may result from a lack of nutrients such as nitrogen, phosphates and other phosphorous-containing compounds, potassium, calcium, cadmium, magnesium, manganese, iron and boron, amongst others.

The methods as described herein are advantageously applicable to any plant. The abovementioned growth characteristics may advantageously be modified in any plant. Plants that are particularly useful in the methods as described herein include all plants which belong to the superfamily Viridiplantae, in particular monocotyledonous and dicotyledonous plants including fodder or forage legumes, ornamental plants, food crops, trees or shrubs. As described herein, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera *Helianthus, Tagetes* e.g. the species *Helianthus annuus* [sunflower], *Tagetes lucida, Tagetes erecta* or *Tagetes tenuifolia* [Marigold]; Brassicaceae such as the genus *Brassica,* e.g. the species *Brassica napus, Brassica rapa* ssp. [canola, oilseed rape, turnip rape]; Fabaceae such as the genera *Glycine* e.g. the species *Glycine max, Soja hispida* or *Soja max* [soybean]; Linaceae such as the genera *Linum* e.g. the species *Linum usitatissimum,* [flax, linseed]; Poaceae such as the genera *Hordeum, Secale, Avena, Sorghum, Oryza, Zea, Triticum* e.g. the species *Hordeum vulgare* [barley], *Secale cereale* [rye], *Avena sativa, Avena fatua, Avena byzantina, Avena fatua* var. sativa, *Avena hybrida* [oat], *Sorghum bicolor* [sorghum, millet], *Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat]; Solanaceae such as the genera *Solanum, Lycopersicon* e.g. the species *Solanum tuberosum* [potato], *Lycopersicon esculentum, Lycopersicon lycopersicum, Lycopersicon pyriforme, Solanum integrifolium* or *Solanum lycopersicum* [tomato].

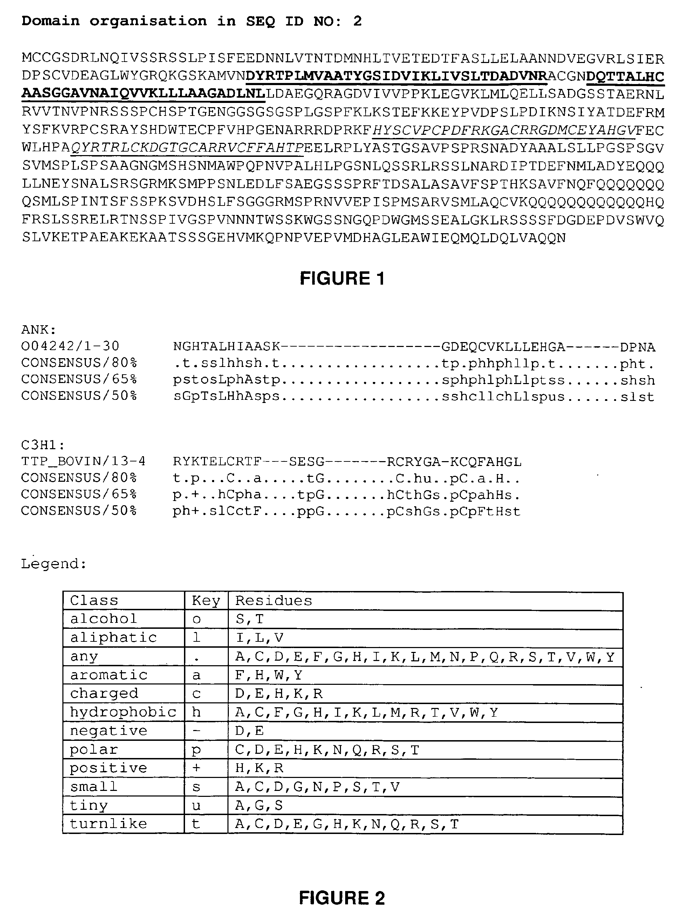

The term "AtAZ polypeptide or a homologue thereof" as defined herein refers to proteins that comprise at least one ankyrin repeat and at least one Zinc-finger C3H1 domain, which ankyrin repeat is located upstream of the C3H1 domain. Preferably, the AZ protein comprises two ankyrin repeats and two Zinc-finger C3H1 domains such as in the protein represented in SEQ ID NO: 2. Also preferably, the two ankyrin repeats are located close to each other. Further preferably, the Zinc-finger C3H1 domains are also located close to each other and C-terminally of the ankyrin repeats. In SEQ ID NO: 2, the ankyrin repeats are located on positions D90 to R120 and D125 to L157, the two Zn-finger domains are located on positions H301 to V327 and Q336 to P359 (Figure 1).

Also preferably, the AtAZ protein comprises at least one of the following consensus sequences:

(P/A)CSRAY(S/T)HDWTEC (motif 1, SEQ ID NO: 3)
HPGENARRRDPR (motif 2, SEQ ID NO: 4)
HG(V/I)FE(C/S)WLHP(A/S)QY(R/K)TRLCK (motif 3, SEQ ID NO: 5)
CFFAH (motif 4, SEQ ID NO: 6)

Preferably motif 1 is PCSRAYSHDWTEC, and motif 3 is preferably HGVFECWLHPAQYRTRLCK.

More preferably, the AtAZ protein comprises two of the above-mentioned motifs, especially preferably 3 of the above-mentioned motifs, most preferably all four motifs.

The ankyrin repeat (SMART SM00248, Interpro IPR002110), as described in the Interpro database, is one of the most common protein-protein interaction motifs in nature. Ankyrin repeats are (usually tandemly) repeated modules of usually about 33 amino acids. They occur in a large number of functionally diverse proteins mainly from eukaryotes. The few known examples from prokaryotes and viruses may be the result of horizontal gene transfers. The repeat has been found in proteins of diverse function such as transcriptional initiators, cell-cycle regulators, cytoskeletal, ion transporters and signal transducers. The ankyrin fold appears to be defined by its structure rather than its function since there is no specific sequence or structure which is universally recognised by it. The conserved fold of the ankyrin repeat unit is known from several crystal and solution structures. Each repeat folds into a helix-loop-helix structure with a beta-hairpin/loop region projecting out from the helices at a 90° angle. The repeats stack together to form an L-shaped structure.

The Zinc-finger domain ZnF_C3H1 (also known as Znf_CCCH, SMART SM00356; Interpro IPR000571), as described in the Interpro database is thought to be involved in DNA-binding. Zinc fingers exist as different types, depending on the positions of the cysteine residues. Proteins containing zinc finger domains of the C-x8-C-x5-C-x3-H type (wherein x represents any amino acid and the digits 8, 5 and 3 represent the number of amino acids between the conserved C or H residues) include zinc finger proteins from eukaryotes involved in cell cycle or growth phase-related regulation, e.g. human TIS11B (butyrate response factor 1), a probable regulatory protein involved in regulating the response to growth factors, and the mouse TTP growth factor-inducible nuclear protein, which has the same function. The mouse TTP protein is induced by growth factors. Another protein containing this domain is the human splicing factor U2AF 35 kD subunit, which plays a critical role in both constitutive and enhancer-dependent splicing by mediating essential protein-protein interactions and protein-RNA interactions required for 3' splice site selection. It has been shown that different CCCH zinc finger proteins interact with the 3' untranslated region of various mRNA. This type of zinc finger is very often present in two copies.

Figure 2 describes the consensus sequences as defined in the SMART database for the ankyrin domain and the zinc-finger domain; however it should be noted that these consensus sequences might be biased towards animal protein sequences.

The terms "domain" and "motif" are defined in the "definitions" section herein. Specialist databases exist for the identification of domains. The C3H1 or ankyrin domain in a AZ protein may be identified using, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)). A set of tools for in silico analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)). The AZ protein sequence was analysed with the SMART tool (version 4.1; Schultz et al. (1998) Proc. Natl. Acad. Sci. USA 95, 5857-5864; Letunic et al. (2002) Nucleic Acids Res 30, 242-244) and was used to screen the Pfam (Version 17.0, March 2005; Bateman et al. (2004) Nucl. Acids Res. 32, D138-141) and InterPro database (Release 11.0, 26 July 2005; Mulder et al. (2005) Nucl. Acids. Res. 33, D201-205).

By aligning other protein sequences with SEQ ID NO: 2, the corresponding consensus sequences, the C3H1 domain, the ankyrin domain or other sequence motifs may easily be identified. In this way, AZ polypeptides or homologues thereof (encompassing orthologues and paralogues) may readily be identified, using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the C3H1 or ankyrin domain, or one of the motifs defined above) may be used as well. The sequence identity values, which are indicated below as a percentage were determined over the entire conserved domain or nucleic acid or amino acid sequence using the programs mentioned above using the default parameters.

Examples of AZ proteins or homologues thereof are given in Table A of Example 1.

Sequences falling under the definition of "AZ polypeptide or homologue thereof" are as defined in claim 1 and preferably comprise also at least one of the consensus sequence of SEQ ID NO: 3, 4, 5 or 6 as defined above, may be suitable for use in the methods of the invention. Preferably, the polypeptide is a polypeptide from *Arabidopsis thaliana.*

Encompassed by the term "homologues" are orthologous sequences and paralogous sequences. Orthologues and paralogues may be found by performing a so-called reciprocal blast search. This may be done by a first BLAST involving BLASTing a query sequence (for example, SEQ ID NO: 1 or SEQ ID NO: 2) against any sequence database, such as the publicly available NCBI database. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis* sequences). The results of the first and second BLASTs are then compared. A paralogue is identified if a high-ranking hit from the second BLAST is from the same species as from which the query sequence is derived; an orthologue is identified if a high-ranking hit is not from the same species as from which the query sequence is derived. Preferred orthologues are orthologues of SEQ ID NO: 1 or SEQ ID NO: 2. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. Preferably the score is greater than 50, more preferably greater than 100; and preferably the E-value is less than e-5, more preferably less than e-6. In the case of large families, ClustalW may be used, followed by the generation of a neighbour joining tree, to help visualize clustering of related genes and to identify orthologues and paralogues. Examples of sequences orthologous to SEQ ID NO: 2 include SEQ ID NO: 11, SEQ ID NO: 15 and SEQ ID NO: 17. SEQ ID NO: 19 is an example of a paralogue of SEQ ID NO: 2.

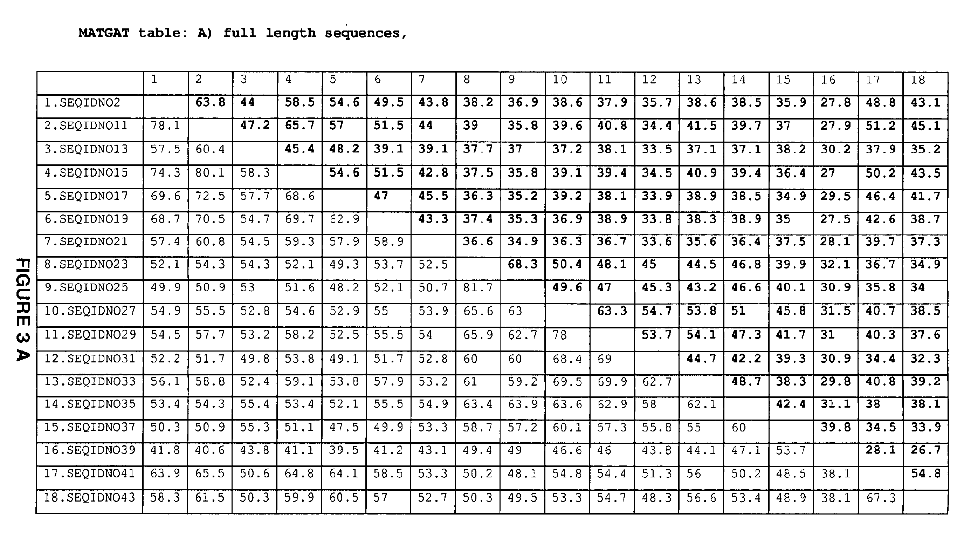

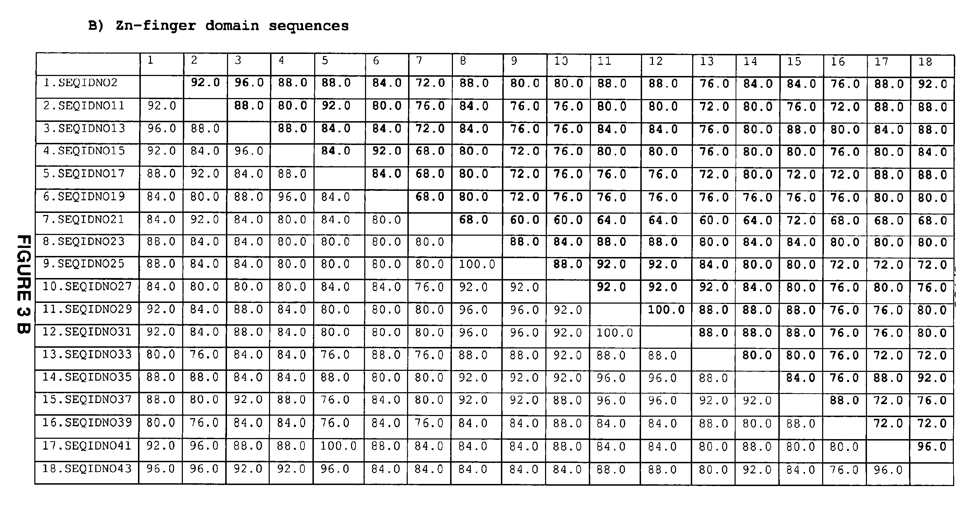

The specification describes AZ proteins that have, besides at least one ankyrin repeat and at least one C3H1 domain, in increasing order of preference, at least 26%, 30%, 35%, 40%, 45%, 50%, 55%, 60%, 65%, 70%, 75%, 80%, 85%, 90%, 95%, 96%, 97%, 98% or 99% sequence identity to the protein of SEQ ID NO: 2. The matrix shown in Figure 3 (Matrix A) shows similarities and identities (in bold) over the full-length of various AZ proteins. In case only specific domains are compared, the identity or similarity may be higher among the different proteins (Matrix B: comparison of a Zn-finger domain sequence).

An assay may be carried out to determine AZ activity. DNA-binding activity and protein-protein interactions may readily be determined *in vitro* or *in vivo* using techniques well known in the art. Examples of *in vitro* assays for DNA binding activity include: gel retardation analysis or yeast one-hybrid assays. An example of an *in vitro* assay for protein-protein interactions is the yeast two-hybrid analysis (Fields and Song (1989) Nature 340:245-6).

Furthermore, as described herein expression of the AZ protein or of a homologue thereof in plants, and in particular in rice, has the effect of increasing yield of the transgenic plant when compared to corresponding wild type plants, wherein increased yield comprises at least one of: total weight of seeds, total number of seeds and number of filled seeds.

An AZ polypeptide or homologue thereof as defined herein is encoded by an AZ nucleic acid/gene. Therefore the term "AZ nucleic acid/gene" as defined herein is any nucleic acid/gene encoding an AZ polypeptide or a homologue thereof as defined above. Examples of AZ nucleic acids include but are not limited to those represented in Table A of Example 1. AZ nucleic acids/genes and variants thereof may be suitable in practising the methods as described herein. Preferably, the variants of an AZ gene originate from *Arabidopsis thaliana.* Variant AZ nucleic acid/genes include portions of an AZ nucleic acid/gene, splice variants, allelic variants and/or nucleic acids capable of hybridising with an AZ nucleic acid/gene.

Reference herein to a "nucleic acid sequence" is taken to mean a polymeric form of a deoxyribonucleotide or a ribonucleotide polymer of any length, either double- or single-stranded, or analogues thereof, that has the essential characteristic of a natural ribonucleotide in that it can hybridise to nucleic acid sequences in a manner similar to naturally occurring polynucleotides.

The term portion as described herein refers to a piece of DNA encoding a polypeptide comprising at least one ankyrin repeat and at least one C3H1 Zn-finger domain. A portion may be prepared, for example, by making one or more deletions to an AZ nucleic acid. The portions may be used in isolated form or they may be fused to other coding (or non-coding) sequences in order to, for example, produce a protein that combines several activities. When fused to other coding sequences, the resulting polypeptide produced upon translation may be bigger than that predicted for the AZ fragment. The portion is typically at least 500, 700 or 900 nucleotides in length, preferably at least 1100, 1300 or 1500 nucleotides in length, more preferably at least 1700, 1900 or 2100 nucleotides in length and most preferably at least 2300 or 2400 nucleotides in length. Preferably, the portion is a portion of a nucleic acid as represented in Table A of Example 1. Most preferably the portion of an AZ nucleic acid is as represented by SEQ ID NO: 1 or SEQ ID NO: 53.

The terms "fragment", "fragment of a sequence" or "part of a sequence" "portion" or "portion thereof" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridising with the nucleic acid molecule as described herein or used in the process as described herein under stringent conditions, while the maximum size is not critical. In some applications, the maximum size usually is not substantially greater than that required to provide the desired activity and/or function(s) of the original sequence. A comparable function means at least 40%, 45% or 50%, preferably at least 60%, 70%, 80% or 90% or more of the function of the original sequence.

Another variant of an AZ nucleic acid/gene as defined herein is a nucleic acid capable of hybridising under reduced stringency conditions, preferably under stringent conditions, with an AZ nucleic acid/gene as hereinbefore defined or with a portion as hereinbefore defined. The hybridizing sequence is typically at least 300 nucleotides in length, preferably at least 400 nucleotides in length, more preferably at least 500 nucleotides in length and most preferably at least 600 nucleotides in length.

Preferably, the hybridising sequence is one that is capable of hybridising to a nucleic acid as represented by SEQ ID NO: 1, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 53, or to a portion of any of the aforementioned sequences, a portion being defined as above. Most preferably the hybridising sequence is capable of hybridising to SEQ ID NO: 1, to SEQ ID NO: 53, or to portions (or probes) thereof. Methods for designing probes are well known in the art. Probes are generally less than 1000 bp, 900 bp, 800 bp, 700 bp, 600 bp in length, preferably less than 500 bp, 400 bp, 300 bp 200 bp or 100 bp in length. Commonly, probe lengths for DNA-DNA hybridizations such as Southern blotting, vary between 100 and 500 bp, whereas the hybridizing region in probes for DNA-DNA hybridizations such as in PCR amplification generally are shorter than 50 but longer than 10 nucleotides, preferably they are 15, 20, 25, 30, 35, 40, 45 or 50 bp in length.

Also useful in the methods of the invention are nucleic acids encoding homologues (including orthologues or paralogues) of the amino acid sequence represented by SEQ ID NO: 2, or derivatives thereof.

Another nucleic acid variant useful in the methods as described herein is a splice variant encoding an AZ polypeptide as defined above. As described herein splice variants are splice variants of the nucleic acid encoding a polypeptide comprising at least on ankyrin repeat and at least one C3H1 domain. Preferably, the AZ polypeptide or the homologue thereof encoded by the splice variant has at least 26%, 30%, 35%, 40%, 45%, 50%, 55%, 60%, 65%, 70%, 75%, 80%, 85%, 90%, 95%, 96%, 97%, 98% or 99% sequence identity to SEQ ID NO: 2. Further the specification describes splice variants represented by the nucleic acids represented in Table A of Example 1. For example, SEQ ID NO: 25 and SEQ ID NO: 47 are encoded by splice variants of the same gene. For example the splice variant represented by SEQ ID NO: 1 or SEQ ID NO: 53.

Another nucleic acid variant useful in the methods as described herein is an allelic variant of a nucleic acid encoding an AZ polypeptide as defined above. Preferably, the polypeptide encoded by the allelic variant is represented by the polypeptide sequences listed in Table A of Example 1. Most preferably, the allelic variant encoding the AZ polypeptide is represented by SEQ ID NO: 1.

A further nucleic acid variant useful in the methods as described herein is a nucleic acid variant obtained by gene shuffling. Furthermore, site-directed mutagenesis may be used to generate variants of AZ nucleic acids. Several methods are available to achieve site-directed mutagenesis; the most common being PCR based methods (Current Protocols in Molecular Biology. Wiley Eds.).

Therefore, the specification describes a method for increasing yield and/or growth rate of a plant, comprising modulating expression in a plant of a variant of an AZ nucleic acid selected from:

(i) a portion of an AZ nucleic acid;
(ii) a nucleic acid hybridising to an AZ nucleic acid;
(iii) a splice variant of a nucleic acid encoding an AZ polypeptide;
(iv) an allelic variant of a nucleic acid encoding an AZ polypeptide; and
(v) a nucleic acid variant encoding an AZ polypeptide obtained by gene shuffling or site directed mutagenesis.

The AZ nucleic acid or variant thereof as defined herein may be derived from any natural or artificial source. This nucleic acid may be modified from its native form in composition and/or genomic environment through deliberate human manipulation. The nucleic acid is preferably of plant origin, further preferably from a dicotyledonous species, further preferably from the family Brassicaceae, more preferably from *Arabidopsis thaliana.* Most preferably, the AZ nucleic acid is the *Arabidopsis thaliana* sequence represented by SEQ ID NO: 1, and the AZ amino acid sequence is as represented by SEQ ID NO: 2. Alternatively, the AZ nucleic acid represented by SEQ ID NO: 53 or the AZ amino acid sequence as represented by SEQ ID NO: 47 may also be useful in the methods of the present invention.

Any reference herein to an AZ polypeptide is therefore taken to mean an AZ protein as defined above. Any nucleic acid encoding such an AZ protein is suitable for use in the methods as described herein.

According to the present specification, modulated, preferably increased expression of the AZ nucleic acid or variant thereof is envisaged. Methods for increasing the expression of genes or gene products are well documented in the art. The expression of a nucleic acid sequence encoding an AZ polypeptide may be modulated by introducing a genetic modification, which may be introduced, for example, by any one (or more) of the following methods: T-DNA activation, TILLING, site-directed mutagenesis, directed evolution and homologous recombination or by introducing and expressing in a plant a nucleic acid encoding an AZ polypeptide or a homologue thereof. Following introduction of the genetic modification, there follows a step of selecting for modified expression of a nucleic acid encoding an AZ polypeptide or a homologue thereof, which modification in expression gives plants having increased yield. Also methods for decreasing the expression of genes or gene products are known in the art.

T-DNA activation is described above. A genetic modification may also be introduced in the locus of an AZ gene using the technique of TILLING (Targeted Induced Local Lesions In Genomes). The locus of a gene as defined herein is taken to mean a genomic region, which includes the gene of interest and 10 kb up- or down stream of the coding region. Site-directed mutagenesis may be used to generate variants of AZ nucleic acids. Several methods are available to achieve site-directed mutagenesis; the most common being PCR based methods (Current Protocols in Molecular Biology. Wiley Eds.). According to the present specification homologous recombination may be applied.

The specification describes a method for introducing a genetic modification is to introduce and express in a plant a nucleic acid encoding an AZ polypeptide or a homologue thereof, as defined above. The nucleic acid to be introduced into a plant may be a full-length nucleic acid or may be a portion or a hybridising sequence as hereinbefore defined.

The specification furthermore describes genetic constructs and vectors to facilitate introduction and/or expression of the nucleotide sequences useful in the methods as described herein.

Therefore, there is described a gene construct comprising:

(i) an AZ nucleic acid or variant thereof, as defined hereinabove;
(ii) one or more control sequences operably linked the nucleic acid sequence of (i);

Constructs useful in the methods as described herein may be created using recombinant DNA technology well known to persons skilled in the art. The gene constructs may be inserted into vectors, which may be commercially available, suitable for transforming into plants and suitable for expression of the gene of interest in the transformed cells. The specification therefore describes the use of a gene construct as defined hereinabove in the methods as described herein.

Plants are transformed with a vector comprising the sequence of interest (i.e., a nucleic acid encoding an AZ polypeptide or homologue thereof). The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are defined above. Advantageously, any type of promoter may be used to drive expression of the nucleic acid sequence.

Suitable promoters, which are functional in plants, are generally known. They may take the form of constitutive or inducible promoters. Suitable promoters can enable the developmental- and/or tissue-specific expression in multicellular eukaryotes; thus, leaf-, root-, flower-, seed-, stomata-, tuber- or fruit-specific promoters may advantageously be used in plants.

Different plant promoters usable in plants are promoters such as, for example, the USP, the LegB4-, the DC3 promoter or the ubiquitin promoter from parsley.

A "plant" promoter comprises regulatory elements, which mediate the expression of a coding sequence segment in plant cells. Accordingly, a plant promoter need not be of plant origin, but may originate from viruses or micro-organisms, in particular for example from viruses which attack plant cells.

The "plant" promoter can also originate from a plant cell, e.g. from the plant which is transformed with the nucleic acid sequence to be expressed in the inventive process and described herein. This also applies to other "plant" regulatory signals, for example in "plant" terminators.

For expression in plants, the nucleic acid molecule must, as described above, be linked operably to or comprise a suitable promoter which expresses the gene at the right point in time and in a cell- or tissue-specific manner. Useful promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugar beet V-ATPase promoters (WO 01/14572). Examples of synthetic constitutive promoters are the Super promoter (WO 95/14098) and promoters derived from G-boxes (WO 94/12015). If appropriate, chemically inducible promoters may furthermore also be used, compare EP-A 388186, EP-A 335528, WO 97/06268. Stable, constitutive expression of the proteins as described herein a plant can be advantageous. However, inducible expression of the polypeptide as described herein is advantageous, if, for example, late expression before the harvest is of advantage, as metabolic manipulation may lead to plant growth retardation.

The expression of plant genes can also be facilitated via a chemically inducible promoter. Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Promoters as described herein are in particular those which bring gene expression in tissues and organs, in seed cells, such as endosperm cells and cells of the developing embryo. Suitable promoters are the oilseed rape napin gene promoter (US 5,608,152), the *Vicia faba* USP promoter (Baeumlein et al., Mol Gen Genet, 1991, 225 (3): 459-67), the *Arabidopsis* oleosin promoter (WO 98/45461), the *Phaseolus vulgaris* phaseolin promoter (US 5,504,200), the *Brassica* Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley lpt2 or lpt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or $\alpha$-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

According to one preferred feature of the invention, the AZ nucleic acid or variant thereof as defined herein is operably linked to a seed-specific promoter. The seed-specific promoter may be active during seed development and/or during germination. Seed-specific promoters are well known in the art. Preferably, the seed-specific promoter is an embryo specific / endosperm specific / aleurone specific promoter. Further preferably, the seed-specific promoter drives expression in at least one of: the embryo, the endosperm, the aleurone. More preferably, the promoter is a WSI18 or a functionally equivalent promoter. Most preferably, the promoter sequence is as represented by SEQ ID NO: 9 or SEQ ID NO: 55. Examples of other seed-specific promoters (embryo specific / endosperm specific / aleurone specific promoters) which may also be used to drive expression of an AZ nucleic acid are provided above.

According to the specification, the AZ nucleic acid or variant thereof as defined herein is operably linked to a constitutive promoter. A constitutive promoter is a constitutive promoter that is also substantially ubiquitously expressed. Further preferably the promoter is derived from a plant, more preferably a monocotyledonous plant. An example of such a promoter is the GOS2 promoter from rice (SEQ ID NO: 54 or SEQ ID NO: 56). Examples of other constitutive promoters which may also be used to drive expression of an AZ nucleic acid as defined above are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs as described herein may further include an origin of replication sequence that is required for maintenance and/or replication in a specific cell type.

One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods as described herein and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The present specification also describes plants, plant parts or plant cells obtainable by the methods as described herein. The present specification therefore describes plants obtainable by the method as described herein, which plants have introduced therein an AZ nucleic acid or variant thereof, as defined above.

The specification also describes a method for the production of transgenic plants having increased yield, comprising introduction and expression in a plant of an AZ nucleic acid or a variant thereof as defined above.

Host plants for the nucleic acids or the vector used in the method as described herein, the expression cassette or construct or vector are, in principle, advantageously all plants, which are capable of synthesizing the polypeptides used in the method as described herein.

More specifically, the present specification describes a method for the production of transgenic plants having increased yield, which method comprises:

(i) introducing and expressing in a plant or plant cell an AZ nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a feature of the present specification, the nucleic acid is introduced into a plant by transformation. The terms "introduction" or "transformation" are described in more detail in the definitions section.

Generally after transformation, plant cells or cell groupings are selected for the presence of one or more markers which are encoded by plant-expressible genes co-transferred with the gene of interest, following which the transformed material is regenerated into a whole plant.

As mentioned, Agrobacteria transformed with an expression vector as described herein may also be used in the manner known per se for the transformation of plants such as plants used as a model, like *Arabidopsis*, or crop plants, such as cereals, maize, oats, rye, barley, wheat, soybean, rice, cotton, sugar beet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soy, peanut, castor-oil plant, sunflower, maize, cotton, flax, oilseed rape, coconut, oil palm, safflower *(Carthamus tinctorius)* or cocoa beans, for example by bathing scarred leaves or leaf segments in an agrobacterial solution and subsequently culturing them on suitable media.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present specification describes to any plant cell or plant produced by any of the methods described herein, and to all plant parts and propagules thereof. The present specification describes further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods as described herein. Described are host cells containing an isolated AZ nucleic acid or variant thereof. Preferred host cells are plant cells. Also described are harvestable parts of a plant such as, but not limited to seeds, leaves, fruits, flowers, stem, roots, rhizomes, tubers and bulbs. Furthermore described are products directly derived from a harvestable part of such a plant, such as dry pellets or powders, oil, fat and fatty acids, starch or proteins.

The present specification describes use of *AZ* nucleic acids or variants thereof and use of AZ polypeptides or homologues thereof.

One such use relates to improving the growth characteristics of plants, in particular in improving yield, especially seed yield. The increased seed yield preferably comprises increased thousand kernel weight.

Nucleic acids encoding AZ polypeptides as defined herein may find use in breeding programmes in which a DNA marker is identified which may be genetically linked to an AZ gene. The nucleic acids/ genes may be used to define a molecular marker. This DNA or protein marker may then be used in breeding programmes to select plants having increased yield.

Allelic variants of an AZ nucleic acid/gene as defined herein may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question, for example, different allelic variants of any one of the nucleic acids listed in Table A of Example 1. Growth performance may be monitored in a greenhouse or in the field. Further optional steps include crossing plants, in which the superior allelic variant was identified, with another plant. This could be used, for example, to make a combination of interesting phenotypic features.

An AZ nucleic acid as defined herein may also be used as probes for genetically and physically mapping the genes that they are a part of, and as markers for traits linked to those genes. Such information may be useful in plant breeding in order to develop lines with desired phenotypes. Such use of AZ nucleic acids or variants thereof requires only a nucleic acid sequence of at least 15 nucleotides in length. The AZ nucleic acids or variants thereof may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the AZ nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the AZ nucleic acid in the genetic map previously obtained using this population (Botstein et al. (1980) Am. J. Hum. Genet. 32: 314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

According to the specification the nucleic acid probes may be used in direct fluorescence *in situ* hybridization (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present specification result in plants having increased yield, as described hereinbefore. These advantageous growth characteristics may also be combined with other economically advantageous traits, such as further yield-enhancing traits, tolerance to various stresses, traits modifying various architectural features and/or biochemical and/or physiological features.

## Description of figures

The present invention will now be described with reference to the following figures in which:

Fig. 1 shows an example of the domain structure of an AZ polypeptide. The protein encoded by SEQ ID NO: 2 comprises two ankyrin repeats (bold, underlined) and two C3H1 domains (italics, underlined).

Fig. 2 shows the Ankyrin (ANK) and C3H1 Zinc finger (C3H1) consensus sequences according to the SMART database, the symbols for the various amino acid groups are given in the legend.

Fig. 3 represents a sequence identity/similarity matrix prepared with MATGAT (BLOSUM62, gap opening penalty 11, gap extension penalty 1). Above the diagonal, in bold, the sequence identities are displayed, below the diagonal, sequence similarities are given for: A) full length protein sequences, B) partial protein sequences comprising the most C-terminal putative Zn-finger domain.

Fig. 4 shows the binary vector p056, for expression in *Oryza sativa* of an *Arabidopsis thaliana* AZ coding sequence under the control of a WSI18 promoter (internal reference PRO0151).

## Examples

The present invention will now be described with reference to the following examples.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Identification of sequences related to the nucleic acid sequence used in the methods as described herein*

Sequences (full length cDNA, ESTs or genomic) related to the nucleic acid sequence used in the methods as described were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid as defined herein was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to the nucleic acid sequence used in the methods as described herein.

**Table A:** Examples of AZ polypeptides:

| Plant Source | Nucleic acid SEQ ID NO: | Protein SEQ ID NO: |
|---|---|---|
| *Arabidopsis thaliana* | 1 | 2 |
| *Eucalyptus grandis* | 10 | 11 |
| *Oryza sativa* | 12 | 13 |
| *Medicago truncatula* | 14 | 15 |
| *Oryza sativa* | 16 | 17 |
| *Arabidopsis thaliana* | 18 | 19 |
| *Oryza sativa* | 20 | 21 |
| *Arabidopsis thaliana* | 22 | 23 |
| *Arabidopsis thaliana* | 24 | 25 |
| *Eucalyptus grandis* | 26 | 27 |

(continued)

| Plant Source | Nucleic acid SEQ ID NO: | Protein SEQ ID NO: |
|---|---|---|
| *Eucalyptus grandis* | 28 | 29 |
| *Glycine max* | 30 | 31 |
| *Eucalyptus grandis* | 32 | 33 |
| *Arabidopsis thaliana* | 34 | 35 |
| *Oryza sativa* | 36 | 37 |
| *Hordeum_vulgare* | 38 | 39 |
| *Pinus radiata* | 40 | 41 |
| *Pinus radiata* | 42 | 43 |
| *Glycine max* | | 44 |
| *Glycine max* | | 45 |
| *Glycine max* | | 46 |
| *Arabidopsis thaliana* | | 47 |
| *Arabidopsis thaliana* | | 48 |
| *Arabidopsis fhaliana* | | 49 |
| *Arabidopsis thaliana* | | 50 |
| *Arabidopsis thaliana* | | 51 |
| *Arabidopsis thaliana* | | 52 |
| *Arabidopsis thaliana* | 53 | |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

***Example 2: Gene Cloning of AZ***

The *Arabidopsis AZ* encoding gene (CDS3104) was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and the original number of clones was of $1.59x10^7$ cfu. Original titer was determined to be $9.6x10^5$ cfu/ml, after a first amplification of $6x10^{11}$ cfu/ml. After plasmid extraction, 200 ng of template was used in a 50 $\mu$l PCR mix. Primers prm06717 (sense, AttB1 site in italic, start codon in bold: 5'-*ggggacaagtttgtacaaaaaagcaggcttaaaca***atg**tgctgtggatcagacc-3') (SEQ ID NO 7) and prm06718 (reverse, complementary, AttB2 site in italic: 5'-185 *ggggaccactttgtacaagaaagctgggt*ggttaggtctctcaattctgc-3') (SEQ ID NO 8), which include the AttB sites for Gateway recombination, were used for PCR amplification. PCR was performed using Hifi Taq DNA polymerase in standard conditions. A PCR fragment of the expected size was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p07. Plasmid pDONR201 was purchased from Invitrogen, as part of the Gateway® technology.

***Example 3: Vector Construction***

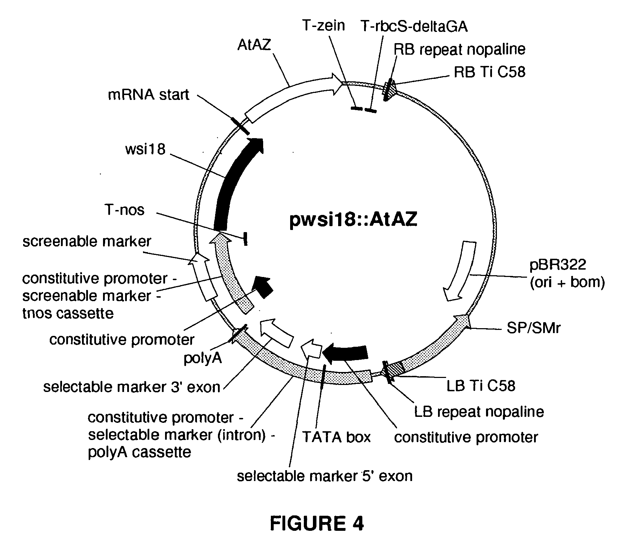

The entry clone p07 were subsequently used in an LR reaction with p02417, a destination vector used for plant (*Oryza sativa)* transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice WSI18 promoter (SEQ ID NO: 9) for seed specific expression (PRO0151) was located upstream of this Gateway cassette (p056, Figure 4).
Many different binary (and super binary) vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of *Agrobacterium tumefaciens.* A plant gene expression cassette consists of at least two genes - a selection marker gene and a plant promoter regulating the transcription of the cDNA or genomic DNA of the trait gene. Various selection marker genes can be used including the *Arabidopsis* gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription.

After the LR recombination step, the resulting expression vector, p056 (Figure 4), was transformed into *Agrobacterium* strain LBA4044 using heat shock or electroporation protocols. Transformed colonies were grown on YEP media and selected by respective antibiotics for two days at 28°C. These *Agrobacterium* cultures were used for the plant transformation.

Other *Agrobacterium tumefaciens* strains can be used for plant transformation and are well known in the art. Examples of such strains are C58C1 or EHA105.

### *Example 4: Plant transformation*

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei et *al.* 1994).

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with Agrobacterium, the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 5: Evaluation setup of AZ expression in rice under the control of the rice WSI18 promoter***

Approximately 15 to 20 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The selected T1 plants were transferred to a greenhouse. Each plant received a unique barcode label to link unambiguously the phenotyping data to the corresponding plant. The selected T1 plants were grown on soil in 10 cm diameter, specially designed pots with transparent bottoms to allow visualization of the roots, under the following environmental settings: photoperiod= 11.5 h, daylight intensity= 30,000 lux or more, daytime temperature= 28°C, night time temperature= 22°C, relative humidity= 60-70%. Transgenic plants and the corresponding nullizygotes were grown side-by-side at random positions. Care was taken that the plants were not subjected to any stress. From the stage of sowing until the stage of maturity the plants were passed several times through a digital imaging cabinet. At each time point digital images (2048x1536 pixels, 16 million colours) were taken of each plant from at least 6 different angles. A digital camera also recorded images through the bottom of the pot during plant growth.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. Root features such as total projected area (which can be correlated to total root volume), average diameter and length of roots above a certain thickness threshold (length of thick roots, or length of thin roots) were deduced from the generated image using appropriate software.

The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of the following seed-related parameters:

The number of filled seeds was determined by counting the number of filled husks that remained after the separation step. The total seed yield (total seed weight) was measured by weighing all filled husks harvested from a plant. Total seed number per plant was measured by counting the number of husks harvested from a plant. The harvest index (HI) in the present invention is defined as the ratio between the total seed yield and the above ground area ($mm^2$), multiplied by a factor $10^6$. Thousand Kernel Weight (TKW) is extrapolated from the number of filled seeds counted and their total weight. The seed fill rate as defined in the present invention is the proportion (expressed as a %) of the number of filled seeds over the total number of seeds (or florets). These parameters were derived in an automated way from the digital images using image analysis software and were analysed statistically. Individual seed parameters (including width, length, area, weight) were measured using a custom-made device consisting of two main components, a weighing and imaging device, coupled to software for image analysis.

A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

The data obtained for *AZ* in the first experiment were confirmed in a second experiment with T2 plants. Four lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.

A total number of 120 AZ transformed plants were evaluated in the T2 generation, that is 30 plants per event of which 15 positives for the transgene, and 15 negatives.

Because two experiments with overlapping events had been carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P-values are obtained by comparing likelihood ratio test to chi square distributions.

### *Example 6: Evaluation* **of** *AZ transformants: measurement of yield-related parameters*

Upon analysis of the seeds as described above, the inventors found that plants transformed with the AZ gene construct had a higher seed yield, expressed as thousand kernel weight, compared to plants lacking the AZ transgene. Furthermore, increased emergence vigour and increased greenness index was observed in plants carrying the transgene compared to the control plants.

For one of the constructs, thousand-kernel weight was increased with 2.7% in the T1 generation. These positive results were again obtained in the T2 generation (increase of 2.1 %). The T2 data were re-evaluated in a combined analysis with the results for the T1 generation, and the obtained p-values showed that the observed effects were highly significant.

## Claims

1. Method for increasing seed yield of plants relative to corresponding wild type plants, comprising introducing and expressing in a plant an AZ nucleic acid or a variant thereof, wherein said AZ nucleic acid encodes an AZ polypeptide or a homologue thereof, wherein the homologue gives plants having increased yield, and wherein said AZ polypeptide or the homologue thereof comprises two ankyrin repeats and two C3H1 Zinc finger domains, and wherein said ankyrin repeats are located upstream of the C3H1 Zinc finger domains.

2. Method according to claim 1, wherein the AZ polypeptide comprises at least one of the following motifs:

   (P/A)CSRAY(S/T)HDWTEC (motif 1, SEQ ID NO: 3)
   HPGENARRRDPR (motif 2, SEQ ID NO: 4)
   HG(V/I)FE(C/S)WLHP(A/S)QY(R/K)TRLCK (motif 3, SEQ ID NO: 5)
   CFFAH (motif 4, SEQ ID NO: 6).

3. Method according to claim 1 or 2, wherein said AZ nucleic acid encodes an AZ polypeptide of SEQ ID NO: 2 or a homologue thereof.

**4.** Method according to any one of claims 1 to 3, wherein said variant is a portion of an AZ nucleic acid or a sequence capable of hybridising to an AZ nucleic acid, which portion or hybridising sequence encodes an AZ polypeptide comprising two ankyrin repeats and two C3H1 Zinc finger domains, and wherein said ankyrin repeats are located upstream of the C3H1 Zinc finger domains

**5.** Method according to any one of claims 1 to 4, wherein said AZ nucleic acid or variant thereof is overexpressed in a plant.

**6.** Method according to any one of claims 1 to 5, wherein said AZ nucleic acid or variant thereof is of plant origin, preferably from a dicotyledonous plant, more preferably from the family Brassicaceae, most preferably the nucleic acid is from Arabidopsis thaliana.

**7.** Method according to any one of claims 1 to 6, wherein said AZ nucleic acid or variant thereof is operably linked to a seed specific promoter.

**8.** Method according to claim 7, wherein said seed-specific promoter is a WSI18 promoter.

**9.** Method according to any one of claims 1 to 8, wherein said increased seed yield comprises increased thousand kernel weight.

**10.** Construct comprising:

(i) an AZ nucleic acid or a variant thereof as defined in any of claims 1 to 4 and 6;
(ii) a seed-specific WSI18 promoter or a constitutive GOS2 promoter operably linked to the nucleic acid sequence of (i)

**11.** Construct according to claim 10, wherein said WSI18 promoter is as represented by SEQ ID NO: 55 or SEQ ID NO: 9.

**12.** Construct according to claim 10, wherein said GOS2 promoter is as represented by SEQ ID NO: 56 or SEQ ID NO: 54.

**13.** Plant transformed with a construct according to any one of claims 10 to 12.

**14.** Method for the production of a transgenic plant having increased seed yield compared to corresponding wild type plants, which method comprises:

(i) introducing and expressing in a plant or plant cell an AZ nucleic acid or variant thereof as defined in any of claims 1 to 4 and 6; and
(ii) cultivating the plant cell under conditions promoting plant growth and development,

**15.** Transgenic plant according to claim 13, wherein said plant is a monocotyledonous plant, such as sugar cane or wherein the plant is a cereal, such as rice, maize, wheat, barley, millet, rye, oats or sorghum.

**16.** Harvestable parts of a plant according to any one of claims 13 or 15, wherein said harvestable parts comprise a construct according to any one of claims 10 to 12.

**17.** Harvestable parts of a plant according to claim 16 wherein said harvestable parts are seeds.

**18.** Products directly derived from a plant according to claim 13 or 15 and/or from harvestable parts of a plant according to claims 16 or 17, wherein said products comprise a construct according to any one of claims 10 to 12.

**19.** Use of an AZ nucleic acid or variant thereof as defined in any of claims 1 to 4 and 6, or use of an AZ polypeptide or a homologue thereof, in increasing seed yield, relative to corresponding wild type plants.

**20.** Use according to claim 19, wherein said increased seed yield comprises increased thousand kernel weight.

**21.** Use of an AZ nucleic acid or variant thereof as defined in any of claims 1 to 4 and 6, or use of an AZ polypeptide or a homologue thereof, as a molecular marker.

22. Use of a construct according to any of claims 10 to 12 in a method for making plants having increased seed yield relative to control plants.

## Patentansprüche

1. Verfahren zum Erhöhen des Samenertrags von Pflanzen im Vergleich zu entsprechenden Pflanzen vom Wildtyp, bei dem man in eine Pflanze eine AZ-Nukleinsäure oder eine Variante davon einbringt und darin exprimiert, wobei die AZ-Nukleinsäure für ein AZ-Polypeptid oder ein Homolog davon codiert, wobei das Homolog Pflanzen mit erhöhtem Ertrag liefert, und wobei das AZ-Polypeptid oder das Homolog davon zwei Ankyrin-Wiederholungseinheiten und zwei C3H1-Zinkfingerdomänen umfasst, und wobei die Ankyrin-Wiederholungseinheiten sich stromaufwärts von den C3H1-Zinkfingerdomänen befinden.

2. Verfahren nach Anspruch 1, wobei das AZ-Polypeptid mindestens eines der folgenden Motive umfasst:

   (P/A)CSRAY(S/T)HDWTEC Motiv 1 SEQ ID NR: 3)
   HPGENARRRDPR (Motiv 2, SEQ ID NR: 4)
   HG(V/I)FE(C/S)WLHP(A/S)QY(R/K)TRLCK (Motiv 3, SEQ ID NR: 5)
   CFFAH (Motiv 4, SEQ ID NR: 6).

3. Verfahren nach Anspruch 1 oder 2, wobei die AZ-Nukleinsäure für ein AZ-Polypeptid der SEQ ID NR: 2 oder ein Homolog davon codiert.

4. Verfahren nach einem der Ansprüche 1 bis 3, wobei es sich bei der Variante um einen Teil einer AZ-Nukleinsäure oder eine zum Hybridisieren mit einer AZ-Nukleinsäure fähige Sequenz handelt, wobei der Teil bzw. die Hybridisierungssequenz für ein AZ-Polypeptid mit zwei Ankyrinwiederholungseinheiten und zwei C3H1-Zinkfingerdomänen codiert, und wobei sich die Ankyrin-Wiederholungseinheiten stromaufwärts von den C3H1-Zinkfingerdomänen befinden.

5. Verfahren nach einem der Ansprüche 1 bis 4, wobei die AZ-Nukleinsäure oder die Variante davon in einer Pflanze überexprimiert wird.

6. Verfahren nach einem der Ansprüche 1 bis 5, wobei die AZ-Nukleinsäure oder die Variante davon pflanzlichen Ursprungs ist, vorzugweise aus einer dikotylen Pflanze, besonders bevorzugt aus der Brassicaceae-Familie; ganz besonders bevorzugt stammt die Nukleinsäure aus Arabidopsis thaliana.

7. Verfahren nach einem der Ansprüche 1 bis 6, wobei die AZ-Nukleinsäure oder die Variante davon funktionell mit einem samenspezifischen Promotor verbunden ist.

8. Verfahren nach Anspruch 7, wobei es sich bei dem samenspezifischen Promotor um einen WSI18-Promotor handelt.

9. Verfahren nach einem der Ansprüche 1 bis 8, wobei der erhöhte Samenertrag ein erhöhtes Tausendkerngewicht umfasst.

10. Konstrukt, umfassend:

    (i) eine wie in einem der Ansprüche 1 bis 4 und 6 definierte AZ-Nukleinsäure oder eine Variante davon;
    (ii) einen samenspezifischen WSI18-Promotor oder einen konstitutiven GOS2-Promotor, der funktionell mit der Nukleinsäuresequenz von (i) verbunden ist.

11. Konstrukt nach Anspruch 10, wobei der WSI18-Promotor wie in SEQ ID NR: 55 oder SEQ ID NR: 9 wiedergegeben ist.

12. Konstrukt nach Anspruch 10, wobei der GOS2-Promotor wie in SEQ ID NR: 56 oder SEQ ID NR: 54 wiedergegeben ist.

13. Pflanze, transformiert mit einem Konstrukt gemäß einem der Ansprüche 10 bis 12.

14. Verfahren zur Herstellung einer transgenen Pflanze mit erhöhtem Samenertrag im Vergleich zu entsprechenden Pflanzen vom Wildtyp, welches Folgendes umfasst:

(i) Einführen einer wie in einem der Ansprüche 1 bis 4 und 6 definierten AZ-Nukleinsäure oder einer Variante davon in eine Pflanze oder Pflanzenzelle und deren Exprimieren darin; und
(ii) Kultivieren der Pflanzenzelle unter das Pflanzenwachstum und die Pflanzenentwicklung fördernden Bedingungen.

**15.** Transgene Pflanze nach Anspruch 13, wobei es sich bei der Pflanze um eine monokotyle Pflanze wie Zuckerrohr handelt oder wobei es sich bei der Pflanze um ein Getreide wie Reis, Mais, Weizen, Gerste, Hirse, Roggen, Hafer oder Sorghum handelt.

**16.** Erntbare Teile einer Pflanze nach einem der Ansprüche 13 oder 15, wobei die erntbaren Teile ein Konstrukt nach einem der Ansprüche 10 bis 12 umfassen.

**17.** Erntbare Teile einer Pflanze nach Anspruch 16, wobei es sich bei den erntbaren Teilen um Samen handelt.

**18.** Produkte, direkt gewonnen aus einer Pflanze nach Anspruch 13 oder 15 und/oder aus erntbaren Teilen einer Pflanze nach Anspruch 16 oder 17, wobei die Produkte ein Konstrukt nach einem der Ansprüche 10 bis 12 umfassen.

**19.** Verwendung einer wie in einem der Ansprüche 1 bis 4 und 6 definierten AZ-Nukleinsäure oder einer Variante davon oder Verwendung eines AZ-Polypeptids oder eines Homologs davon zum Erhöhen des Samenertrags im Vergleich zu entsprechenden Pflanzen vom Wildtyp.

**20.** Verwendung nach Anspruch 19, wobei der erhöhte Samenertrag ein erhöhtes Tausendkerngewicht umfasst.

**21.** Verwendung einer wie in einem der Ansprüche 1 bis 4 und 6 definierten AZ-Nukleinsäure oder einer Variante davon oder Verwendung eines AZ-Polypeptids oder eines Homologs davon als molekularer Marker.

**22.** Verwendung eines Konstrukts nach einem der Ansprüche 10 bis 12 bei einem Verfahren zur Herstellung von Pflanzen mit einem im Vergleich zu Kontrollpflanzen erhöhten Samenertrag.

## Revendications

**1.** Procédé destiné à augmenter le rendement des graines des plantes, par rapport à des plantes de type sauvage correspondantes, comprenant l'introduction et l'expression dans une plante d'un acide nucléique AZ ou d'un variant de celui-ci, dans lequel ledit acide nucléique AZ code pour un polypeptide AZ ou un homologue de celui-ci, dans lequel l'homologue donne des plantes ayant un rendement accru et dans lequel ledit polypeptide AZ ou l'homologue de celui-ci comprend deux répétitions d'ankyrine et deux domaines C3H1 "en doigt de zinc", et dans lequel lesdites répétitions d'ankyrine sont situées en amont des domaines C3H1 "en doigt de zinc".

**2.** Procédé selon la revendication 1, dans lequel le polypeptide AZ comprend au moins un motif parmi les suivantes :

(P/A)CSRAY(S/T)HDWTEC (motif 1, SEQ ID n° : 3)
HPGENARRRDPR (motif 2, SEQ ID n° : 4)
HG(V/I)FE(C/S)WLHP(A/S)QY(R/K)TRLCK (motif 3, SEQ ID n° : 5)
CFFAH (motif 4, SEQ ID n° : 6).

**3.** Procédé selon la revendication 1 ou 2, dans lequel ledit acide nucléique AZ code pour un polypeptide AZ, de SEQ ID n° : 2, ou un homologue de celui-ci.

**4.** Procédé selon l'une quelconque des revendications 1 à 3, dans lequel ledit variant est une partie d'un acide nucléique AZ ou une séquence étant capable de s'hybrider avec un acide nucléique AZ, laquelle partie ou séquence d'hybridation code pour un polypeptide AZ comprenant deux répétitions d'ankyrine et deux domaines C3H1 "en doigt de zinc", et dans lequel lesdites répétitions d'ankyrine sont situées en amont des domaines C3H1 "en doigt de zinc".

**5.** Procédé selon l'une quelconque des revendications 1 à 4, dans lequel ledit acide nucléique AZ ou ledit variant de celui-ci est surexprimé chez une plante.

6. Procédé selon l'une quelconque des revendications 1 à 5, dans lequel ledit acide nucléique AZ ou ledit variant de celui-ci est d'origine végétale, de préférence il provient d'une plante dicotylédone, mieux préféré il provient de la famille des Brassicaceae, de manière préférée entre toutes l'acide nucléique provient d'Arabidopsis thaliana.

7. Procédé selon l'une quelconque des revendications 1 à 6, dans lequel ledit acide nucléique AZ ou ledit variant de celui-ci est lié, de manière opérationnelle, à un promoteur spécifique des graines.

8. Procédé selon la revendication 7, dans lequel ledit promoteur spécifique des graines est un promoteur WSI18.

9. Procédé selon l'une quelconque des revendications 1 à 8, dans lequel ledit rendement accru des graines comprend un poids de mille grains accru.

10. Construit comprenant :

(i) un acide nucléique AZ ou un variant de celui-ci, tel que défini dans l'une quelconque des revendications 1 à 4 et 6 ;
(ii) un promoteur WSI18 spécifique des graines ou un promoteur constitutif GOS2 étant lié, de manière opérationnelle, à la séquence d'acide nucléique de (i).

11. Construit selon la revendication 10, dans lequel ledit promoteur WSI18 est tel que représenté par la SEQ ID n° : 55 ou la SEQ ID n° : 9.

12. Construit selon la revendication 10, dans lequel ledit promoteur GOS2 est tel que représenté par la SEQ ID n° : 56 ou la SEQ ID n° : 54.

13. Plante transformée avec un construit selon l'une quelconque des revendications 10 à 12.

14. Procédé de production d'une plante transgénique ayant un rendement accru des graines, en comparaison avec des plantes de type sauvage correspondantes, lequel procédé comprend les étapes consistant à :

(i) introduire et exprimer dans une plante ou une cellule végétale un acide nucléique AZ ou un variant de celui-ci, tel que défini dans l'une quelconque des revendications 1 à 4 et 6 ; et
(ii) cultiver la cellule végétale dans des conditions qui favorisent la croissance et le développement des plantes.

15. Plante transgénique selon la revendication 13, dans laquelle ladite plante est une plante monocotylédone, telle que la canne à sucre, ou dans laquelle la plante est une céréale, telle que le riz, le maïs, le blé, l'orge, le millet, le seigle, l'avoine ou le sorgho.

16. Parties récoltables d'une plante selon l'une quelconque des revendications 13 ou 15, dans laquelle lesdites parties récoltables comprennent un construit selon l'une quelconque des revendications 10 à 12.

17. Parties récoltables d'une plante selon la revendication 16, dans laquelle lesdites parties récoltables sont des graines.

18. Produits directement dérivés d'une plante, selon la revendication 13 ou 15, et/ou des parties récoltables d'une plante, selon les revendications 16 ou 17, dans laquelle lesdits produits comprennent un construit selon l'une quelconque des revendications 10 à 12.

19. Utilisation d'un acide nucléique AZ ou d'un variant de celui-ci, tel que défini dans l'une quelconque des revendications 1 à 4 et 6, ou utilisation d'un polypeptide AZ ou d'un homologue de celui-ci, dans l'accroissement du rendement des graines par rapport à des plantes de type sauvage correspondantes.

20. Utilisation selon la revendication 19, dans laquelle ledit rendement accru des graines comprend un poids de mille grains accru.

21. Utilisation d'un acide nucléique AZ ou d'un variant de celui-ci, tel que défini dans l'une quelconque des revendications 1 à 4 et 6, ou utilisation d'un polypeptide AZ ou d'un homologue de celui-ci comme marqueur moléculaire.

22. Utilisation d'un construit, selon l'une quelconque des revendications 10 à 12, dans un procédé destiné à élaborer des plantes ayant un rendement accru des graines par rapport à des plantes témoins.

**Domain organisation in SEQ ID NO: 2**

MCCGSDRLNQIVSSRSSLPISFEEDNNLVTNTDMNHLTVETEDTFASLLELAANNDVEGVRLSIER
DPSCVDEAGLWYGRQKGSKAMVN**DYRTPLMVAATYGSIDVIKLIVSLTDADVNR**ACGN**DQTTALHC**
**AASGGAVNAIQVVKLLLAAGADLNL**LDAEGQRAGDVIVVPPKLEGVKLMLQELLSADGSSTAERNL
RVVTNVPNRSSSPCHSPTGENGGSGSGSPLGSPFKLKSTEFKKEYPVDPSLPDIKNSIYATDEFRM
YSFKVRPCSRAYSHDWTECPFVHPGENARRRDPRKF*HYSCVPCPDFRKGACRRGDMCEYAHGV*FEC
WLHPA*QYRTRLCKDGTGCARRVCFFAHTP*EELRPLYASTGSAVPSPRSNADYAAALSLLPGSPSGV
SVMSPLSPSAAGNGMSHSNMAWPQPNVPALHLPGSNLQSSRLRSSLNARDIPTDEFNMLADYEQQQ
LLNEYSNALSRSGRMKSMPPSNLEDLFSAEGSSSPRFTDSALASAVFSPTHKSAVFNQFQQQQQQQ
QSMLSPINTSFSSPKSVDHSLFSGGGRMSPRNVVEPISPMSARVSMLAQCVKQQQQQQQQQQQQHQ
FRSLSSRELRTNSSPIVGSPVNNNTWSSKWGSSNGQPDWGMSSEALGKLRSSSSFDGDEPDVSWVQ
SLVKETPAEAKEKAATSSSGEHVMKQPNPVEPVMDHAGLEAWIEQMQLDQLVAQQN

**FIGURE 1**

```
ANK:
O04242/1-30       NGHTALHIAASK------------------GDEQCVKLLLEHGA------DPNA
CONSENSUS/80%     .t.sslhhsh.t..................tp.phhphllp.t.......pht.
CONSENSUS/65%     pstosLphAstp..................sphphlphLlptss......shsh
CONSENSUS/50%     sGpTsLHhAsps..................sshcllchLlspus......slst

C3H1:
TTP_BOVIN/13-4    RYKTELCRTF---SESG-------RCRYGA-KCQFAHGL
CONSENSUS/80%     t.p...C..a.....tG........C.hu..pC.a.H..
CONSENSUS/65%     p.+..hCpha....tpG.......hCthGs.pCpahHs.
CONSENSUS/50%     ph+.slCctF....ppG.......pCshGs.pCpFtHst
```

Legend:

| Class | Key | Residues |
|---|---|---|
| alcohol | o | S,T |
| aliphatic | l | I,L,V |
| any | . | A,C,D,E,F,G,H,I,K,L,M,N,P,Q,R,S,T,V,W,Y |
| aromatic | a | F,H,W,Y |
| charged | c | D,E,H,K,R |
| hydrophobic | h | A,C,F,G,H,I,K,L,M,R,T,V,W,Y |
| negative | - | D,E |
| polar | p | C,D,E,H,K,N,Q,R,S,T |
| positive | + | H,K,R |
| small | s | A,C,D,G,N,P,S,T,V |
| tiny | u | A,G,S |
| turnlike | t | A,C,D,E,G,H,K,N,Q,R,S,T |

**FIGURE 2**

**MATGAT table: A) full length sequences,**

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1.SEQIDNO2 | | **63.8** | **44** | **58.5** | **54.6** | **49.5** | **43.8** | **38.2** | **36.9** | **38.6** | **37.9** | **35.7** | **38.6** | **38.5** | **35.9** | **27.8** | **48.8** | **43.1** |
| 2.SEQIDNO11 | 78.1 | | **47.2** | **65.7** | **57** | **51.5** | **44** | **39** | **35.8** | **39.6** | **40.8** | **34.4** | **41.5** | **39.7** | **37** | **27.9** | **51.2** | **45.1** |
| 3.SEQIDNO13 | 57.5 | 60.4 | | **45.4** | **48.2** | **39.1** | **39.1** | **37.7** | **37** | **37.2** | **38.1** | **33.5** | **37.1** | **37.1** | **38.2** | **30.2** | **37.9** | **35.2** |
| 4.SEQIDNO15 | 74.3 | 80.1 | 58.3 | | **54.6** | **51.5** | **42.8** | **37.5** | **35.8** | **39.1** | **39.4** | **34.5** | **40.9** | **39.4** | **36.4** | **27** | **50.2** | **43.5** |
| 5.SEQIDNO17 | 69.6 | 72.5 | 57.7 | 68.6 | | **47** | **45.5** | **36.3** | **35.2** | **39.2** | **38.1** | **33.9** | **38.9** | **38.5** | **34.9** | **29.5** | **46.4** | **41.7** |
| 6.SEQIDNO19 | 68.7 | 70.5 | 54.7 | 69.7 | 62.9 | | **43.3** | **37.4** | **35.3** | **36.9** | **38.9** | **33.8** | **38.3** | **38.9** | **35** | **27.5** | **42.6** | **38.7** |
| 7.SEQIDNO21 | 57.4 | 60.8 | 54.5 | 59.3 | 57.9 | 58.9 | | **36.6** | **34.9** | **36.3** | **36.7** | **33.6** | **35.6** | **36.4** | **37.5** | **28.1** | **39.7** | **37.3** |
| 8.SEQIDNO23 | 52.1 | 54.3 | 54.3 | 52.1 | 49.3 | 53.7 | 52.5 | | **68.3** | **50.4** | **48.1** | **45** | **44.5** | **46.8** | **39.9** | **32.1** | **36.7** | **34.9** |
| 9.SEQIDNO25 | 49.9 | 50.9 | 53 | 51.6 | 48.2 | 52.1 | 50.7 | 81.7 | | **49.6** | **47** | **45.3** | **43.2** | **46.6** | **40.1** | **30.9** | **35.8** | **34** |
| 10.SEQIDNO27 | 54.9 | 55.5 | 52.8 | 54.6 | 52.9 | 55 | 53.9 | 65.6 | 63 | | **63.3** | **54.7** | **53.8** | **51** | **45.8** | **31.5** | **40.7** | **38.5** |
| 11.SEQIDNO29 | 54.5 | 57.7 | 53.2 | 58.2 | 52.5 | 55.5 | 54 | 65.9 | 62.7 | 78 | | **53.7** | **54.1** | **47.3** | **41.7** | **31** | **40.3** | **37.6** |
| 12.SEQIDNO31 | 52.2 | 51.7 | 49.8 | 53.8 | 49.1 | 51.7 | 52.8 | 60 | 60 | 68.4 | 69 | | **44.7** | **42.2** | **39.3** | **30.9** | **34.4** | **32.3** |
| 13.SEQIDNO33 | 56.1 | 58.8 | 52.4 | 59.1 | 53.8 | 57.9 | 53.2 | 61 | 59.2 | 69.5 | 69.9 | 62.7 | | **48.7** | **38.3** | **29.8** | **40.8** | **39.2** |
| 14.SEQIDNO35 | 53.4 | 54.3 | 55.4 | 53.4 | 52.1 | 55.5 | 54.9 | 63.4 | 63.9 | 63.6 | 62.9 | 58 | 62.1 | | **42.4** | **31.1** | **38** | **38.1** |
| 15.SEQIDNO37 | 50.3 | 50.9 | 55.3 | 51.1 | 47.5 | 49.9 | 53.3 | 58.7 | 57.2 | 60.1 | 57.3 | 55.8 | 55 | 60 | | **39.8** | **34.5** | **33.9** |
| 16.SEQIDNO39 | 41.8 | 40.6 | 43.8 | 41.1 | 39.5 | 41.2 | 43.1 | 49.4 | 49 | 46.6 | 46 | 43.8 | 44.1 | 47.1 | 53.7 | | **28.1** | **26.7** |
| 17.SEQIDNO41 | 63.9 | 65.5 | 50.6 | 64.8 | 64.1 | 58.5 | 53.3 | 50.2 | 48.1 | 54.8 | 54.4 | 51.3 | 56 | 50.2 | 48.5 | 38.1 | | **54.8** |
| 18.SEQIDNO43 | 58.3 | 61.5 | 50.3 | 59.9 | 60.5 | 57 | 52.7 | 50.3 | 49.5 | 53.3 | 54.7 | 48.3 | 56.6 | 53.4 | 48.9 | 38.1 | 67.3 | |

**FIGURE 3 A**

B) Zn-finger domain sequences

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1.SEQIDNO2 | | 92.0 | 96.0 | 88.0 | 88.0 | 84.0 | 72.0 | 88.0 | 80.0 | 80.0 | 88.0 | 88.0 | 76.0 | 84.0 | 84.0 | 76.0 | 88.0 | 92.0 |
| 2.SEQIDNO11 | 92.0 | | 88.0 | 80.0 | 92.0 | 80.0 | 76.0 | 84.0 | 76.0 | 76.0 | 80.0 | 80.0 | 72.0 | 80.0 | 76.0 | 72.0 | 88.0 | 88.0 |
| 3.SEQIDNO13 | 96.0 | 88.0 | | 88.0 | 84.0 | 84.0 | 72.0 | 84.0 | 76.0 | 76.0 | 84.0 | 84.0 | 76.0 | 80.0 | 88.0 | 80.0 | 84.0 | 88.0 |
| 4.SEQIDNO15 | 92.0 | 84.0 | 96.0 | | 84.0 | 92.0 | 68.0 | 80.0 | 72.0 | 76.0 | 80.0 | 80.0 | 76.0 | 80.0 | 80.0 | 76.0 | 80.0 | 84.0 |
| 5.SEQIDNO17 | 88.0 | 92.0 | 84.0 | 88.0 | | 84.0 | 68.0 | 80.0 | 72.0 | 76.0 | 76.0 | 76.0 | 72.0 | 80.0 | 72.0 | 72.0 | 88.0 | 88.0 |
| 6.SEQIDNO19 | 84.0 | 80.0 | 88.0 | 96.0 | 84.0 | | 68.0 | 80.0 | 72.0 | 76.0 | 76.0 | 76.0 | 76.0 | 76.0 | 76.0 | 76.0 | 80.0 | 80.0 |
| 7.SEQIDNO21 | 84.0 | 92.0 | 84.0 | 80.0 | 84.0 | 80.0 | | 68.0 | 60.0 | 60.0 | 64.0 | 64.0 | 60.0 | 64.0 | 72.0 | 68.0 | 68.0 | 68.0 |
| 8.SEQIDNO23 | 88.0 | 84.0 | 84.0 | 80.0 | 80.0 | 80.0 | 80.0 | | 88.0 | 84.0 | 88.0 | 88.0 | 80.0 | 84.0 | 84.0 | 80.0 | 80.0 | 80.0 |
| 9.SEQIDNO25 | 88.0 | 84.0 | 84.0 | 80.0 | 80.0 | 80.0 | 80.0 | 100.0 | | 88.0 | 92.0 | 92.0 | 84.0 | 80.0 | 80.0 | 72.0 | 72.0 | 72.0 |
| 10.SEQIDNO27 | 84.0 | 80.0 | 80.0 | 80.0 | 84.0 | 84.0 | 76.0 | 92.0 | 92.0 | | 92.0 | 92.0 | 92.0 | 84.0 | 80.0 | 76.0 | 80.0 | 76.0 |
| 11.SEQIDNO29 | 92.0 | 84.0 | 88.0 | 84.0 | 80.0 | 80.0 | 80.0 | 96.0 | 96.0 | 92.0 | | 100.0 | 88.0 | 88.0 | 88.0 | 76.0 | 76.0 | 80.0 |
| 12.SEQIDNO31 | 92.0 | 84.0 | 88.0 | 84.0 | 80.0 | 80.0 | 80.0 | 96.0 | 96.0 | 92.0 | 100.0 | | 88.0 | 88.0 | 88.0 | 76.0 | 76.0 | 80.0 |
| 13.SEQIDNO33 | 80.0 | 76.0 | 84.0 | 84.0 | 76.0 | 88.0 | 76.0 | 88.0 | 88.0 | 92.0 | 88.0 | 88.0 | | 80.0 | 80.0 | 76.0 | 72.0 | 72.0 |
| 14.SEQIDNO35 | 88.0 | 88.0 | 84.0 | 84.0 | 88.0 | 80.0 | 80.0 | 92.0 | 92.0 | 92.0 | 96.0 | 96.0 | 88.0 | | 84.0 | 76.0 | 88.0 | 92.0 |
| 15.SEQIDNO37 | 88.0 | 80.0 | 92.0 | 88.0 | 76.0 | 84.0 | 80.0 | 92.0 | 92.0 | 88.0 | 96.0 | 96.0 | 92.0 | 92.0 | | 88.0 | 72.0 | 76.0 |
| 16.SEQIDNO39 | 80.0 | 76.0 | 84.0 | 84.0 | 76.0 | 84.0 | 76.0 | 84.0 | 84.0 | 88.0 | 84.0 | 84.0 | 88.0 | 80.0 | 88.0 | | 72.0 | 72.0 |
| 17.SEQIDNO41 | 92.0 | 96.0 | 88.0 | 88.0 | 100.0 | 88.0 | 84.0 | 84.0 | 84.0 | 88.0 | 84.0 | 84.0 | 80.0 | 88.0 | 80.0 | 80.0 | | 96.0 |
| 18.SEQIDNO43 | 96.0 | 96.0 | 92.0 | 92.0 | 96.0 | 84.0 | 84.0 | 84.0 | 84.0 | 84.0 | 88.0 | 88.0 | 80.0 | 92.0 | 84.0 | 76.0 | 96.0 | |

FIGURE 3 B

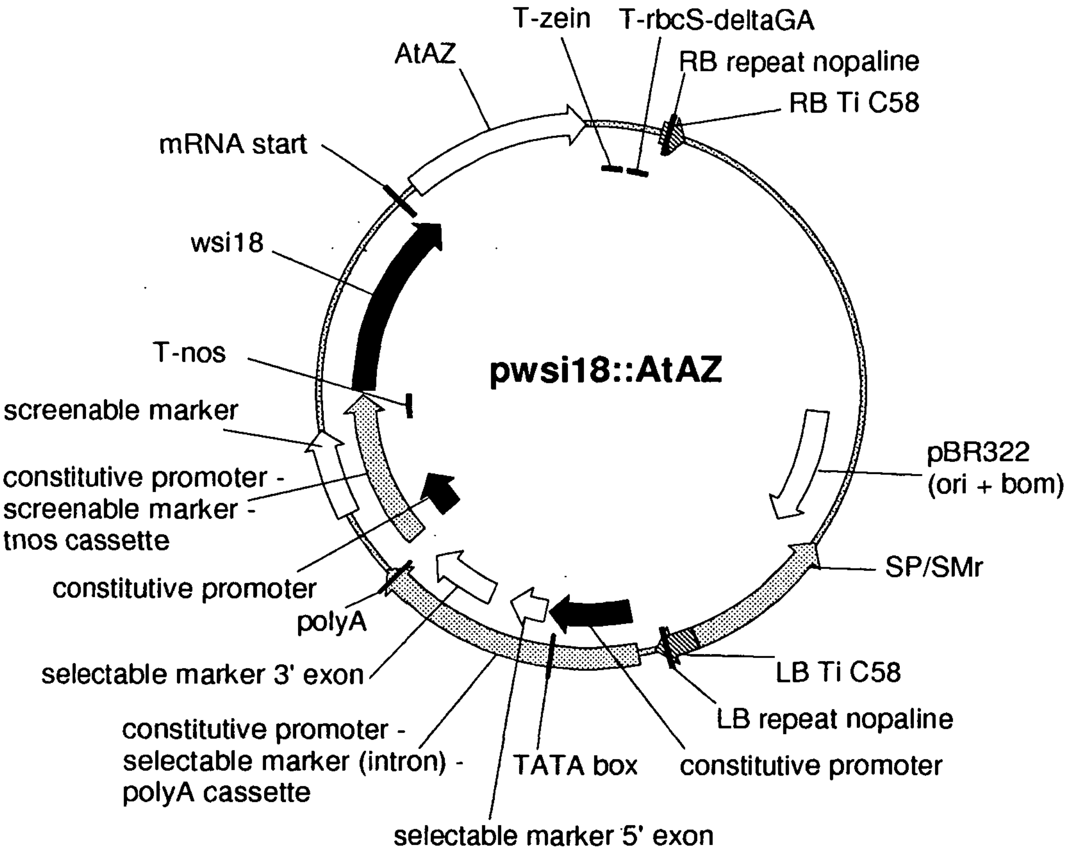
T-zein
T-rbcS-deltaGA
AtAZ
RB repeat nopaline
RB Ti C58
mRNA start
wsi18
T-nos
pwsi18::AtAZ
screenable marker
pBR322
(ori + bom)
constitutive promoter -
screenable marker -
tnos cassette
SP/SMr
constitutive promoter
polyA
LB Ti C58
selectable marker 3' exon
LB repeat nopaline
constitutive promoter -
selectable marker (intron) -
polyA cassette
TATA box
constitutive promoter
selectable marker 5' exon

**FIGURE 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0244389 A
- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5352605 A
- WO 8402913 A
- EP 388186 A
- EP 335528 A
- WO 9706268 A
- WO 9519443 A
- WO 9321334 A
- US 5187267 A
- WO 9612814 A
- EP 0375091 A
- US 5608152 A
- WO 9845461 A
- US 5504200 A
- WO 9113980 A
- WO 0026388 A
- WO 9515389 A
- WO 9523230 A
- WO 9916890 A
- US 5677474 A
- US 5530149 A
- EP 571741 A
- JP 6062870 A
- WO 9808962 A
- US 5689040 A
- EP 781849 A
- DE 19644478 A
- EP 0249676 A
- US 57673666 B
- US 6225105 B
- US 5164310 A


### Non-patent literature cited in the description


- **WANG et al.** *Planta,* 2003, vol. 218, 1-14
- **TITTONELL et al.** *Agric Ecosys & Environ,* 2005, vol. 105, 213
- **FASOULA ; TOLLENAAR.** *Maydica,* 2005, vol. 50, 39
- **STEEGE et al.** *Plant Physiology,* 2005, vol. 139, 1078
- **HITTALMANI et al.** *Theoretical Applied Genetics,* 2003, vol. 107, 679
- **REBETZKE et al.** *Crop Science,* 2002, vol. 42, 739
- **GARDENER et al.** Physiology of Crop Plants. Iowa State University Press, 1985, 68-73
- **STEGMAIER et al.** *Genome informatics,* 2004, vol. 15, 276-286
- **LI ; THOMAS.** *Plant Cell,* 1998, vol. 10, 383-398
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994

- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **TAKAIWA et al.** *FEBS Letts.,* 1987, vol. 221, 43-47
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **SELINGER et al.** *Genetics,* 1994, vol. 149, 1125-38
- **BUCHMAN ; BERG.** *Mol. Cell biol.,* 1988, vol. 8, 4395-4405

- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER.** *Bioassays,* 1992, vol. 14, 807-15
- **SCHWAB et al.** *Dev. Cell,* 2005, vol. 8, 517-527
- **SCHWAB et al.** *Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **RABBANI et al.** *Plant Physiol,* 2003, vol. 133, 1755-1767
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAIPress, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280

- **GASTEIGER et al.** ExPASy: the proteomics server for in-depth protein knowledge and analysis. *Nucleic Acids Res.,* 2003, vol. 31, 3784-3788
- **BATEMAN et al.** *Nucl. Acids Res.,* 2004, vol. 32, D138-141
- **MULDER et al.** *Nucl. Acids. Res.,* 2005, vol. 33, D201-205
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **FIELDS ; SONG.** *Nature,* 1989, vol. 340, 245-6
- Current Protocols in Molecular Biology
- **BENFEY et al.** *EMBO J.,* 1989, vol. 8, 2195-2202
- **FRANCK et al.** *Cell,* 1980, vol. 21, 285-294
- **WARD et al.** *Plant. Mol. Biol.,* 1993, 22
- **LEISNER.** *Proc Natl Acad Sci USA,* 1988, vol. 85 (5), 2553-2557
- **COMAI.** *Plant Mol Biol,* 1990, vol. 15 (3), 373-381
- **SCHENK.** *Plant Mol Biol,* 1999, vol. 39 (6), 1221-1230
- **JAIN et al.** *Crop Science,* 1999, vol. 39 (6), 1696-1701
- **SHAW et al.** *Nucleic Acids Res.,* 1994, vol. 12 (20), 7831-7846
- **GATZ et al.** *Plant J.,* 1992, vol. 2, 397-404
- **WARD et al.** *Plant. Mol. Biol.,* 1993, vol. 22, 361-366
- **BAEUMLEIN et al.** *Mol Gen Genet,* 1991, vol. 225 (3), 459-67
- **BAEUMLEIN et al.** *Plant Journal,* 1992, vol. 2 (2), 233-9
- **STOCKHAUS et al.** *EMBO J.,* 1989, vol. 8, 2445
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- Plant Molecular Biology Labfax. BIOS Scientific Publications Ltd (UK) and Blackwell Scientific Publications (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **AN, G.** Agrobacterium Protocols. Methods in Molecular Biology. Humana Press, vol. 44, 47-62
- **BEVAN.** *Nucleic Acid Research,* 1984, vol. 12, 8711-8721
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659

SEQUENCE LISTING

<110> CropDesign N.V.

<120> Plants having increased yield and method for making the same

<130> PF58401-prio

<160> 347

<170> PatentIn version 3.3

<210> 1
<211> 2151
<212> DNA

<213> Arabidopsis thaliana

<400> 1

```
atgtgctgtg gatcagaccg attaaaccag atcgtgtcat caagatcttc gttgccaatt     60
tctttcgagg aagataacaa tcttgttacc aacacagaca tgaatcactt aacagtcgaa    120
acagaggata cgtttgcgag cttgcttgag cttgcagcta acaacgatgt tgaaggtgta    180
aggctatcta tcgagagaga cccttcttgt gtagacgaag ctggtctctg gtacggtcgt    240
caaaaaggtt ctaaagctat ggtcaacgat tacaggactc cgttgatggt tgctgctact    300
tacggaagca ttgatgtgat caagcttatt gtttctttga ctgatgctga cgtgaaccgt    360
gcttgcggga atgatcagac cactgcgtta cactgcgctg cttctggagg agctgtgaat    420
gctatccaag ttgttaagct gcttcttgca gctggagctg atttgaatct gttggatgct    480
gaaggtcaac gagctggtga tgttattgtt gttcctccta agcttgaagg cgtgaagctg    540
atgcttcagg agcttctttc tgctgatgga tcatctactg cggagcggaa tctacgggtt    600
gtgacaaatg ttccgaatag aagctcatct ccgtgtcatt ctcctactgg agagaatggt    660
ggatcagggt ctggttcacc gctcggctct ccttttaagc tgaaatctac tgaattcaag    720
aaagagtatc cggttgatcc gtctttgcca gatatcaaga acagtatcta cgcgactgat    780
gagtttagaa tgtattcctt caaggtccgg ccttgctctc gtgcttattc acatgattgg    840
actgagtgtc cttttgttca cccgggtgaa aacgcgagga ggagagaccc gaggaagttc    900
cattacagct gcgttccttg cccggatttt aggaaaggag cttgtaggag aggagatatg    960
tgtgagtatg cgcacggtgt gtttgaatgc tggcttcatc cggctcagta caggacccgt   1020
ctttgcaaag atggaacagg ctgtgctcgg cgggtttgtt tctttgcgca tacacccgag   1080
gagcttcgac ctttgtacgc atcaactggt tcagcggttc cttcgcctag atcgaatgct   1140
gattatgcag ctgctttgag tctccttcct ggttctccat caggagtctc tgtcatgtcc   1200
ccgctttccc catcagcagc ggggaacgga atgtctcatt cgaatatggc ttggccacaa   1260
ccaaatgtcc ctgcgttgca cttaccagga agcaatctac agtcaagcag gctaaggtct   1320
tctctcaatg caagggatat cccgacggat gagttcaata tgttagcgga ttacgagcag   1380
cagcaactcc tcaacgagta ttccaatgct ctgagccgtt ctggtcggat gaaatcaatg   1440
cctccttcga atcttgaaga tcttttctca gcagaaggct cttcatctcc ccggttcact   1500
gattccgctt tagcttccgc ggtgttctcg cctacacaca agtcagctgt cttcaaccag   1560
ttccaacaac agcaacagca gcagcagagc atgttgtctc caatcaacac aagcttttct   1620
tcaccaaaga gcgttgacca ctcattgttt tcaggtggag gaagaatgtc tcctcggaat   1680
gttgttgaac caatatcacc catgagtgct cgggtttcca tgttggctca gtgcgtgaag   1740
caacaacaac agcaacagca gcagcagcag cagcaacatc agttccgtag ccttagctcc   1800
agagagctca gaacaaactc tagcccaatc gttggttcac cggtaaacaa caacacatgg   1860
tcatcaaaat ggggatcttc aaatggtcaa ccggattggg gaatgagctc agaagcactt   1920
ggtaagttga gatcttcgtc atcgtttgat ggtgatgagc ctgatgtgtc atgggtccag   1980
tcactggtga aggagactcc agcagaagcc aaagagaaag cagcaacatc ttcctcaggg   2040
gaacacgtga tgaagcagcc aaatccggtt gaaccggtaa tggatcatgc tgggctagaa   2100
gcttggattg agcaaatgca gctcgatcag cttgtggctc agcagaattg a             2151
```

<210> 2
<211> 716
<212> PRT
<213> Arabidopsis thaliana

<400> 2

```
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
            20                  25                  30
Asp Met Asn His Leu Thr Val Glu Thr Glu Asp Thr Phe Ala Ser Leu
        35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu
```

```
    450                 455                 460
Asn Glu Tyr Ser Asn Ala Leu Ser Arg Ser Gly Arg Met Lys Ser Met
465                 470                 475                 480
Pro Pro Ser Asn Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Ser Met Leu Ser Pro Ile Asn Thr Ser Phe Ser Ser Pro Lys Ser
    530                 535                 540
Val Asp His Ser Leu Phe Ser Gly Gly Gly Arg Met Ser Pro Arg Asn
545                 550                 555                 560
Val Val Glu Pro Ile Ser Pro Met Ser Ala Arg Val Ser Met Leu Ala
                565                 570                 575
Gln Cys Val Lys Gln Gln Gln Gln Gln Gln Gln Gln Gln Gln Gln Gln
            580                 585                 590
His Gln Phe Arg Ser Leu Ser Ser Arg Glu Leu Arg Thr Asn Ser Ser
        595                 600                 605
Pro Ile Val Gly Ser Pro Val Asn Asn Asn Thr Trp Ser Ser Lys Trp
    610                 615                 620
Gly Ser Ser Asn Gly Gln Pro Asp Trp Gly Met Ser Ser Glu Ala Leu
625                 630                 635                 640
Gly Lys Leu Arg Ser Ser Ser Ser Phe Asp Gly Asp Glu Pro Asp Val
                645                 650                 655
Ser Trp Val Gln Ser Leu Val Lys Glu Thr Pro Ala Glu Ala Lys Glu
            660                 665                 670
Lys Ala Ala Thr Ser Ser Ser Gly Glu His Val Met Lys Gln Pro Asn
        675                 680                 685
Pro Val Glu Pro Val Met Asp His Ala Gly Leu Glu Ala Trp Ile Glu
    690                 695                 700
Gln Met Gln Leu Asp Gln Leu Val Ala Gln Gln Asn
705                 710                 715
```

<210> 3
<211> 13
<212> PRT
<213> Artificial sequence

<220>
<223> motif 1

<220>
<221> VARIANT
<222> (1) .. (1)
<223> \replace = "Ala"

<220>
<221> VARIANT
<222> (7) .. (7)
<223> \replace = "Thr"

<400> 3

```
Pro Cys Ser Arg Ala Tyr Ser His Asp Trp Thr Glu Cys
1               5                   10
```

<210> 4
<211> 12

<212> PRT
<213> Artificial sequence

<220>
<223> motif 2

<400> 4

```
His Pro Gly Glu Asn Ala Arg Arg Arg Asp Pro Arg
1               5                   10
```

<210> 5
<211> 19
<212> PRT
<213> Artificial sequence

<220>
<223> motif 3

<220>
<221> VARIANT
<222> (3) .. (3)
<223> \replace = "Ile"

<220>
<221> VARIANT
<222> (6)..(6)
<223> \replace = "Ser"

<220>
<221> VARIANT
<222> (11)..(11)
<223> \replace = "Ser"

<220>
<221> VARIANT
<222> (14)..(14)
<223> \replace = "Lys"

<400> 5

```
His Gly Val Phe Glu Cys Trp Leu His Pro Ala Gln Tyr Arg Thr Arg
1               5                   10                  15
Leu Cys Lys
```

<210> 6
<211> 5
<212> PRT
<213> Artificial sequence

<220>
<223> motif 4

<400> 6

```
Cys Phe Phe Ala His
1               5
```

<210> 7
<211> 54
<212> DNA
<213> Artificial sequence

<220>
<223> primer: prm06717

<400> 7
ggggacaagt ttgtacaaaa aagcaggctt aaacaatgtg ctgtggatca gacc 54

<210> 8
<211> 50
<212> DNA
<213> Artificial sequence

<220>
<223> primer: prm06718

<400> 8
ggggaccact ttgtacaaga aagctgggtg gttaggtctc tcaattctgc 50

<210> 9
<211> 981
<212> DNA
<213> Oryza sativa

<400> 9

```
aagaggcaag agcatccgta ttaaccagcc ttttgagact tgagagtgtg tgtgactcga      60
tccagcgtag tttcagttcg tgtgttggtg agtgattcca gccaagtttg cgatggcttc     120
tcagcaggaa cgggctagct accacgccgg cgagaccaag gcccgcgccg aggagaagac     180
ggggcgcatg atgggcacgg cgcaggagaa ggcgcgggag gccaaggaca cggcgtccga     240
cgccgcgggg cgcgcgatgg gcaggggaca cggcgccaag gaggcgacca aggagaaggc     300
gtacgagacc aaggacgcga ccaaggagaa ggcgtacgag gcaaaggacg cggcctccga     360
cgccaccggc cgcgccatgg acaagggccg cggcgccgcg ggcgccacga gggacaaggc     420
gtacgatgcc aaggacaggg cggctgacac ggcgcagtcc gccgccgacc gcgcccgcga     480
cggcgccggg cagaccggga gctacattgg acagaccgcc gaggccgcca agcagaaagc     540
ggccggcgcc gcgcagtacg ccaaggagac cgcgatcgcc ggcaaggaca agaccggcgc     600
cgtgctccag caggcagggg agcaggtgaa gagcgtggcg gtgggggcga aggacgcggt     660
gatgtacacg ctcgggatgt caggcgataa caagaacaac gccgctgccg gcaaggacac     720
cagcacctac aagcctggaa ctgggagtga ctaccagtaa tacggtagaa gaagcatgtg     780
tcgtctttgg cactgatgcc aaagtgtacg tgttgtatcc tctttttaa gtttcagctc      840
gacttcgacg tgttcggtgt cacactttgg tttttcagtt gtgctcaact gttcatgttt     900
ctggttccat ggagggccag tgtggaggtc aatgtttaag ctttcgtttt aaaatctgat     960
aataaagttg gttaagacct g                                               981
```

<210> 10
<211> 3372
<212> DNA
<213> Eucalyptus grandis

<400> 10

```
ggaagacgaa gagcaacaaa ataggtctca ctccctcctc tctcctctct cctctctctt     60
ctttctctct cttctgcttc taacaaagtc tcttccttga gagacagggc tgcgtcgtcg    120
tctttctctc tcctcgctgc gagcttctga gaaagttcaa ttctttttct cttgttctct    180
ctctctaccc ttctgggtac cactgtgaag ctccggtctt ttctattttt tttttttttg    240
ggctatctgg gtctgggcaa atccatcgcg cgctctgctc tggactgaga ggccgtcagt    300
ggctttagat ctgcgacgcc tcttgcttgc tcagtgagct gggctagttc aaatcgacga    360
agaaagcatg cgctagtgat tggtgtgggt aatacactgc attcgatctc tactaagtat    420
ccccaagtat actaagatcc cgttctcagc catgagtcaa ctgaccattc agactgagga    480
cacttttgcc agcttgcttg agcttgctgc taacaacgac acagaatctt tcggacggtg    540
tgtggaacgt gatccttcga gcatagatga aattggatat tggtatggtc gccaaaaggg    600
ttcgaagcag gtggtcaata tgcaaagaac tcctcttatg gtggctgcta catatggtag    660

tgttgatgta atgagactca ttctttgcct atctgatgct gatgtgaatc gaacctgcag    720
cacagacaag agcacagccc ttcactgtgc tgcctctggt ggtgctgtga atgctgtaga    780
tgctgtgagg ctactcctgt cagctggtgc tgacccaagt ttagcagatg ctaacggtca    840
gcggcctgtg gatgttattg ttgttcctcc aaagctcctt tcaataaagt ttgctcttga    900
agagctcttg tcgaccgaag gatctgtaaa tgaacacaat ctgagagtgt ccgtagccac    960
ttccaattca acctctcccc cactttcatc ttccccggat aatggttccc cagcatctgc   1020
taattgttct tcccccaaga actcaaagtt aagtgatgcc cctgttcttt atgcatcaga   1080
aaagaaggaa tacccggtgg atccatctct tccagatatc aagaatagca tttactcaac   1140
agatgaattc cgaatgtatt cttttaaagt gcggccttgt tcacgagcgt actcgcatga   1200
ttggacggag tgcccttttg ttcatccagg ggagaatgcc cgtagaaggg atccaaggaa   1260
gttccactac agctgtgtcc cttgccctga tttccggaag ggtgcttgta gacgtggaga   1320
tatgtgtgaa tatgctcatg gtgtttttga gtgctggctc catcctgctc agtatcggac   1380
tcgattatgc aaggatggta caagttgtgc tcggagagtg tgcttctttg cccacacgga   1440
gcaagagctg cgtccattgt acgtctccac tggttctgct gttccgtctc ctcgctcgag   1500
tacctctgga gctgctgcca tggattttgc tgcagccatg agcctcttac ctggttcccc   1560
atcatcagta tccatcatgt cccccttcacc cttcactcct cccatgtctc catctgctaa   1620
tggtatttct cacccatctg ttgcctggcc ccagcaaaat gtaccaactt tgcatcttcc   1680
cggaagcaat cttcagtcca gccgcttgag atcttctctt aatgcaagag atattcctca   1740
ggaggatttt gacttgctgt cagattatga tgtgcaacag cagcagctcc taaatgagtt   1800
ttccatcctt tcacaacaat cgatgggtgc taattccttg aaccgttctg gtcggctgaa   1860
aactttgacc ccctcaaacc ttgatgatct cttctctgct gagagctcat cccctcgcta   1920
cgctgatcaa gccctggctt ctgctgtttt ttcaccaacg cacaaatctg cagtaatcaa   1980
tcaatttcag cagcagcagc agagcatgtt atcacccatc aacacaacct tctctcctaa   2040
gagtgtcgac cacccctttgt tgcaagcgtc tttcggtgtt caatctgggc gaatgtcccc   2100
tcgtaacatg gatcccatct ctcctataag ttctcgtgtg tcgatgttgg cccaacgaga   2160
gaaacagcaa cagcaattac gcagcctaag ctctcgtgaa ctcggttcca attcagccgc   2220
cattgtgggt tcccccgtgg gttcttggtc gaaatgggga gctacaaatg ggaaaccaga   2280
ctgggctgtt agtgcagatg aactaggtaa gcttcgcagg tctaattcat ttgagcttgg   2340
gaacaatggt gaggagccag atctttcatg ggttcaatcc ctcgttaaag aatctcctac   2400
cgagatgaaa gaaaagcttt cgtcaactct ctctggtgtt ccagcccccg ctacatccag   2460
tgaggttccg agtatcagct cgcagatgga atcggttgat cacgaagtgc taggagcatg   2520
gctccagcag atgcagctcg atccgctcgt ggctcagcaa aactaggttg tttttttttcc  2580
tacatggcct tgaggaagta gacagcggaa agtttttttt ggtaaatact atgtttttttc  2640
tggaaatttt tgatgctggg ggtggggtct ggaagaagat aacaaggcag gaaaggggtc   2700
agtgaagtca ctggagaaaa ggaattcatt tttaaccatt ttatcattct attacaacag   2760
aaagtaggga aaaaaaagga agaccctctg ggttatgaag agaaattaaa cccaggctag   2820
gcgttctcct ttctaatatt tccaatttta ggtccatatt actgtcattt cctttttgcc   2880
gtcttatcat atttcatcaa aatggaactg gggactaatg tttgttccat tctttcgctc   2940
ttctgattta tttgcaccct tggggtaaga tcaaaagaga aattatgatc attttctttt   3000
gaggatattt tttttcccca atatttgtga gaatgaaagt taagagggga tatgatgtgt   3060
ctggtgttgt agtatgaaaa accaataacc gagttcacct gttgctgctg gtggtagaag   3120
aagtggagaa gaagctatga tcctttgatg taacagtcaa tcaaacattt taatactttt   3180
attttttgtt tcctcatgta atccatcctt tgtgattgtc ctctctctct ctctctctct   3240
ctctctctcc ctccccgtgt tctttcttca taagcgtctt gcttgtcgat ctgtaaatta   3300
ttgaaaaggg tcatggaaag ccgtgccggt gtggattctc atttttgcaa aaaaaaaaaa   3360
aaaaaaaaaa aa                                                       3372
```

<210> 11
<211> 704
<212> PRT
<213> Eucalyptus grandis

<400> 11

```
Met Ser Gln Leu Thr Ile Gln Thr Glu Asp Thr Phe Ala Ser Leu Leu
1               5                   10                  15
Glu Leu Ala Ala Asn Asn Asp Thr Glu Ser Phe Gly Arg Cys Val Glu
            20                  25                  30
Arg Asp Pro Ser Ser Ile Asp Glu Ile Gly Tyr Trp Tyr Gly Arg Gln
        35                  40                  45
```

```
Lys Gly Ser Lys Gln Val Val Asn Met Gln Arg Thr Pro Leu Met Val
    50                  55                  60
Ala Ala Thr Tyr Gly Ser Val Asp Val Met Arg Leu Ile Leu Cys Leu
65                  70                  75                  80
Ser Asp Ala Asp Val Asn Arg Thr Cys Ser Thr Asp Lys Ser Thr Ala
                85                  90                  95
Leu His Cys Ala Ala Ser Gly Gly Ala Val Asn Ala Val Asp Ala Val
            100                 105                 110
Arg Leu Leu Leu Ser Ala Gly Ala Asp Pro Ser Leu Ala Asp Ala Asn
        115                 120                 125
Gly Gln Arg Pro Val Asp Val Ile Val Val Pro Pro Lys Leu Leu Ser
    130                 135                 140
Ile Lys Phe Ala Leu Glu Glu Leu Leu Ser Thr Glu Gly Ser Val Asn
145                 150                 155                 160
Glu His Asn Leu Arg Val Ser Val Ala Thr Ser Asn Ser Thr Ser Pro
                165                 170                 175
Pro Leu Ser Ser Ser Pro Asp Asn Gly Ser Pro Ala Ser Ala Asn Cys
            180                 185                 190
Ser Ser Pro Lys Asn Ser Lys Leu Ser Asp Ala Pro Val Leu Tyr Ala
        195                 200                 205
Ser Glu Lys Lys Glu Tyr Pro Val Asp Pro Ser Leu Pro Asp Ile Lys
    210                 215                 220
Asn Ser Ile Tyr Ser Thr Asp Glu Phe Arg Met Tyr Ser Phe Lys Val
225                 230                 235                 240
Arg Pro Cys Ser Arg Ala Tyr Ser His Asp Trp Thr Glu Cys Pro Phe
                245                 250                 255
Val His Pro Gly Glu Asn Ala Arg Arg Arg Asp Pro Arg Lys Phe His
            260                 265                 270
Tyr Ser Cys Val Pro Cys Pro Asp Phe Arg Lys Gly Ala Cys Arg Arg
        275                 280                 285
Gly Asp Met Cys Glu Tyr Ala His Gly Val Phe Glu Cys Trp Leu His
    290                 295                 300
Pro Ala Gln Tyr Arg Thr Arg Leu Cys Lys Asp Gly Thr Ser Cys Ala
305                 310                 315                 320
Arg Arg Val Cys Phe Phe Ala His Thr Glu Gln Glu Leu Arg Pro Leu
                325                 330                 335
Tyr Val Ser Thr Gly Ser Ala Val Pro Ser Pro Arg Ser Ser Thr Ser
            340                 345                 350
Gly Ala Ala Ala Met Asp Phe Ala Ala Ala Met Ser Leu Leu Pro Gly
        355                 360                 365
Ser Pro Ser Ser Val Ser Ile Met Ser Pro Ser Pro Phe Thr Pro Pro
    370                 375                 380
Met Ser Pro Ser Ala Asn Gly Ile Ser His Pro Ser Val Ala Trp Pro
385                 390                 395                 400
Gln Gln Asn Val Pro Thr Leu His Leu Pro Gly Ser Asn Leu Gln Ser
                405                 410                 415
Ser Arg Leu Arg Ser Ser Leu Asn Ala Arg Asp Ile Pro Gln Glu Asp
            420                 425                 430
Phe Asp Leu Leu Ser Asp Tyr Asp Val Gln Gln Gln Gln Leu Leu Asn
        435                 440                 445
Glu Phe Ser Ile Leu Ser Gln Gln Ser Met Gly Ala Asn Ser Leu Asn
    450                 455                 460
Arg Ser Gly Arg Leu Lys Thr Leu Thr Pro Ser Asn Leu Asp Asp Leu
465                 470                 475                 480
Phe Ser Ala Glu Ser Ser Ser Pro Arg Tyr Ala Asp Gln Ala Leu Ala
                485                 490                 495
Ser Ala Val Phe Ser Pro Thr His Lys Ser Ala Val Ile Asn Gln Phe
            500                 505                 510
Gln Gln Gln Gln Gln Ser Met Leu Ser Pro Ile Asn Thr Thr Phe Ser
```

```
        515                 520                 525
Pro Lys Ser Val Asp His Pro Leu Leu Gln Ala Ser Phe Gly Val Gln
    530                 535                 540
Ser Gly Arg Met Ser Pro Arg Asn Met Asp Pro Ile Ser Pro Ile Ser
545                 550                 555                 560
Ser Arg Val Ser Met Leu Ala Gln Arg Glu Lys Gln Gln Gln Gln Leu
                565                 570                 575
Arg Ser Leu Ser Ser Arg Glu Leu Gly Ser Asn Ser Ala Ala Ile Val
            580                 585                 590
Gly Ser Pro Val Gly Ser Trp Ser Lys Trp Gly Ala Thr Asn Gly Lys
        595                 600                 605
Pro Asp Trp Ala Val Ser Ala Asp Glu Leu Gly Lys Leu Arg Arg Ser
    610                 615                 620
Asn Ser Phe Glu Leu Gly Asn Asn Gly Glu Glu Pro Asp Leu Ser Trp
625                 630                 635                 640
Val Gln Ser Leu Val Lys Glu Ser Pro Thr Glu Met Lys Glu Lys Leu
                645                 650                 655
Ser Ser Thr Leu Ser Gly Val Pro Ala Pro Ala Thr Ser Ser Glu Val
            660                 665                 670
Pro Ser Ile Ser Ser Gln Met Glu Ser Val Asp His Glu Val Leu Gly
        675                 680                 685
Ala Trp Leu Gln Gln Met Gln Leu Asp Pro Leu Val Ala Gln Gln Asn
    690                 695                 700
```

<210> 12
<211> 1860
<212> DNA
<213> Oryza sativa

<400> 12

```
atgggggagc ctgggggcgc cgaggcggcc gtctccgcga ggctgctcga gctggcggcc     60
gacgacaacg cggcggggct cggggagctc ctcgcggcgt ggccctccct cgccgacgag    120
cccgcgccgt ggtacacccc ggcgcggggc gcggagccgc tgaccccgct catggtcgcc    180
gccgtgtacg gctcggtggg ctgcctcgac gcgctcctct cgccgcccta cctcgtggac    240
cccaaccgcg cctcggcgtc gtcgctctcc accccgctcc acctcgccgc cgcgggcggg    300
tccgcctccg cccccgcggc ggtctcccgc ctcctcgccg ccggcgccga cccggccctc    360
ctcgaccacc tccagcgccg ggcgtccgac ctcgtcgcgc tcccgcccaa ctcgctcccg    420
ctcaagaacc acctcctctc cctcctcggc gcccgcaagg agtggcctcc cgacccctcc    480
ctccccgaca tcaagaacgg cgcctacgcc tccgacgact tcaggatgta ctcgttcaag    540
gtgcgcgcgt gctcgcgggc ctactcccat gactggacgg agtgcccctt cgtccacccc    600
ggcgagaacg cgcggcggcg cgacccgagg aagtaccact acagctgcgt gccgtgcccg    660
gagttcaaga agggggccgg gtgcaggaga ggggacatgt gcgagtacgc gcacggggtg    720
ttcgagagct ggctccaccc ggcgcagtac cggacgcgcc tctgcaagga cggcgtcggc    780
tgcgcccgcc gcgtctgctt cttcgcccac acgcccgacg agctccgccc gctctacgtc    840
tccacgggct ccgccgtgcc gtcgccgcgc ggggcgttgg agatggcggc ggcggcggcg    900
gcgatgggga tggggctgtc gtcgccgggg tcgtcgtcgt tcacgccgcc gctatcgccg    960
tcggccggcg gggcgggggg cgggggcggg ggcagcggcg gcggcggcgc gtggccgcag   1020
cagccgagcg tgccggcgct ctgcctgccc gggagcgccg ggaacctcca cctgagccgg   1080
ctgcgcacgt cgctgagcgc gcgcgacatg gccgtcgacg agctgctcgc cgcggcggcg   1140
gcggcggcgg actacgacgg cctcgtcgcc tcccccgcct ccatccggtc cgcgaggggg   1200
aaggcgcttg tgccgtcaaa tctcgacgag ctcttctccg ctgagctcgc cgccgccgcg   1260
gcgtcgcgct cgccgcgcta cgccgaccaa ggcggcgccg cgttctcccc gacccgcaag   1320
gccaccgtgc tcaaccaatt ccagctgcag cagcagcata gcttgctctc gccgcgggcg   1380
gccgcggtga caccagagcc ggtctcccca atgagctccc gcctcctcgc cgcgctggcg   1440
cagcgggaga agatgcagca gcagacgctg cggagcatga gctcacggga cctcggcaac   1500
gccgcgtcgc tgctggtcgg ctcgccggtg agctcgagca tgtccaaatg gggttcccc    1560
tccggcaacc cggactgggg cgccgacgac gaggagctcg gccgcctcaa gcgttgctcc   1620
tcgttcgagc tccggtccgg agccgccaat ggcaaccatg agcctgacct ctcatggtc    1680
aacaccctag tgaaggagcc gacaccggag aagatgatga cgacgacatc ggcaatggat   1740

tccattggca tcttgggaca gaacacaagc cgtgatcaca tcgtcggagg cgaggatgac   1800
actgccggag tcatcagcag ctggcttgaa cagctccagc tcgatgagat ggttgtctag   1860
```

<210> 13
<211> 619
<212> PRT
<213> Oryza sativa

<400> 13

```
Met Gly Glu Pro Gly Gly Ala Glu Ala Ala Val Ser Ala Arg Leu Leu
1               5                   10                  15
Glu Leu Ala Ala Asp Asp Asn Ala Ala Gly Leu Gly Glu Leu Leu Ala
            20                  25                  30
Ala Trp Pro Ser Leu Ala Asp Glu Pro Ala Pro Trp Tyr Thr Pro Ala
        35                  40                  45
Arg Gly Ala Glu Pro Leu Thr Pro Leu Met Val Ala Ala Val Tyr Gly
    50                  55                  60
Ser Val Gly Cys Leu Asp Ala Leu Leu Ser Pro Pro Tyr Leu Val Asp
65                  70                  75                  80
Pro Asn Arg Ala Ser Ala Ser Ser Leu Ser Thr Pro Leu His Leu Ala
                85                  90                  95
Ala Ala Gly Gly Ser Ala Ser Ala Pro Ala Ala Val Ser Arg Leu Leu
            100                 105                 110
Ala Ala Gly Ala Asp Pro Ala Leu Leu Asp His Leu Gln Arg Arg Ala
        115                 120                 125
Ser Asp Leu Val Ala Leu Pro Pro Asn Ser Leu Pro Leu Lys Asn His
    130                 135                 140
Leu Leu Ser Leu Leu Gly Ala Arg Lys Glu Trp Pro Pro Asp Pro Ser
145                 150                 155                 160
Leu Pro Asp Ile Lys Asn Gly Ala Tyr Ala Ser Asp Asp Phe Arg Met
                165                 170                 175
Tyr Ser Phe Lys Val Arg Ala Cys Ser Arg Ala Tyr Ser His Asp Trp
            180                 185                 190
Thr Glu Cys Pro Phe Val His Pro Gly Glu Asn Ala Arg Arg Arg Asp
        195                 200                 205
Pro Arg Lys Tyr His Tyr Ser Cys Val Pro Cys Pro Glu Phe Lys Lys
    210                 215                 220
Gly Ala Gly Cys Arg Arg Gly Asp Met Cys Glu Tyr Ala His Gly Val
225                 230                 235                 240
Phe Glu Ser Trp Leu His Pro Ala Gln Tyr Arg Thr Arg Leu Cys Lys
                245                 250                 255
Asp Gly Val Gly Cys Ala Arg Arg Val Cys Phe Phe Ala His Thr Pro
            260                 265                 270
Asp Glu Leu Arg Pro Leu Tyr Val Ser Thr Gly Ser Ala Val Pro Ser
        275                 280                 285
Pro Arg Gly Ala Leu Glu Met Ala Ala Ala Ala Ala Ala Met Gly Met
    290                 295                 300
Gly Leu Ser Ser Pro Gly Ser Ser Ser Phe Thr Pro Pro Leu Ser Pro
305                 310                 315                 320
Ser Ala Gly Gly Gly Gly Gly Gly Gly Gly Gly Ser Gly Gly Gly Gly
                325                 330                 335
Ala Trp Pro Gln Gln Pro Ser Val Pro Ala Leu Cys Leu Pro Gly Ser
            340                 345                 350
Ala Gly Asn Leu His Leu Ser Arg Leu Arg Thr Ser Leu Ser Ala Arg
        355                 360                 365
Asp Met Ala Val Asp Glu Leu Leu Ala Ala Ala Ala Ala Ala Ala Asp
    370                 375                 380
Tyr Asp Gly Leu Val Ala Ser Pro Ala Ser Ile Arg Ser Ala Arg Gly
385                 390                 395                 400
```

```
Lys Ala Leu Val Pro Ser Asn Leu Asp Glu Leu Phe Ser Ala Glu Leu
                405                 410                 415
Ala Ala Ala Ala Ala Ser Arg Ser Pro Arg Tyr Ala Asp Gln Gly Gly
            420                 425                 430
Ala Ala Phe Ser Pro Thr Arg Lys Ala Thr Val Leu Asn Gln Phe Gln
        435                 440                 445
Leu Gln Gln Gln His Ser Leu Leu Ser Pro Arg Ala Ala Ala Val Thr
    450                 455                 460
Pro Glu Pro Val Ser Pro Met Ser Ser Arg Leu Leu Ala Ala Leu Ala
465                 470                 475                 480
Gln Arg Glu Lys Met Gln Gln Gln Thr Leu Arg Ser Met Ser Ser Arg
                485                 490                 495
Asp Leu Gly Asn Ala Ala Ser Leu Leu Val Gly Ser Pro Val Ser Ser
            500                 505                 510
Ser Met Ser Lys Trp Gly Phe Pro Ser Gly Asn Pro Asp Trp Gly Ala
        515                 520                 525
Asp Asp Glu Glu Leu Gly Arg Leu Lys Arg Cys Ser Ser Phe Glu Leu
    530                 535                 540
Arg Ser Gly Ala Ala Asn Gly Asn His Glu Pro Asp Leu Ser Trp Val
545                 550                 555                 560
Asn Thr Leu Val Lys Glu Pro Thr Pro Glu Lys Met Met Thr Thr Thr
                565                 570                 575
Ser Ala Met Asp Ser Ile Gly Ile Leu Gly Gln Asn Thr Ser Arg Asp
            580                 585                 590
His Ile Val Gly Gly Glu Asp Asp Thr Ala Gly Val Ile Ser Ser Trp
        595                 600                 605
Leu Glu Gln Leu Gln Leu Asp Glu Met Val Val
    610                 615
```

<210> 14
<211> 2106
<212> DNA
<213> Medicago truncatula

<400> 14

```
atgaaaaatc taactgttcg tactgatgat tctttttcca gcttacttga acatgcttct     60
aacaatgatt ttgaagattt caaggtagct ctagatagtg atgcttcact tattaatgaa    120
gttggcttct ggtatgtccg tcaaaaggga tctaaccaaa ttgttcttga gcaccgaacc    180
cctttaatgg tggctgcttc ctatgggagt attgatattc taaagcttat actctcatat    240
cccgaggctg atgttaattt ctcctgtgga actgataaaa gcactgctct tcactgtgct    300
gcctcaagtg gttcagttaa tgctgttgat gctataaaat tgcttttatc agctggtgct    360
gatatcaatt ctgtggatgc taatgggaaa cgccctgtgg atgttatcgt tgttcctatt    420
gttgttcctc ataagctcga aggtgttaaa acaattcttg aagaacttct ctcagacagt    480
gcttctgaag gatctgtgga tgattgctct cttcccctgt ctcttatttc atcgagtcct    540
ggttcatctg cccctttatc atctgctgaa aatggatctc catcctctcc tgtggctccc    600
aagtttacag atacagctgt taattctaca tcagaaaaga aagagtatcc agttgaccca    660
tctcttcctg acataaaaaa cagcatgtat gccacagatg aattccgcat gtattcattc    720
aaggttcgtc cttgttctcg tgcatactct catgattgga ctgagtgtcc ttttgtgcat    780
cctggagaga atgctcgaag gagagaccct agaaagtttc actacagctg tgtgccatgc    840
cctgatttta ggaaaggggc ttgccgacgt tcggatatgt gtgaatatgc tcatggagta    900
ttcgagtgct ggctacaccc agctcagtat cggacaaggc tgtgcaaaga cggtatgggt    960
tgtaaccgaa gggtgtgctt cttcgctcac tcacctgaag agctgcgtcc gctgtatgtg   1020
tccactggtt ctgctgttcc ttcaccccga tcagctgctt ctactgctaa tgtcatggac   1080
atggctgctg ctatgagcct tttccctggt tcaccatcat caatctcttt gatgtctcaa   1140
tcaccctttg cacagcctcc tctatctcca tctgcaaatg gcaataatgc ttggccacag   1200
cccaatgtgc cagctcttca tttaccagga agcattaatc aaactagtcg tttgagatct   1260
tctcttagtg cccgtgatat gccacacgac gacttcaaca atatgttgca agactttgat   1320
gggcagcagc agatactaaa tgacttgagc tgtttctcac agccccgtcc tggtgctatt   1380
tcagttggtc gatctggccg ccctaaaaca ctaactccct caaatctgga tgatcttttt   1440

tgtgctgaga ttgcttcatc tcctaggtat tccgaccccg ctgcggcttc tgtattttcc   1500
ccaacacaca aatctgctgt cttcaaccag tttcaacagc ttcaaagctc cttatcaccc   1560
atcaacacaa atgtcatgtc tcctacaaac gtagagcatc ccctgttcca ccaggcttca   1620
tatggtctct cttctcctgg aaggatgtca ccaagaagta tggaagccct atctccaatg   1680
agttctcggc tgtcagcttt tgctcagcgt gagaaacaac agcagcagca gcaacagctg   1740
cgtagcctca gctcaagaga actcggtgct aacaatcctc tctcagctgt tgggtcccct   1800
gttaactcct ggtccaagtg gggatcatcc cctattggaa aagctgattg gtcggtaaat   1860
ccaaatgact tcggtcaaac acagagatca acttcttttg agcatggaaa caatggagaa   1920
gagcctgatg taggttgggt ccattccctt gtcaaggatc ccacacctga gaagaaagag   1980
aagcttgcag gttccggccc aattccatcc gttgaaaaga atcccaatcc tcaagcggac   2040
ggcattgatc actctgtttt gggagcttgg ctcgagcaac tgcagctgga tcaacttgta   2100
gtctag                                                              2106
```

<210> 15
<211> 701
<212> PRT
<213> Medicago truncatula

<400> 15

```
Met Lys Asn Leu Thr Val Arg Thr Asp Asp Ser Phe Ser Ser Leu Leu
1               5                   10                  15
Glu His Ala Ser Asn Asn Asp Phe Glu Asp Phe Lys Val Ala Leu Asp
            20                  25                  30
Ser Asp Ala Ser Leu Ile Asn Glu Val Gly Phe Trp Tyr Val Arg Gln
        35                  40                  45
Lys Gly Ser Asn Gln Ile Val Leu Glu His Arg Thr Pro Leu Met Val
    50                  55                  60
Ala Ala Ser Tyr Gly Ser Ile Asp Ile Leu Lys Leu Ile Leu Ser Tyr
65                  70                  75                  80
Pro Glu Ala Asp Val Asn Phe Ser Cys Gly Thr Asp Lys Ser Thr Ala
                85                  90                  95
Leu His Cys Ala Ala Ser Ser Gly Ser Val Asn Ala Val Asp Ala Ile
            100                 105                 110
Lys Leu Leu Leu Ser Ala Gly Ala Asp Ile Asn Ser Val Asp Ala Asn
        115                 120                 125
Gly Lys Arg Pro Val Asp Val Ile Val Val Pro Ile Val Val Pro His
    130                 135                 140
Lys Leu Glu Gly Val Lys Thr Ile Leu Glu Glu Leu Leu Ser Asp Ser
145                 150                 155                 160
Ala Ser Glu Gly Ser Val Asp Asp Cys Ser Leu Pro Leu Ser Leu Ile
                165                 170                 175
Ser Ser Ser Pro Gly Ser Ser Ala Pro Leu Ser Ser Ala Glu Asn Gly
            180                 185                 190
Ser Pro Ser Ser Pro Val Ala Pro Lys Phe Thr Asp Thr Ala Val Asn
        195                 200                 205
Ser Thr Ser Glu Lys Lys Glu Tyr Pro Val Asp Pro Ser Leu Pro Asp
    210                 215                 220
Ile Lys Asn Ser Met Tyr Ala Thr Asp Glu Phe Arg Met Tyr Ser Phe
225                 230                 235                 240
Lys Val Arg Pro Cys Ser Arg Ala Tyr Ser His Asp Trp Thr Glu Cys
                245                 250                 255
Pro Phe Val His Pro Gly Glu Asn Ala Arg Arg Arg Asp Pro Arg Lys
            260                 265                 270
Phe His Tyr Ser Cys Val Pro Cys Pro Asp Phe Arg Lys Gly Ala Cys
        275                 280                 285
Arg Arg Ser Asp Met Cys Glu Tyr Ala His Gly Val Phe Glu Cys Trp
    290                 295                 300
Leu His Pro Ala Gln Tyr Arg Thr Arg Leu Cys Lys Asp Gly Met Gly
305                 310                 315                 320
```

```
Cys Asn Arg Arg Val Cys Phe Phe Ala His Ser Pro Glu Glu Leu Arg
                325                 330                 335
Pro Leu Tyr Val Ser Thr Gly Ser Ala Val Pro Ser Pro Arg Ser Ala
            340                 345                 350
Ala Ser Thr Ala Asn Val Met Asp Met Ala Ala Ala Met Ser Leu Phe
        355                 360                 365
Pro Gly Ser Pro Ser Ser Ile Ser Leu Met Ser Gln Ser Pro Phe Ala
    370                 375                 380
Gln Pro Pro Leu Ser Pro Ser Ala Asn Gly Asn Asn Ala Trp Pro Gln
385                 390                 395                 400
Pro Asn Val Pro Ala Leu His Leu Pro Gly Ser Ile Asn Gln Thr Ser
                405                 410                 415
Arg Leu Arg Ser Ser Leu Ser Ala Arg Asp Met Pro His Asp Asp Phe
            420                 425                 430
Asn Asn Met Leu Gln Asp Phe Asp Gly Gln Gln Gln Ile Leu Asn Asp
        435                 440                 445
Leu Ser Cys Phe Ser Gln Pro Arg Pro Gly Ala Ile Ser Val Gly Arg
    450                 455                 460
Ser Gly Arg Pro Lys Thr Leu Thr Pro Ser Asn Leu Asp Asp Leu Phe
465                 470                 475                 480
Cys Ala Glu Ile Ala Ser Ser Pro Arg Tyr Ser Asp Pro Ala Ala Ala
                485                 490                 495
Ser Val Phe Ser Pro Thr His Lys Ser Ala Val Phe Asn Gln Phe Gln
            500                 505                 510
Gln Leu Gln Ser Ser Leu Ser Pro Ile Asn Thr Asn Val Met Ser Pro
        515                 520                 525
Thr Asn Val Glu His Pro Leu Phe His Gln Ala Ser Tyr Gly Leu Ser
    530                 535                 540
Ser Pro Gly Arg Met Ser Pro Arg Ser Met Glu Ala Leu Ser Pro Met
545                 550                 555                 560
Ser Ser Arg Leu Ser Ala Phe Ala Gln Arg Glu Lys Gln Gln Gln Gln
                565                 570                 575
Gln Gln Gln Leu Arg Ser Leu Ser Ser Arg Glu Leu Gly Ala Asn Asn
            580                 585                 590
Pro Leu Ser Ala Val Gly Ser Pro Val Asn Ser Trp Ser Lys Trp Gly
        595                 600                 605
Ser Ser Pro Ile Gly Lys Ala Asp Trp Ser Val Asn Pro Asn Asp Phe
    610                 615                 620
Gly Gln Thr Gln Arg Ser Thr Ser Phe Glu His Gly Asn Asn Gly Glu
625                 630                 635                 640
Glu Pro Asp Val Gly Trp Val His Ser Leu Val Lys Asp Pro Thr Pro
                645                 650                 655
Glu Lys Lys Glu Lys Leu Ala Gly Ser Gly Pro Ile Pro Ser Val Glu
            660                 665                 670
Lys Asn Pro Asn Pro Gln Ala Asp Gly Ile Asp His Ser Val Leu Gly
        675                 680                 685
Ala Trp Leu Glu Gln Leu Gln Leu Asp Gln Leu Val Val
    690                 695                 700
```

<210> 16
<211> 2841
<212> DNA
<213> Oryza sativa

<400> 16

```
atgaacggca cgccgatctc cgcgtccgcc gcggccggcg tcgacggagt cggcgcggcg     60
gtggcgctgg cggccgcgac caagaagagt gccgccgcgg cggccgccgt cgccgagatg    120
gcgaaaaccc tcaccgtcga cacggacgac gccttcgcgg ggctcctcga gctcgccgcg    180
gacgacgacg cggagggcct gcgccgcgcg ctggagcgcg ccccgcccgc cgccgcggac    240

gaggcgggcc tctggtacgg ccgccgcaag gtcctcgagc accgcacgcc gctgatggtc    300
gcggccacct atggcagcct cgcggtgctt cgcctgctgc tgtccctccc gtccgtcgat    360
gtcaatcgcc gctgtggctc cgacggcacc accgccctcc actgtgcggc gtctggtggc    420
tcgccgtctt gtgtggaggc cgtcaagctg ctgcttgctg ctggggctga tgctgatgcc    480
acggatgctt ccggatatcg tccagctgat gtgatctctg ttcctccaaa gatgtttgac    540
gccaagattg ccctccaaga tcttcttgga tgcccaaagg ctgggcatgg cgttctccgg    600
gtggtgacaa gggccgcaaa ctctatgttg tcacctgtat catcccctac agcagaagat    660
gcacgatctc catcagctgc tgtgatgatg acgacaaagt ttgcagatct tccaagggtt    720
gtgacatcgg aaaagaaaga atatccagtg gatccgtccc ttcccgatat caagaacagc    780
atctatgctt ccgatgagtt ccgcatgtac tcatttaaga tcaggccatg ctcgcgggcg    840
tactcacatg attggactga gtgcccgttt gttcacccag gggagaacgc acggcgtcgg    900
gaccctcgca agtatcacta cagctgtgtg ccatgccccg actttagaaa gggagtttgc    960
cggcgtggtg acatgtgtga atatgctcat ggcgtgttcg agtgttggct ccatccagca   1020
cagtaccgta ctcgcctttg caaggatggc acaagctgta atcgccgtgt ctgtttcttt   1080
gcgcatacaa ctgatgagct ccgaccacta tatgtttcca ctggatctgc agtaccatcc   1140
ccaagagcct cggcaacagc tacaatggag atggctgcag caatgggctt gatgcctggt   1200
tctccatcat cagtttcagc agtcatgtcc ccatttacac caccaatgtc cccttcaggc   1260
aatgggatgc ccccttcatt gggctggcag cagccaaatg ttccgacact acaccttcca   1320
ggcagcagcc ttcagtcgag ccggctccgt acctcactta gtgcaaggga tatgcctgct   1380
gatgattact ccctgatgca ggatattgat tcacagctta taaatgattt gtgctattca   1440
cgtattggtt catcaacagg aaaccacacg tctcggacca agtccctaaa tccgtcaaac   1500
ttggatgatc tcttctctgc tgagatggtc tcttccccga ggtatagtaa tgctgatcag   1560
ggtggtatgt tttcaccatc tcacaaggct gctttcctta atcagttcca gcaacagcag   1620
caggcacttc tttcaccaat caacacagtc ttctccccga agtctgtgga caaccagcag   1680
ttgccttcac actcatctct gttgcaagca tcacttggta tatcctcccc tggccgcatg   1740
tctcctcgat gtgttgaatc tgggtcccct atgaactctc atcttgctgc tgctcttgct   1800
cagcgtgaga agcaacagca gacaatgaga agtctcagtt ctcgtgatct tgggccgagt   1860
gctgcaagag catcaggtgt tgttggctcc cctctaagct catcatggtc aaagtgggga   1920
tcaccttcag ggacacctga ctggggtgtt aatggtgaag aattgggcaa gcttcgccgg   1980
tcatcatcgt ttgagctgag atctggtggt gatgatccag atctctcttg ggtacacaca   2040
ctggttaagg aatctccacc agagaagcaa gtcactactg ctgaatccat aaactctgtt   2100
ggaccttcac cactgatgcc tcccagtgta agcaacggtg aaggtcctag tctgaatgcc   2160
ccgctggatg ggcatgacca agctgctgtt attggagcat tgcttgaaca gatgcagctt   2220
gatcagcata ttggtagtct agcaacataa gcgctgaatg agcctggaaa gtgcaaggag   2280
ttattattct tagttaatga atttggagta atttttttcc tgttcattaa gatggtcagc   2340
aagcaaaagg atggatagct gatggtggtg attcagagat tggttttctt tactttattg   2400
aggtaaatca tatacattat tgaggttcca gtaggttgaa agattgaagt accttgattg   2460
gggtcgtttc aagaccgacc caggtagaat cgcaccccgg cagcttcaat tcatcggtca   2520
aaaatatttc cctgttttgt taattaaccc cgttaaaaaa gaagactcgt ttggtgtttc   2580
ggaattcttt tctttacctt agcggtgttt attttgttta ttatgatatt gatacttgat   2640
gtactgatgg gtataaggtt ggttaccagg catgctatag tggtatatca agtcccaaag   2700
tattcttttt ctccctttca ccatttgtcg aggatcatac tatggccttg ttttggtcag   2760
atcttgaggc ctgtataatc cttggatttg taataatgta atattgtcat tgaacttaca   2820
ttgctattgt tttgcaatcg c                                              2841
```

<210> 17
<211> 749
<212> PRT
<213> Oryza sativa

<400> 17

```
Met Asn Gly Thr Pro Ile Ser Ala Ser Ala Ala Ala Gly Val Asp Gly
1               5                   10                  15
Val Gly Ala Ala Val Ala Leu Ala Ala Ala Thr Lys Lys Ser Ala Ala
            20                  25                  30
Ala Ala Ala Ala Val Ala Glu Met Ala Lys Thr Leu Thr Val Asp Thr
        35                  40                  45
Asp Asp Ala Phe Ala Gly Leu Leu Glu Leu Ala Ala Asp Asp Asp Ala
    50                  55                  60
```

```
Glu Gly Leu Arg Arg Ala Leu Glu Arg Ala Pro Pro Ala Ala Ala Asp
65                  70                  75                  80
Glu Ala Gly Leu Trp Tyr Gly Arg Arg Lys Val Leu Glu His Arg Thr
                85                  90                  95
Pro Leu Met Val Ala Ala Thr Tyr Gly Ser Leu Ala Val Leu Arg Leu
            100                 105                 110
Leu Leu Ser Leu Pro Ser Val Asp Val Asn Arg Arg Cys Gly Ser Asp
        115                 120                 125
Gly Thr Thr Ala Leu His Cys Ala Ala Ser Gly Gly Ser Pro Ser Cys
    130                 135                 140
Val Glu Ala Val Lys Leu Leu Leu Ala Ala Gly Ala Asp Ala Asp Ala
145                 150                 155                 160
Thr Asp Ala Ser Gly Tyr Arg Pro Ala Asp Val Ile Ser Val Pro Pro
                165                 170                 175
Lys Met Phe Asp Ala Lys Ile Ala Leu Gln Asp Leu Leu Gly Cys Pro
            180                 185                 190
Lys Ala Gly His Gly Val Leu Arg Val Val Thr Arg Ala Ala Asn Ser
        195                 200                 205
Met Leu Ser Pro Val Ser Ser Pro Thr Ala Glu Asp Ala Arg Ser Pro
    210                 215                 220
Ser Ala Ala Val Met Met Thr Thr Lys Phe Ala Asp Leu Pro Arg Val
225                 230                 235                 240
Val Thr Ser Glu Lys Lys Glu Tyr Pro Val Asp Pro Ser Leu Pro Asp
                245                 250                 255
Ile Lys Asn Ser Ile Tyr Ala Ser Asp Glu Phe Arg Met Tyr Ser Phe
            260                 265                 270
Lys Ile Arg Pro Cys Ser Arg Ala Tyr Ser His Asp Trp Thr Glu Cys
        275                 280                 285
Pro Phe Val His Pro Gly Glu Asn Ala Arg Arg Arg Asp Pro Arg Lys
    290                 295                 300
Tyr His Tyr Ser Cys Val Pro Cys Pro Asp Phe Arg Lys Gly Val Cys
305                 310                 315                 320
Arg Arg Gly Asp Met Cys Glu Tyr Ala His Gly Val Phe Glu Cys Trp
                325                 330                 335
Leu His Pro Ala Gln Tyr Arg Thr Arg Leu Cys Lys Asp Gly Thr Ser
            340                 345                 350
Cys Asn Arg Arg Val Cys Phe Phe Ala His Thr Thr Asp Glu Leu Arg
        355                 360                 365
Pro Leu Tyr Val Ser Thr Gly Ser Ala Val Pro Ser Pro Arg Ala Ser
    370                 375                 380
Ala Thr Ala Thr Met Glu Met Ala Ala Ala Met Gly Leu Met Pro Gly
385                 390                 395                 400
Ser Pro Ser Ser Val Ser Ala Val Met Ser Pro Phe Thr Pro Pro Met
                405                 410                 415
Ser Pro Ser Gly Asn Gly Met Pro Pro Ser Leu Gly Trp Gln Gln Pro
            420                 425                 430
Asn Val Pro Thr Leu His Leu Pro Gly Ser Ser Leu Gln Ser Ser Arg
        435                 440                 445
Leu Arg Thr Ser Leu Ser Ala Arg Asp Met Pro Ala Asp Asp Tyr Ser
    450                 455                 460
Leu Met Gln Asp Ile Asp Ser Gln Leu Ile Asn Asp Leu Cys Tyr Ser
465                 470                 475                 480
Arg Ile Gly Ser Ser Thr Gly Asn His Thr Ser Arg Thr Lys Ser Leu
                485                 490                 495
Asn Pro Ser Asn Leu Asp Asp Leu Phe Ser Ala Glu Met Val Ser Ser
            500                 505                 510
Pro Arg Tyr Ser Asn Ala Asp Gln Gly Gly Met Phe Ser Pro Ser His
        515                 520                 525
Lys Ala Ala Phe Leu Asn Gln Phe Gln Gln Gln Gln Gln Ala Leu Leu
```

```
        530                 535                 540
Ser Pro Ile Asn Thr Val Phe Ser Pro Lys Ser Val Asp Asn Gln Gln
545                 550                 555                 560
Leu Pro Ser His Ser Ser Leu Leu Gln Ala Ser Leu Gly Ile Ser Ser
                565                 570                 575
Pro Gly Arg Met Ser Pro Arg Cys Val Glu Ser Gly Ser Pro Met Asn
            580                 585                 590
Ser His Leu Ala Ala Ala Leu Ala Gln Arg Glu Lys Gln Gln Gln Thr
        595                 600                 605
Met Arg Ser Leu Ser Ser Arg Asp Leu Gly Pro Ser Ala Ala Arg Ala
    610                 615                 620
Ser Gly Val Val Gly Ser Pro Leu Ser Ser Ser Trp Ser Lys Trp Gly
625                 630                 635                 640
Ser Pro Ser Gly Thr Pro Asp Trp Gly Val Asn Gly Glu Glu Leu Gly
                645                 650                 655
Lys Leu Arg Arg Ser Ser Ser Phe Glu Leu Arg Ser Gly Gly Asp Asp
            660                 665                 670
Pro Asp Leu Ser Trp Val His Thr Leu Val Lys Glu Ser Pro Pro Glu
        675                 680                 685
Lys Gln Val Thr Thr Ala Glu Ser Ile Asn Ser Val Gly Pro Ser Pro
    690                 695                 700
Leu Met Pro Pro Ser Val Ser Asn Gly Glu Gly Pro Ser Leu Asn Ala
705                 710                 715                 720
Pro Leu Asp Gly His Asp Gln Ala Ala Val Ile Gly Ala Leu Leu Glu
                725                 730                 735
Gln Met Gln Leu Asp Gln His Ile Gly Ser Leu Ala Thr
            740                 745
```

<210> 18
<211> 2769
<212> DNA
<213> Arabidopsis thaliana

<400> 18

```
acaccagtta ccctctcatc cgttttcgtt ttttttttct ctctttcaaa aatctctcag     60
ctgaggttga tcgatcttct tcttcttctt cctcactctt tagatttgtt ccttttgcat    120
tttacacttt tggatctgaa aatgtggttc tctgtttcgc ccgttaccgt ttagattcag    180
ttctgttttt ttcttacccg atcgcttgat tcggactgtg atctttgatc tttttcttc     240
tccagtgccg tgaaggatgt gtggtcttgc taagaagctg gatatagagg atactttgac    300
atcactgtca gaccaagaga atgaatcttt ggccaaaccc atgaatgatg ctgctgaatg    360
ggaacattcg ttttctgcct tgcttgagtt tgctgcagac aacgatgtgg aggggtttag    420
gcggcaactc tctgatgtgt cttgtatcaa ccagatgggt ctttggtaca gacggcagag    480
gtttgttaga agaatggttc ttgagcaaag aaccccgctg atggttgctt cgttatatgg    540
gagtttagat gttgtgaagt ttattctttc tttcccggaa gcggagttga atctgtcttg    600
tggtcctgat aaaagtactg ctcttcattg cgctgcttct ggtgcttctg tgaattcctt    660
ggatgttgtc aagttgcttt tgagtgtagg agcagatcct aatatccctg atgctcatgg    720
aaatcgtcct gttgatgttc ttgttgtgtc tccacacgct cctggtttga gaaccatcct    780
tgaagagatc ttgaagaaag acgagattat atctgaagat ctgcatgcct cgtcatctag    840
cttgggatca agtttccggt ctctctcatc atcccctgat aatggttcct cgttactctc    900
cttagattca gtatcctctc cgactaagcc acacggtact gatgtaactt tcgcatcaga    960
gaagaaagag tacccaattg atccatcatt gcctgatatc aaaagcggga tttattcaac   1020
cgatgagttt cgtatgttct cgttcaagat ccgcccatgt tctcgagcat attcccatga   1080
ctggactgaa tgtccatttg cacacccagg tgagaatgca aggagaagag acccgaggaa   1140
gtttcactat acgtgtgttc catgcccgga ttttaagaaa ggatcctgta agcaaggtga   1200
tatgtgtgaa tatgctcatg ggtttttga atgctggcta caccctgctc agtacagaac   1260
acgattgtgc aaggacggaa tgggttgcaa ccgaagggtt tgcttctttg ctcacgcaaa   1320
tgaggagttg cgtcccttgt acccttccac aggatctgga ttgccatctc ctcgggcttc   1380
gtctgctgtt tccgcctcta ctatggacat ggcgtcagtt ttgaacatgt taccaggctc   1440
accatctgct gctcaacatt cgttcacccc accaatatct ccttctggaa atggtagtat   1500

gccccattca tcgatgggtt ggcctcagca gaacataccg gcgttgaatc ttcctggaag   1560
caatatccag ttgagtcgtc tgagatcttc tcttaacgct agagatattc cttctgagca   1620
gcttagcatg ctgcatgagt ttgaaatgca acgtcagctt gctggcgata tgcacagtcc   1680
acgctttatg aatcattccg ctcgtcctaa gacactgaac ccttcaaatc tggaggaact   1740
cttctcagct gaggttgcat ctcctcgttt ctctgatcaa cttgctgttt catctgttct   1800
atcgccttcc cacaagtccg cgcttcttaa tcagctgcag aataataagc agagcatgct   1860
ttctcctatc aagacaaatc taatgtcttc tccaaagaat gtggagcaac attctcttct   1920
gcagcaagcc tcgtcacccc gaggcggaga gcctatttcc ccaatgaatg ctcgaatgaa   1980
acagcagcta cattcacgca gcctaagctc ccgtgatttt ggatctagtc tgccccgtga   2040
tttaatgccg actgattctg gttcgccatt aagtccatgg tcaagttggg accagaccca   2100
tggaagcaag gtggattggt cagtccaatc agatgagtta ggtcggttga gaaaatctca   2160
ttccttggct aataacccaa acagggaagc agatgtttca tgggctcagc agatgttaaa   2220
agactcttca tcacctagga acggaaaccg tgttgtgaac atgaatggtg caaggccatt   2280
gactcaaggt ggttcgagtg tgaatcctca caacagtgac actcgtgaga gcgacattct   2340
tgatgcgtgg cttgaacagc tgcacctaga tcgctgagcc tcagctgcga gagagaggtt   2400
cacatttctg tgaagctgtg aaactgatga ttcgtttatt tattattcaa gaaagcaaac   2460
ggaaacaaaa gcaaactccg ggtaagcttt tttcgattct aataacccta aaaggctcag   2520
ttttttcagg cttctttctg aaatttcttt actttcttat ttttatcacc tcattaaatt   2580
aattattgta tcatctctgt tgtaacaatg gccaaagtgc gcctctatta cttcccggat   2640
ttctgattta catttttgt atcctctcag tttgtcaatt gtttctaata tctccttcat   2700
atttgtcaaa gaacactgta tgagaaataa taacatattg tttcagctaa taagattcat   2760
tcatttcct                                                           2769
```

<210> 19
<211> 706
<212> PRT
<213> Arabidopsis thaliana

<400> 19

```
Met Cys Gly Leu Ala Lys Lys Leu Asp Ile Glu Asp Thr Leu Thr Ser
1               5                   10                  15
Leu Ser Asp Gln Glu Asn Glu Ser Leu Ala Lys Pro Met Asn Asp Ala
            20                  25                  30
Ala Glu Trp Glu His Ser Phe Ser Ala Leu Leu Glu Phe Ala Ala Asp
        35                  40                  45
Asn Asp Val Glu Gly Phe Arg Arg Gln Leu Ser Asp Val Ser Cys Ile
    50                  55                  60
Asn Gln Met Gly Leu Trp Tyr Arg Arg Gln Arg Phe Val Arg Arg Met
65                  70                  75                  80
Val Leu Glu Gln Arg Thr Pro Leu Met Val Ala Ser Leu Tyr Gly Ser
                85                  90                  95
Leu Asp Val Val Lys Phe Ile Leu Ser Phe Pro Glu Ala Glu Leu Asn
            100                 105                 110
Leu Ser Cys Gly Pro Asp Lys Ser Thr Ala Leu His Cys Ala Ala Ser
        115                 120                 125
Gly Ala Ser Val Asn Ser Leu Asp Val Val Lys Leu Leu Leu Ser Val
    130                 135                 140
Gly Ala Asp Pro Asn Ile Pro Asp Ala His Gly Asn Arg Pro Val Asp
145                 150                 155                 160
Val Leu Val Val Ser Pro His Ala Pro Gly Leu Arg Thr Ile Leu Glu
                165                 170                 175
Glu Ile Leu Lys Lys Asp Glu Ile Ile Ser Glu Asp Leu His Ala Ser
            180                 185                 190
Ser Ser Ser Leu Gly Ser Ser Phe Arg Ser Leu Ser Ser Ser Pro Asp
        195                 200                 205
Asn Gly Ser Ser Leu Leu Ser Leu Asp Ser Val Ser Ser Pro Thr Lys
    210                 215                 220
Pro His Gly Thr Asp Val Thr Phe Ala Ser Glu Lys Lys Glu Tyr Pro
225                 230                 235                 240
```

Ile Asp Pro Ser Leu Pro Asp Ile Lys Ser Gly Ile Tyr Ser Thr Asp
245 250 255
Glu Phe Arg Met Phe Ser Phe Lys Ile Arg Pro Cys Ser Arg Ala Tyr
260 265 270
Ser His Asp Trp Thr Glu Cys Pro Phe Ala His Pro Gly Glu Asn Ala
275 280 285
Arg Arg Arg Asp Pro Arg Lys Phe His Tyr Thr Cys Val Pro Cys Pro
290 295 300
Asp Phe Lys Lys Gly Ser Cys Lys Gln Gly Asp Met Cys Glu Tyr Ala
305 310 315 320
His Gly Val Phe Glu Cys Trp Leu His Pro Ala Gln Tyr Arg Thr Arg
325 330 335
Leu Cys Lys Asp Gly Met Gly Cys Asn Arg Arg Val Cys Phe Phe Ala
340 345 350
His Ala Asn Glu Glu Leu Arg Pro Leu Tyr Pro Ser Thr Gly Ser Gly
355 360 365
Leu Pro Ser Pro Arg Ala Ser Ser Ala Val Ser Ala Ser Thr Met Asp
370 375 380
Met Ala Ser Val Leu Asn Met Leu Pro Gly Ser Pro Ser Ala Ala Gln
385 390 395 400
His Ser Phe Thr Pro Pro Ile Ser Pro Ser Gly Asn Gly Ser Met Pro
405 410 415
His Ser Ser Met Gly Trp Pro Gln Gln Asn Ile Pro Ala Leu Asn Leu
420 425 430
Pro Gly Ser Asn Ile Gln Leu Ser Arg Leu Arg Ser Ser Leu Asn Ala
435 440 445
Arg Asp Ile Pro Ser Glu Gln Leu Ser Met Leu His Glu Phe Glu Met
450 455 460
Gln Arg Gln Leu Ala Gly Asp Met His Ser Pro Arg Phe Met Asn His
465 470 475 480
Ser Ala Arg Pro Lys Thr Leu Asn Pro Ser Asn Leu Glu Glu Leu Phe
485 490 495
Ser Ala Glu Val Ala Ser Pro Arg Phe Ser Asp Gln Leu Ala Val Ser
500 505 510
Ser Val Leu Ser Pro Ser His Lys Ser Ala Leu Leu Asn Gln Leu Gln
515 520 525
Asn Asn Lys Gln Ser Met Leu Ser Pro Ile Lys Thr Asn Leu Met Ser
530 535 540
Ser Pro Lys Asn Val Glu Gln His Ser Leu Leu Gln Gln Ala Ser Ser
545 550 555 560
Pro Arg Gly Gly Glu Pro Ile Ser Pro Met Asn Ala Arg Met Lys Gln
565 570 575
Gln Leu His Ser Arg Ser Leu Ser Ser Arg Asp Phe Gly Ser Ser Leu
580 585 590
Pro Arg Asp Leu Met Pro Thr Asp Ser Gly Ser Pro Leu Ser Pro Trp
595 600 605
Ser Ser Trp Asp Gln Thr His Gly Ser Lys Val Asp Trp Ser Val Gln
610 615 620
Ser Asp Glu Leu Gly Arg Leu Arg Lys Ser His Ser Leu Ala Asn Asn
625 630 635 640
Pro Asn Arg Glu Ala Asp Val Ser Trp Ala Gln Gln Met Leu Lys Asp
645 650 655
Ser Ser Ser Pro Arg Asn Gly Asn Arg Val Val Asn Met Asn Gly Ala
660 665 670
Arg Pro Leu Thr Gln Gly Gly Ser Ser Val Asn Pro His Asn Ser Asp
675 680 685
Thr Arg Glu Ser Asp Ile Leu Asp Ala Trp Leu Glu Gln Leu His Leu
690 695 700
Asp Arg

705

<210> 20
<211> 2674
<212> DNA
<213> Oryza sativa

<400> 20

```
tgcgagtcct cctcctcttc tcgtcgccgt gctactctcg ctttctctct ctctctctct      60
cacttgttcc ccaaggcgag aagcagccgc cgccggcgag cgtcgcgggg gaggggaggg     120
aagggaggga ggagcggtgg atccgggctt gattggattg ggtcggattc gattttggat     180
caaccccgga gggcgggagc ggttgctaca gatgcgttga gctttggtta atctatccgg     240
cgagagataa tgggcgagct tgctgatctc gttgtcgtgc cgtcgcagcc gccgctcgcc     300
ggcggccggc gggacaggct ggcggcgctg ctggagctcg cggcggcgga tgatgttgat     360
gggctcaggg gggcgctcgc ggagggaggc gaggaggcgg cggagttggc tgatggggtc     420
gggctgtggt atggtcggag caaggcgtac gaggcgcgca cgccgctgat ggtggcggcg     480
acgtacggca gcgccggggt ggtctcgctg ctggtgggcc tcggcggttg cgtcgacgtc     540
aaccgtcgcc ctggagccga cggcgccacc gcgctccact gcgccgcctc cggtggctcg     600
cgcaacgccg tcgctgttgt caagctgctt ttggccgctg gcgccgatcc ggccaccccc     660
gattccgccg gccgcttccc cgccgacgtc atcctagctc ctccggcttc gccagatgcc     720
cttggcgatc tcgaggtgct cctcggccgc cgccgagcac tcgccgtggc gacctcggtg     780
gcttcaggtt cgtcatcccc tccgctctcg tcctcaccag atgagggcaa caggtcgccc     840
tcgtcgcgtt cgtcgtcgct gtctcccatc actgtggatc gtgggaagaa ggagtatccg     900
gtggatccaa ctctgccgga catcaagagc agcgtgtatg cttcggatga gttccgcatg     960
tttgcgttca aggtccggcc ctgctcccgt gcctactcac acgactggac tgagtgcccg    1020
tttgtgcacc ccggcgagaa cgcccgccgc cgtgatcccc gcaagcaccc atacactgct    1080
gtgccttgcc ccaactttcg ccggcctggt ggctgcccta gcggcgatag ctgtgagttc    1140
tcgcatggcg tgtttgagag ctggctacac ccatcacagt atcgcacaag gctctgcaag    1200
gagggagcag cttgcgcccg tcgcatttgc ttctttgccc atgatgagga tgagctccgc    1260
catgtgcctc acaacagtgg tgccggcctg ctgtctcccc gcgcttcttc atccattgat    1320
atgactgctg cagctgcgct cgggcttctt ccaggttctc ctaccagaca ctttgcaccg    1380
ccgcctgtgt caccatctgc tgggagcaat ggaggagctg ctgctgcgca ttggctccaa    1440
ggcagtaggc tgcgttcttc tttcaatgca agggatgctg ctgttgatga ccttggcatg    1500
ctcctcgaat gggaatcaca ataccttggg gcactctgcc tgccacccag cagccgcccc    1560
caaccacgcc tttcagctgg tctgagtatc aggccaacaa ttgctccatc caatcttgaa    1620
gacatgtatg cttcagacat ggcaatgtct ccgaggttcc ctaatgacca aggtcactca    1680
gtctactcac cagcccacaa atcagccctc ctcaacaagc ttcatcaaca gaagggcctc    1740
ttatcacctg ttaacaccaa cagaatgtac tccccaaggg ctcttgatcc gtcatctttg    1800
gcacattctc catttggtgg catgtctccc cggtcccccc gtaccatgga acctacatca    1860
cccctaagtg ctcgtgtagg agcccctgcc acacagcggc cttctgttgg ttcaccacgg    1920
aattccagtg cttggggcac cgtggggtcc ccgatgggta aggttgactg ggtgtcgat     1980
agcgaggagc tagtccgctt gagacgccct gcacaaccag ggtttggaga agatgagaca    2040
gatgtatcat gggtgcagtc actggtaagc aatgctgagc ttaatggcaa gaggggcgaa    2100
gtacaaggca tgcctggtac ttctgcattg atgaacaggc ctgacctgaa caatcagggt    2160
gacttgttgg accagacggt gatcggtgct tggcttgagc agatgcacct ggatcagaag    2220
tgatttccaa gggaagccat gaagtcccaa agtggatgaa gcctttattt tgccaaggtt    2280
atttaccaaa gaatagttgt tggtcctagt aaataataat ttattctttt taattcttga    2340
aatttttggt gggcaaagtc agagatggtg gtcaagttca acaaaacatt tggtcacaga    2400
ttggtagctg aaatcagttc cagagattgg taaacaacct cattacttgg ggtcctaact    2460
agtattcttt tgattagctc agatgagtct ttattttagt gggttaaaat tcatatgttc    2520
cccatggtta ttatgtccat gatctcttcc taacaaaaga gagattataa ttgtccattt    2580
ttcatttatc aatgaatgat tttgttaaaa caatgtaagt tacattctta attttttctc    2640
tgttcaatgg aattaccttc cttggttagt cctc                                2674
```

<210> 21
<211> 657
<212> PRT

<213> Oryza sativa

<400> 21

```
Met Gly Glu Leu Ala Asp Leu Val Val Val Pro Ser Gln Pro Pro Leu
1               5                   10                  15
Ala Gly Gly Arg Arg Asp Arg Leu Ala Ala Leu Leu Glu Leu Ala Ala
            20                  25                  30
Ala Asp Asp Val Asp Gly Leu Arg Gly Ala Leu Ala Glu Gly Gly Glu
        35                  40                  45
Glu Ala Ala Glu Leu Ala Asp Gly Val Gly Leu Trp Tyr Gly Arg Ser
    50                  55                  60
Lys Ala Tyr Glu Ala Arg Thr Pro Leu Met Val Ala Ala Thr Tyr Gly
65                  70                  75                  80
Ser Ala Gly Val Val Ser Leu Leu Val Gly Leu Gly Gly Cys Val Asp
                85                  90                  95
Val Asn Arg Arg Pro Gly Ala Asp Gly Ala Thr Ala Leu His Cys Ala
            100                 105                 110
Ala Ser Gly Gly Ser Arg Asn Ala Val Ala Val Val Lys Leu Leu Leu
        115                 120                 125
Ala Ala Gly Ala Asp Pro Ala Thr Pro Asp Ser Ala Gly Arg Phe Pro
    130                 135                 140
Ala Asp Val Ile Leu Ala Pro Pro Ala Ser Pro Asp Ala Leu Gly Asp
145                 150                 155                 160
Leu Glu Val Leu Leu Gly Arg Arg Arg Ala Leu Ala Val Ala Thr Ser
                165                 170                 175
Val Ala Ser Gly Ser Ser Ser Pro Pro Leu Ser Ser Ser Pro Asp Glu
            180                 185                 190
Gly Asn Arg Ser Pro Ser Ser Arg Ser Ser Ser Leu Ser Pro Ile Thr
        195                 200                 205
Val Asp Arg Gly Lys Lys Glu Tyr Pro Val Asp Pro Thr Leu Pro Asp
    210                 215                 220
Ile Lys Ser Ser Val Tyr Ala Ser Asp Glu Phe Arg Met Phe Ala Phe
225                 230                 235                 240
Lys Val Arg Pro Cys Ser Arg Ala Tyr Ser His Asp Trp Thr Glu Cys
                245                 250                 255
Pro Phe Val His Pro Gly Glu Asn Ala Arg Arg Arg Asp Pro Arg Lys
            260                 265                 270
His Pro Tyr Thr Ala Val Pro Cys Pro Asn Phe Arg Arg Pro Gly Gly
        275                 280                 285
Cys Pro Ser Gly Asp Ser Cys Glu Phe Ser His Gly Val Phe Glu Ser
    290                 295                 300
Trp Leu His Pro Ser Gln Tyr Arg Thr Arg Leu Cys Lys Glu Gly Ala
305                 310                 315                 320
Ala Cys Ala Arg Arg Ile Cys Phe Phe Ala His Asp Glu Asp Glu Leu
                325                 330                 335
Arg His Val Pro His Asn Ser Gly Ala Gly Leu Leu Ser Pro Arg Ala
            340                 345                 350
Ser Ser Ser Ile Asp Met Thr Ala Ala Ala Ala Leu Gly Leu Leu Pro
        355                 360                 365
Gly Ser Pro Thr Arg His Phe Ala Pro Pro Pro Val Ser Pro Ser Ala
    370                 375                 380
Gly Ser Asn Gly Gly Ala Ala Ala Ala His Trp Leu Gln Gly Ser Arg
385                 390                 395                 400
Leu Arg Ser Ser Phe Asn Ala Arg Asp Ala Ala Val Asp Asp Leu Gly
                405                 410                 415
Met Leu Leu Glu Trp Glu Ser Gln Tyr Leu Gly Ala Leu Cys Leu Pro
            420                 425                 430
Pro Ser Ser Arg Pro Gln Pro Arg Leu Ser Ala Gly Leu Ser Ile Arg
        435                 440                 445
Pro Thr Ile Ala Pro Ser Asn Leu Glu Asp Met Tyr Ala Ser Asp Met
    450                 455                 460
```

```
Ala Met Ser Pro Arg Phe Pro Asn Asp Gln Gly His Ser Val Tyr Ser
465                 470                 475                 480
Pro Ala His Lys Ser Ala Leu Leu Asn Lys Leu His Gln Gln Lys Gly
                485                 490                 495
Leu Leu Ser Pro Val Asn Thr Asn Arg Met Tyr Ser Pro Arg Ala Leu
            500                 505                 510
Asp Pro Ser Ser Leu Ala His Ser Pro Phe Gly Gly Met Ser Pro Arg
        515                 520                 525
Ser Pro Arg Thr Met Glu Pro Thr Ser Pro Leu Ser Ala Arg Val Gly
    530                 535                 540
Ala Pro Ala Thr Gln Arg Pro Ser Val Gly Ser Pro Arg Asn Ser Ser
545                 550                 555                 560
Ala Trp Gly Thr Val Gly Ser Pro Met Gly Lys Val Asp Trp Gly Val
                565                 570                 575
Asp Ser Glu Glu Leu Val Arg Leu Arg Arg Pro Ala Gln Pro Gly Phe
            580                 585                 590
Gly Glu Asp Glu Thr Asp Val Ser Trp Val Gln Ser Leu Val Ser Asn
        595                 600                 605
Ala Glu Leu Asn Gly Lys Arg Gly Glu Val Gln Gly Met Pro Gly Thr
    610                 615                 620
Ser Ala Leu Met Asn Arg Pro Asp Leu Asn Asn Gln Gly Asp Leu Leu
625                 630                 635                 640
Asp Gln Thr Val Ile Gly Ala Trp Leu Glu Gln Met His Leu Asp Gln
                645                 650                 655
Lys
```

<210> 22
<211> 2223
<212> DNA
<213> Arabidopsis thaliana

<400> 22

```
ttcttcaaaa accccaacca cttcttctcc ccaaaaacct ccaaagtttc aatctttact      60
tctctctttt tctccaagtt atcttctttt ctaggaagag atatgtgcgg tgcaaagagc     120
aacctttgct catctaaaac cctaacagaa gtcgaattca tgaggcagaa atcagaagac     180
ggagcttccg ccacgtgtct cctcgaattc gccgcctgtg atgatctttc atcgtttaag     240
agagagatcg aagagaatcc atcggtggag attgatgagt cagggttttg gtattgcaga     300
cgggtcgggt ctaagaagat gggttttgaa gaaagaacac cacttatggt tgctgctatg     360
tatggaagca tggaagtgtt gaattacata attgccacag gaagatccga tgtgaacaga     420
gtttgcagtg acgagaaagt cactgctctt cactgtgcag tttctggctg ttctgtttct     480
atcgttgaga tcatcaagat cttgcttgat gcttctgctt cacctaattg tgttgacgct     540
aatgggaaca aaccggttga tttgttggct aaagattctc ggtttgttcc taaccagagt     600
agaaaggcgg ttgaggtttt actgaccggg attcatggtt cggttatgga agaagaggag     660
gaggaactga agagtgttgt gactaagtat ccagctgatg catcacttcc tgatattaac     720
gaaggtgttt atggaactga tgattttagg atgtttagct ttaaggttaa gccatgttct     780
agggcttatt cacatgattg gactgaatgt ccttttgttc atcctggtga gaatgcaagg     840
aggagagatc ctaggaagta tccttacact tgtgtgcctt gtcccgagtt tcgtaaaggg     900
tcttgtccta aaggagattc gtgtgagtac gcgcacggtg ttttcgagtc ttggcttcac     960
ccggcgcagt ataggacacg gctttgcaaa gatgagactg gttgtgctag gagagtttgt    1020
ttctttgctc atagacggga tgagttaaga ccggttaatg cttctactgg ttctgcaatg    1080
gtttcaccaa ggtcgtctaa tcagtctcct gagatgtctg ttatgtctcc tttgacgctg    1140
ggatcatcgc caatgaactc tcctatggct aatggtgttc ctttgtctcc aagaaatggt    1200
ggtttatggc agaacagagt taatagcctt acaccaccac cgttgcagct taatggtagc    1260
agattgaagt cgactttgag tgctagagat atggatatgg agatggaact taggtttcgc    1320
ggtttggata accggagact tggtgatctc aagccatcca acctcgaaga gactttcgga    1380
tcatatgact cagcttctgt gatgcaactt caatcaccaa gcaggcattc tcagatgaac    1440
cactatccgt cttcacctgt gaggcagcct cctcctcatg gattcgaatc ttcagcagcc    1500
atggcagctg cagtgatgaa tgcaagatcc tcagcgtttg cgaaacgcag cttgagtttc    1560

aaaccagctc cagtagcttc taatgtctcc gattggggat caccaaatgg gaagcttgag    1620
tggggaatgc aaagagatga gctgaacaag ttgaggagaa gtgcctcctt cggcattcat    1680
ggaaacaaca acaacagtgt gtcacgccct gctagagact acagtgacga gccagatgtg    1740
tcgtgggtga actcactggt gaaagagaat gcaccagaga gagtgaatga gagggttggg    1800
aatacggtga atggtgcagc gagtagagac aagtttaagc tgccgtcgtg ggcagagcaa    1860
atgtatatag accatgagca gcagattgtg gcataagaag cagaaagaaa gatgtgggat    1920
ttatattgct tttgtcttct gggcctctct acacagaatc taacaaatct ggcaataatt    1980
ctttgatttg tgtttgaccc atagtttggt tactagtata tgttttttta tgttcttttt    2040
ttctttgtca ttctcttgtc cttcgtgaca ctatgtaatg attaaaagca aataattgat    2100
gcatgagttc aaatgttctt tgaaggatcc atcttattag ctttgtaatt gttgtgatat    2160
cttaatctta ttggttacgt atttcaagtg ctttagaaaa aatgggccta agagattttg    2220
ggg                                                                  2223
```

<210> 23
<211> 597
<212> PRT
<213> Arabidopsis thaliana

<400> 23

```
Met Cys Gly Ala Lys Ser Asn Leu Cys Ser Ser Lys Thr Leu Thr Glu
1               5                   10                  15
Val Glu Phe Met Arg Gln Lys Ser Glu Asp Gly Ala Ser Ala Thr Cys
            20                  25                  30
Leu Leu Glu Phe Ala Ala Cys Asp Asp Leu Ser Ser Phe Lys Arg Glu
        35                  40                  45
Ile Glu Glu Asn Pro Ser Val Glu Ile Asp Glu Ser Gly Phe Trp Tyr
    50                  55                  60
Cys Arg Arg Val Gly Ser Lys Lys Met Gly Phe Glu Glu Arg Thr Pro
65                  70                  75                  80
Leu Met Val Ala Ala Met Tyr Gly Ser Met Glu Val Leu Asn Tyr Ile
                85                  90                  95
Ile Ala Thr Gly Arg Ser Asp Val Asn Arg Val Cys Ser Asp Glu Lys
            100                 105                 110
Val Thr Ala Leu His Cys Ala Val Ser Gly Cys Ser Val Ser Ile Val
        115                 120                 125
Glu Ile Ile Lys Ile Leu Leu Asp Ala Ser Ala Ser Pro Asn Cys Val
    130                 135                 140
Asp Ala Asn Gly Asn Lys Pro Val Asp Leu Leu Ala Lys Asp Ser Arg
145                 150                 155                 160
Phe Val Pro Asn Gln Ser Arg Lys Ala Val Glu Val Leu Leu Thr Gly
                165                 170                 175
Ile His Gly Ser Val Met Glu Glu Glu Glu Glu Glu Leu Lys Ser Val
            180                 185                 190
Val Thr Lys Tyr Pro Ala Asp Ala Ser Leu Pro Asp Ile Asn Glu Gly
        195                 200                 205
Val Tyr Gly Thr Asp Asp Phe Arg Met Phe Ser Phe Lys Val Lys Pro
    210                 215                 220
Cys Ser Arg Ala Tyr Ser His Asp Trp Thr Glu Cys Pro Phe Val His
225                 230                 235                 240
Pro Gly Glu Asn Ala Arg Arg Arg Asp Pro Arg Lys Tyr Pro Tyr Thr
                245                 250                 255
Cys Val Pro Cys Pro Glu Phe Arg Lys Gly Ser Cys Pro Lys Gly Asp
            260                 265                 270
Ser Cys Glu Tyr Ala His Gly Val Phe Glu Ser Trp Leu His Pro Ala
        275                 280                 285
Gln Tyr Arg Thr Arg Leu Cys Lys Asp Glu Thr Gly Cys Ala Arg Arg
    290                 295                 300
Val Cys Phe Phe Ala His Arg Arg Asp Glu Leu Arg Pro Val Asn Ala
305                 310                 315                 320
```

```
Ser Thr Gly Ser Ala Met Val Ser Pro Arg Ser Ser Asn Gln Ser Pro
                325                 330                 335
Glu Met Ser Val Met Ser Pro Leu Thr Leu Gly Ser Ser Pro Met Asn
            340                 345                 350
Ser Pro Met Ala Asn Gly Val Pro Leu Ser Pro Arg Asn Gly Gly Leu
        355                 360                 365
Trp Gln Asn Arg Val Asn Ser Leu Thr Pro Pro Pro Leu Gln Leu Asn
    370                 375                 380
Gly Ser Arg Leu Lys Ser Thr Leu Ser Ala Arg Asp Met Asp Met Glu
385                 390                 395                 400
Met Glu Leu Arg Phe Arg Gly Leu Asp Asn Arg Arg Leu Gly Asp Leu
                405                 410                 415
Lys Pro Ser Asn Leu Glu Glu Thr Phe Gly Ser Tyr Asp Ser Ala Ser
            420                 425                 430
Val Met Gln Leu Gln Ser Pro Ser Arg His Ser Gln Met Asn His Tyr
        435                 440                 445
Pro Ser Ser Pro Val Arg Gln Pro Pro Pro His Gly Phe Glu Ser Ser
    450                 455                 460
Ala Ala Met Ala Ala Ala Val Met Asn Ala Arg Ser Ser Ala Phe Ala
465                 470                 475                 480
Lys Arg Ser Leu Ser Phe Lys Pro Ala Pro Val Ala Ser Asn Val Ser
                485                 490                 495
Asp Trp Gly Ser Pro Asn Gly Lys Leu Glu Trp Gly Met Gln Arg Asp
            500                 505                 510
Glu Leu Asn Lys Leu Arg Arg Ser Ala Ser Phe Gly Ile His Gly Asn
        515                 520                 525
Asn Asn Asn Ser Val Ser Arg Pro Ala Arg Asp Tyr Ser Asp Glu Pro
    530                 535                 540
Asp Val Ser Trp Val Asn Ser Leu Val Lys Glu Asn Ala Pro Glu Arg
545                 550                 555                 560
Val Asn Glu Arg Val Gly Asn Thr Val Asn Gly Ala Ala Ser Arg Asp
                565                 570                 575
Lys Phe Lys Leu Pro Ser Trp Ala Glu Gln Met Tyr Ile Asp His Glu
            580                 585                 590
Gln Gln Ile Val Ala
        595
```

<210> 24
<211> 1761
<212> DNA
<213> Arabidopsis thaliana

<400> 24

```
atggaaaaag atagtattat gtgcagtgga ccaaagagca atctctgctc ttcaagaacc      60
ttaacagaaa tcgaatcaag gcaaaaggaa gaagaaacaa tgcttctcct cgaattcgct     120
gcttgtgatg atcttgactc gttcaagaga gaggttgaag agaaagggct tgatttggat     180
gagtcagggt tatggtattg cagacgtgtc ggttctaaga agatgggtct tgaagaaaga     240
acacctttaa tggttgcagc tatgtatgga agcataaagg ttttgacttt catcgtttcc     300
actggaaaat ctgatgtgaa cagagcttgt ggtgaagaga gagttactcc gcttcactgt     360
gctgttgctg gctgttctgt gaatatgatt gaagtcatca atgtcttgct tgatgcttct     420
gctttggtta actctgttga tgctaatggg aatcaacctt tggatgtgtt tgttcgagtt     480
tcgaggtttg tggctagtcc gaggaggaaa gcggttgagt tgttgctgag aggaggaggt     540
gttggaggat tgatcgatga ggcggttgaa gaagagatca agattgtctc taagtatcca     600
gctgatgctt ctttaccgga tataaacgaa ggggtttatg gaagtgatga gtttaggatg     660
tatagcttta aggttaagcc atgttctagg gcttattctc atgattggac cgagtgtgct     720
tttgttcatc cgggagaaaa tgcgaggagg agagatccga ggaagtatcc ttacacttgt     780
gtcccctgtc ccgagttccg taaaggatca tgcccgaaag gagattcttg cgagtatgct     840
cacggggttt tcgagtcgtg gcttcacccc gcgcagtata aaacccggct ttgtaaagat     900
gaaacgggtt gtgcaaggaa agtttgtttc tttgctcata aacgcgaaga gatgagacct     960

gttaatgctt caactggctc tgccgtggct cagtctccgt ttagcagctt ggagatgatg    1020
ccagggttgt ctcctcttgc ttattcttca ggagtttcga ctcctccggt ttctccaatg    1080
gctaatggtg ttccttcctc tccaagaaac ggcggatcat ggcagaacag agtcaatacc    1140
cttactccac cggctttgca gctcaatggt ggaagcagat tgaagtccac actgagcgct    1200
agagatatcg atatggagat ggagatggaa ttgagactcc gcggttttgg caacaatgtg    1260
gaagagacgt tcgggtctta tgtttcctct ccaagtagga attctcaaat gggtcaaaac    1320
atgaaccaac attatccatc ttccccggtg agacaaccgc catctcaaca cgggttcgaa    1380
tcttcagcag ctgcagcggt tgcagtgatg aaagcgagat caaccgcctt tgcgaaacgt    1440
agcttgagct tcaaaccagc tactcaagca gcaccacagt cgaatctctc ggattgggga    1500
tctccaaacg ggaagctgga atggggaatg aaaggagaag agctgaataa gatgagaaga    1560
agtgtttcct ttggaatcca tggaaacaac aacaataacg cagctagaga ctacagggac    1620
gagccagatg tgtcatgggt taactcttta gttaaagaca gtactgtggt gtctgagaga    1680
agctttggaa tgaatgagag ggttcggata atgtcgtggg ctgagcaaat gtacagagag    1740
aaggagcaga ctgtggtgta a                                              1761
```

<210> 25
<211> 586
<212> PRT
<213> Arabidopsis thaliana

<400> 25

```
Met Glu Lys Asp Ser Ile Met Cys Ser Gly Pro Lys Ser Asn Leu Cys
1               5                   10                  15
Ser Ser Arg Thr Leu Thr Glu Ile Glu Ser Arg Gln Lys Glu Glu Glu
            20                  25                  30
Thr Met Leu Leu Leu Glu Phe Ala Ala Cys Asp Asp Leu Asp Ser Phe
        35                  40                  45
Lys Arg Glu Val Glu Glu Lys Gly Leu Asp Leu Asp Glu Ser Gly Leu
    50                  55                  60
Trp Tyr Cys Arg Arg Val Gly Ser Lys Lys Met Gly Leu Glu Glu Arg
65                  70                  75                  80
Thr Pro Leu Met Val Ala Ala Met Tyr Gly Ser Ile Lys Val Leu Thr
                85                  90                  95
Phe Ile Val Ser Thr Gly Lys Ser Asp Val Asn Arg Ala Cys Gly Glu
            100                 105                 110
Glu Arg Val Thr Pro Leu His Cys Ala Val Ala Gly Cys Ser Val Asn
        115                 120                 125
Met Ile Glu Val Ile Asn Val Leu Leu Asp Ala Ser Ala Leu Val Asn
    130                 135                 140
Ser Val Asp Ala Asn Gly Asn Gln Pro Leu Asp Val Phe Val Arg Val
145                 150                 155                 160
Ser Arg Phe Val Ala Ser Pro Arg Arg Lys Ala Val Glu Leu Leu Leu
                165                 170                 175
Arg Gly Gly Gly Val Gly Gly Leu Ile Asp Glu Ala Val Glu Glu Glu
            180                 185                 190
Ile Lys Ile Val Ser Lys Tyr Pro Ala Asp Ala Ser Leu Pro Asp Ile
        195                 200                 205
Asn Glu Gly Val Tyr Gly Ser Asp Glu Phe Arg Met Tyr Ser Phe Lys
    210                 215                 220
Val Lys Pro Cys Ser Arg Ala Tyr Ser His Asp Trp Thr Glu Cys Ala
225                 230                 235                 240
Phe Val His Pro Gly Glu Asn Ala Arg Arg Arg Asp Pro Arg Lys Tyr
                245                 250                 255
Pro Tyr Thr Cys Val Pro Cys Pro Glu Phe Arg Lys Gly Ser Cys Pro
            260                 265                 270
Lys Gly Asp Ser Cys Glu Tyr Ala His Gly Val Phe Glu Ser Trp Leu
        275                 280                 285
His Pro Ala Gln Tyr Lys Thr Arg Leu Cys Lys Asp Glu Thr Gly Cys
    290                 295                 300
```

```
Ala Arg Lys Val Cys Phe Phe Ala His Lys Arg Glu Glu Met Arg Pro
305                 310                 315                 320
Val Asn Ala Ser Thr Gly Ser Ala Val Ala Gln Ser Pro Phe Ser Ser
                325                 330                 335
Leu Glu Met Met Pro Gly Leu Ser Pro Leu Ala Tyr Ser Ser Gly Val
            340                 345                 350
Ser Thr Pro Pro Val Ser Pro Met Ala Asn Gly Val Pro Ser Ser Pro
        355                 360                 365
Arg Asn Gly Gly Ser Trp Gln Asn Arg Val Asn Thr Leu Thr Pro Pro
    370                 375                 380
Ala Leu Gln Leu Asn Gly Gly Ser Arg Leu Lys Ser Thr Leu Ser Ala
385                 390                 395                 400
Arg Asp Ile Asp Met Glu Met Glu Met Glu Leu Arg Leu Arg Gly Phe
                405                 410                 415
Gly Asn Asn Val Glu Glu Thr Phe Gly Ser Tyr Val Ser Ser Pro Ser
            420                 425                 430
Arg Asn Ser Gln Met Gly Gln Asn Met Asn Gln His Tyr Pro Ser Ser
        435                 440                 445
Pro Val Arg Gln Pro Pro Ser Gln His Gly Phe Glu Ser Ser Ala Ala
    450                 455                 460
Ala Ala Val Ala Val Met Lys Ala Arg Ser Thr Ala Phe Ala Lys Arg
465                 470                 475                 480
Ser Leu Ser Phe Lys Pro Ala Thr Gln Ala Ala Pro Gln Ser Asn Leu
                485                 490                 495
Ser Asp Trp Gly Ser Pro Asn Gly Lys Leu Glu Trp Gly Met Lys Gly
            500                 505                 510
Glu Glu Leu Asn Lys Met Arg Arg Ser Val Ser Phe Gly Ile His Gly
        515                 520                 525
Asn Asn Asn Asn Asn Ala Ala Arg Asp Tyr Arg Asp Glu Pro Asp Val
    530                 535                 540
Ser Trp Val Asn Ser Leu Val Lys Asp Ser Thr Val Val Ser Glu Arg
545                 550                 555                 560
Ser Phe Gly Met Asn Glu Arg Val Arg Ile Met Ser Trp Ala Glu Gln
                565                 570                 575
Met Tyr Arg Glu Lys Glu Gln Thr Val Val
            580                 585
```

<210> 26
<211> 2709
<212> DNA
<213> Eucalyptus grandis

<400> 26

```
cttctgaaag ctttttgact taagacgaga gagaaggaga gaaggtcccc ctcctcgtcc     60
tcgtcccccc gtggattttg aagaagaaaa gtcgcacctt ccttctcctt tcccactcct    120
ccctctgctc gaagcttttc tcttccgcag aattacataa aaacctcgac tttgcgcatc    180
attccgattc acctcacacc ttcactttcc cactcgaggt ctcccccctc ttttcctagc    240
tctttccctt tccctccctc tctctcgaga atcgccgcat ttggaggagc tccaatctgc    300
tttgctttgc tttgctctct tcttgctcgg ttccccctca taaggagtcg attatgtgca    360
acggttcttc gaagggtaaa cttttcccct cgagtatggg catggagggc gaattccaca    420
acaaggatgg cgaagcaccc cgtaaatgct ctgccttgct tgaattggca gcctcggacg    480
atctctcgtc gttcaaaagt gaagtggaag agaagggctg cgacgttgat gaggccagct    540
tttggtatgg taggagaatc gggtcgaaga agatgggttt tgaagagagg actccattga    600
tgatctctgc tttgtttgga agcaccaagg tcttgaaata cataatcgag accgccagag    660
ctgatgtcaa caggtcttgt gggtccgaca aggtggccgc cctccattgc gcagccgcgg    720
gtgggtccag ttcttcactt gaaattgtga agctcttgat tgaggcctca gcggatatta    780
attctgtaga tggcaatgga aataggccca tcgacgtgct tgccccggca gggaagtctc    840
gctgcaattc cagaaataag tttgttagat cgttgctgaa aggtgaaaac tatgtcgtgg    900
aaggtgacca atcctttgac atagaaggag aggagaagct agtcgctctt ccaaaggagg    960

gaggcgagaa gaaagagtat cctgttgatg tctctctacc tgacataaac aatgggttct   1020
acagtaccga tgagttccgg atgtatgctt tcaaggtgaa gccttgctcg agggcttact   1080
cccacgactg gaccgagtgc ccgtttgtgc accctgggga gaacgcgagg aggagggacc   1140
cacgcaagta cccttacagc tgtgtccctt gtcctgagtt tcgcaagggt tcgtgcgtaa   1200
ggggggatgc ttgtgagtat gctcatggag tctttgagtc gtggcttcac ccagcgcaat   1260
accgaacccg gctgtgcaag gatgaaactg gttgtactcg caaagtttgc ttctttgctc   1320
acaagtccga agaattgcgt cccgtgtatg cttccacagg ttctgctatg ccctcaccca   1380
agtccttttc agctaatgcc ctagacatga caaccctgag cccccttatcc cttaattcac   1440
catctctgcc tttgcctgct acttccacgc cccccatgtc acctttggct gcctcatctt   1500
cacccaaggg catgaacttg tggcataaca aaattaacct gaccccacca agcctgcagc   1560
ttcctggcag ccggctgaag acggctatga gtgcgcggga cttcgatttt gagttggaat   1620
ttcttgggct ggaaaagcaa gcttctcagc ggcagcaact gatagaagag atttctcgtc   1680
tctcatcgcc ctctcatatg tggaactcgg aatttggcag aaccgcagag ctgaagccca   1740
ctaaccttga tgatgcgttt ggatctcttg acacttctct tttgtctccg ttgcaggggt   1800
cgtcgatgaa aacatcgact cctacccagt tgcaatcccc cacagggctt aaaatttcga   1860
atttgaacca actccgtgcg agctacccgt ctagcagctt gtcgtcctct cctgtgagga   1920
agacctcttc ttttgggttc gactcatcca gtgcagttgc tgcagcagtc atgaactcac   1980
ggtctgctgc tatgacgaag cggagccaga gcttcattga ccgtggagca gtgggtcaac   2040
ggtctggact cattggacct gctaattctg ctcctaggat gtccaacctt tcggactggg   2100
gctcgcctga tgggaagttg gattggggtg ttcaagggga cgagctcaac aagcttagga   2160
agtccgcttc cttcggcttt agaaacaaca gtatggcgaa cccaaacaac gtggcgtctc   2220
ccagtgctga tgagccggac gtgtcgtggg ttggttcatt ggtgaaggat gtggctccgc   2280
ccgaagggta tccacagtat ctgtacatag aacaggagca gatggtggca taactaaagc   2340
gaagagcacc acacgaactc tctcctgatg gcttaagatg acttgtttga cattctttat   2400
attcttacaa acagcgcgtt cttaggagtt agctggagga aagaaggaaa cggtattgag   2460
tttgagattc aggctcttag ctggacagcg aaaatttggg gaaggaagag aatttggttt   2520
cttgcccaac ttagataatg atgcttttga aggcttaaaa gaaagatgaa ggcaaacatt   2580
cttttgttag tattgtatta ttgttttaat ttttcatccc ctctgtcggg gtgtggtggg   2640
tgtcgatgtt tctttcatca gtaaaatata taatgaggtt tactcatcta ttttctacta   2700
aaaaaaaaa                                                          2709
```

<210> 27
<211> 659
<212> PRT
<213> Eucalyptus grandis

<400> 27

```
Met Cys Asn Gly Ser Ser Lys Gly Lys Leu Phe Pro Ser Ser Met Gly
1               5                   10                  15
Met Glu Gly Glu Phe His Asn Lys Asp Gly Glu Ala Pro Arg Lys Cys
            20                  25                  30
Ser Ala Leu Leu Glu Leu Ala Ala Ser Asp Asp Leu Ser Ser Phe Lys
        35                  40                  45
Ser Glu Val Glu Glu Lys Gly Cys Asp Val Asp Glu Ala Ser Phe Trp
    50                  55                  60
Tyr Gly Arg Arg Ile Gly Ser Lys Lys Met Gly Phe Glu Glu Arg Thr
65                  70                  75                  80
Pro Leu Met Ile Ser Ala Leu Phe Gly Ser Thr Lys Val Leu Lys Tyr
                85                  90                  95
Ile Ile Glu Thr Ala Arg Ala Asp Val Asn Arg Ser Cys Gly Ser Asp
            100                 105                 110
Lys Val Ala Ala Leu His Cys Ala Ala Ala Gly Gly Ser Ser Ser Ser
        115                 120                 125
Leu Glu Ile Val Lys Leu Leu Ile Glu Ala Ser Ala Asp Ile Asn Ser
    130                 135                 140
Val Asp Gly Asn Gly Asn Arg Pro Ile Asp Val Leu Ala Pro Ala Gly
145                 150                 155                 160
Lys Ser Arg Cys Asn Ser Arg Asn Lys Phe Val Arg Ser Leu Leu Lys
                165                 170                 175
```

```
Gly Glu Asn Tyr Val Val Glu Gly Asp Gln Ser Phe Asp Ile Glu Gly
            180                 185                 190
Glu Glu Lys Leu Val Ala Leu Pro Lys Glu Gly Gly Glu Lys Lys Glu
        195                 200                 205
Tyr Pro Val Asp Val Ser Leu Pro Asp Ile Asn Asn Gly Phe Tyr Ser
    210                 215                 220
Thr Asp Glu Phe Arg Met Tyr Ala Phe Lys Val Lys Pro Cys Ser Arg
225                 230                 235                 240
Ala Tyr Ser His Asp Trp Thr Glu Cys Pro Phe Val His Pro Gly Glu
                245                 250                 255
Asn Ala Arg Arg Arg Asp Pro Arg Lys Tyr Pro Tyr Ser Cys Val Pro
            260                 265                 270
Cys Pro Glu Phe Arg Lys Gly Ser Cys Val Arg Gly Asp Ala Cys Glu
        275                 280                 285
Tyr Ala His Gly Val Phe Glu Ser Trp Leu His Pro Ala Gln Tyr Arg
    290                 295                 300
Thr Arg Leu Cys Lys Asp Glu Thr Gly Cys Thr Arg Lys Val Cys Phe
305                 310                 315                 320
Phe Ala His Lys Ser Glu Glu Leu Arg Pro Val Tyr Ala Ser Thr Gly
                325                 330                 335
Ser Ala Met Pro Ser Pro Lys Ser Phe Ser Ala Asn Ala Leu Asp Met
            340                 345                 350
Thr Thr Leu Ser Pro Leu Ser Leu Asn Ser Pro Ser Leu Pro Leu Pro
        355                 360                 365
Ala Thr Ser Thr Pro Pro Met Ser Pro Leu Ala Ala Ser Ser Ser Pro
    370                 375                 380
Lys Gly Met Asn Leu Trp His Asn Lys Ile Asn Leu Thr Pro Pro Ser
385                 390                 395                 400
Leu Gln Leu Pro Gly Ser Arg Leu Lys Thr Ala Met Ser Ala Arg Asp
                405                 410                 415
Phe Asp Phe Glu Leu Glu Phe Leu Gly Leu Glu Lys Gln Ala Ser Gln
            420                 425                 430
Arg Gln Gln Leu Ile Glu Glu Ile Ser Arg Leu Ser Ser Pro Ser His
        435                 440                 445
Met Trp Asn Ser Glu Phe Gly Arg Thr Ala Glu Leu Lys Pro Thr Asn
    450                 455                 460
Leu Asp Asp Ala Phe Gly Ser Leu Asp Thr Ser Leu Leu Ser Pro Leu
465                 470                 475                 480
Gln Gly Ser Ser Met Lys Thr Ser Thr Pro Thr Gln Leu Gln Ser Pro
                485                 490                 495
Thr Gly Leu Lys Ile Ser Asn Leu Asn Gln Leu Arg Ala Ser Tyr Pro
            500                 505                 510
Ser Ser Ser Leu Ser Ser Ser Pro Val Arg Lys Thr Ser Ser Phe Gly
        515                 520                 525
Phe Asp Ser Ser Ser Ala Val Ala Ala Ala Val Met Asn Ser Arg Ser
    530                 535                 540
Ala Ala Met Thr Lys Arg Ser Gln Ser Phe Ile Asp Arg Gly Ala Val
545                 550                 555                 560
Gly Gln Arg Ser Gly Leu Ile Gly Pro Ala Asn Ser Ala Pro Arg Met
                565                 570                 575
Ser Asn Leu Ser Asp Trp Gly Ser Pro Asp Gly Lys Leu Asp Trp Gly
            580                 585                 590
Val Gln Gly Asp Glu Leu Asn Lys Leu Arg Lys Ser Ala Ser Phe Gly
        595                 600                 605
Phe Arg Asn Asn Ser Met Ala Asn Pro Asn Asn Val Ala Ser Pro Ser
    610                 615                 620
Ala Asp Glu Pro Asp Val Ser Trp Val Gly Ser Leu Val Lys Asp Val
625                 630                 635                 640
Ala Pro Pro Glu Gly Tyr Pro Gln Tyr Leu Tyr Ile Glu Gln Glu Gln
```

```
                    645                 650                 655
Met Val Ala
```

<210> 28
<211> 2518
<212> DNA
<213> Eucalyptus grandis

<400> 28

```
tctccttcga gtttctttct tcactagaat tcgctcccga gtctgttgtt gctcgtgtag      60
ttttgcttac tccgtccttc gtttagctcg ctgaccagcg cggagctagg agcggtcgct     120
aaaggattac tcgtacaaaa cgtaaactca gctctgccaa ttttcccatg gagggggaat     180
cttacttcga gaaagatgaa aaatattcta attgctcaat cttgctcgaa ttatctgctt     240
cggacgatct cccagctttt gaaaggaaag cgaaagagaa gggctgtaac attgatggtg     300
ctagcttctg gtacggtaga agaattggct caaggaagat gggtcttgaa gagaggactc     360
ctctcatggt ggcttccttg tttggaagct ctagggttgt gaagtacatt ctcgaatctg     420
gcaaagtcga tgtaaatagg gcttgtggtt cggacaaggt cactgccctt cactgtgctg     480
ttgccagtgg ctctgcttct gcggtggagg ttgtcaagct cttgcttcac gcatctgccg     540
atgctaattg cattgatggc aatggaaaga agccaattga tgtgatagcc cttccattaa     600
agtcacgcgg cgattcaagg aggaagctga tggagctgtt gctgaaaggc gataattctg     660
atggggaatt tgaatcccac gaggagaagc cgattgccgc accgcaagca tccaaagagg     720
gaagcgaaaa gaaagagtat caatttcctg ttgatatctc tctgcctgac ataaatgttg     780
ggatttacag tactgatgag ttcagaatgt atgctttcaa agtaaagcct tgctcgcggg     840
catactccca tgactggaca gagtgcccat ttgttcatcc tggcgagaat gcgaggaggc     900
gggaccctcg caagtacccc tacagctgcg tcccttgccc tgaatttcgg aagggatctt     960
gccaaaaggg tgactcctgt gagtacgcgc acggcgtatt tgagtcgtgg cttcatcctg    1020
cacagtatag aacaagactg tgcaaggatg agactggatg tgctcgcaaa gtttgtttct    1080
ttgctcacaa gcccgaagaa ttaaggcctg tctatgcttc gacgggatca gctatgcctt    1140
ccccaaaatc ctactcatca agtgggctgg acatgtccac attgagtcct ctctcaatca    1200
gttctccgtc agcatcgttg cctgttactt caacagcacc catgtctcct cttgcagcct    1260
cgtcatctcc gatgtctgtg aacatgtggc agagcaaggc taacaagctc tccccgccaa    1320
tgctgcagct ctcaggtagt aggctgaaga ctgctttgag tgctagggac ttggacctgg    1380
agatggaatt gcgtggtcta gagagtcaga tggccactca acagcatcag ttgatggaag    1440
agatatctcg tctctcctca ccatcatcct gctttagtag taggattggg gaagtgaaac    1500
ccactaacct cgatgacgtt tttgggtctc cggatcctgc tttgctgcct caattgcagg    1560
ggctgtcaag accttcaaca ccaagccagt tgcaatctcc aactgggctt cagatgcgcc    1620
agaatgcaac ccagtttcgt ggggcgtacc agagcaatgc aaatgcattg tcatctccag    1680
caatgaagca ggcaccttct tatgggtttg actcatctag tgcagttgca gcagcggtga    1740
tgaattcgag gtcagccgct tttgcgaagc ggagtcagag ttttatcgac aggggaatgg    1800
cgtgccctgg aattgccaat tcttccccta tgatgtcttc agctatgtcg agctggagct    1860
cacctcatgg gaaattggat tggggcgtcc aaggagatga gttgaatagg ctgaggaaag    1920
ctgcttcctt taagatgaga agcagcaccg gagcaggtgc taatactgtc tcggcagcag    1980
ccatggctga tgagccagat atttcttggg tcagttcatt ggttaaggac gtgccttctg    2040
cggaggacgc gatgttcgct gcagagaaag gacagcgcac ttatgggaaa gacatccgcg    2100
aaaggattac cccatgggtg gagcagctgt acagagaagt gccacggatg gcgatgtaag    2160
attgccactg caagtcggat gccttagtat gctgactaat tgatattctt tgcatttgtt    2220
ttgaggcatt tggtagccat tagatacgag aaaaggccaa gcagcaggtg gtgtcttggc    2280
aaggaatagg atgcacatag tctgttatcg agtagaatag acttgggaac aatggttata    2340
gccaaatgtt aaaagttatg atattctttt ccaattcttt ctcttcctca tagtaggttt    2400
ctcaccaagt cttttagtga gagcctgcgg gatgtactat atgtttccct tatgtaacgt    2460
ctcttcgttg aaagaaatgg ctttataata taaagcatca agttttttaa aaaaaaa       2518
```

<210> 29
<211> 663
<212> PRT
<213> Eucalyptus grandis

<400> 29

```
Met Glu Gly Glu Ser Tyr Phe Glu Lys Asp Glu Lys Tyr Ser Asn Cys
1               5                   10                  15
Ser Ile Leu Leu Glu Leu Ser Ala Ser Asp Asp Leu Pro Ala Phe Glu
            20                  25                  30
Arg Lys Ala Lys Glu Lys Gly Cys Asn Ile Asp Gly Ala Ser Phe Trp
        35                  40                  45
Tyr Gly Arg Arg Ile Gly Ser Arg Lys Met Gly Leu Glu Glu Arg Thr
    50                  55                  60
Pro Leu Met Val Ala Ser Leu Phe Gly Ser Ser Arg Val Val Lys Tyr
65                  70                  75                  80
Ile Leu Glu Ser Gly Lys Val Asp Val Asn Arg Ala Cys Gly Ser Asp
                85                  90                  95
Lys Val Thr Ala Leu His Cys Ala Val Ala Ser Gly Ser Ala Ser Ala
            100                 105                 110
Val Glu Val Val Lys Leu Leu Leu His Ala Ser Ala Asp Ala Asn Cys
        115                 120                 125
Ile Asp Gly Asn Gly Lys Lys Pro Ile Asp Val Ile Ala Leu Pro Leu
    130                 135                 140
Lys Ser Arg Gly Asp Ser Arg Arg Lys Leu Met Glu Leu Leu Leu Lys
145                 150                 155                 160
Gly Asp Asn Ser Asp Gly Glu Phe Glu Ser His Glu Glu Lys Pro Ile
                165                 170                 175
Ala Ala Pro Gln Ala Ser Lys Glu Gly Ser Glu Lys Lys Glu Tyr Gln
            180                 185                 190
Phe Pro Val Asp Ile Ser Leu Pro Asp Ile Asn Val Gly Ile Tyr Ser
        195                 200                 205
Thr Asp Glu Phe Arg Met Tyr Ala Phe Lys Val Lys Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Tyr Pro Tyr Ser Cys Val Pro
                245                 250                 255
Cys Pro Glu Phe Arg Lys Gly Ser Cys Gln Lys Gly Asp Ser Cys Glu
            260                 265                 270
Tyr Ala His Gly Val Phe Glu Ser Trp Leu His Pro Ala Gln Tyr Arg
        275                 280                 285
Thr Arg Leu Cys Lys Asp Glu Thr Gly Cys Ala Arg Lys Val Cys Phe
    290                 295                 300
Phe Ala His Lys Pro Glu Glu Leu Arg Pro Val Tyr Ala Ser Thr Gly
305                 310                 315                 320
Ser Ala Met Pro Ser Pro Lys Ser Tyr Ser Ser Ser Gly Leu Asp Met
                325                 330                 335
Ser Thr Leu Ser Pro Leu Ser Ile Ser Ser Pro Ser Ala Ser Leu Pro
            340                 345                 350
Val Thr Ser Thr Ala Pro Met Ser Pro Leu Ala Ala Ser Ser Ser Pro
        355                 360                 365
Met Ser Val Asn Met Trp Gln Ser Lys Ala Asn Lys Leu Ser Pro Pro
    370                 375                 380
Met Leu Gln Leu Ser Gly Ser Arg Leu Lys Thr Ala Leu Ser Ala Arg
385                 390                 395                 400
Asp Leu Asp Leu Glu Met Glu Leu Arg Gly Leu Glu Ser Gln Met Ala
                405                 410                 415
Thr Gln Gln His Gln Leu Met Glu Glu Ile Ser Arg Leu Ser Ser Pro
            420                 425                 430
Ser Ser Cys Phe Ser Ser Arg Ile Gly Glu Val Lys Pro Thr Asn Leu
        435                 440                 445
Asp Asp Val Phe Gly Ser Pro Asp Pro Ala Leu Leu Pro Gln Leu Gln
    450                 455                 460
Gly Leu Ser Arg Pro Ser Thr Pro Ser Gln Leu Gln Ser Pro Thr Gly
```

```
465                 470                 475                 480
Leu Gln Met Arg Gln Asn Ala Thr Gln Phe Arg Gly Ala Tyr Gln Ser
                485                 490                 495
Asn Ala Asn Ala Leu Ser Ser Pro Ala Met Lys Gln Ala Pro Ser Tyr
            500                 505                 510
Gly Phe Asp Ser Ser Ser Ala Val Ala Ala Ala Val Met Asn Ser Arg
        515                 520                 525
Ser Ala Ala Phe Ala Lys Arg Ser Gln Ser Phe Ile Asp Arg Gly Met
    530                 535                 540
Ala Cys Pro Gly Ile Ala Asn Ser Ser Pro Met Met Ser Ser Ala Met
545                 550                 555                 560
Ser Ser Trp Ser Ser Pro His Gly Lys Leu Asp Trp Gly Val Gln Gly
                565                 570                 575
Asp Glu Leu Asn Arg Leu Arg Lys Ala Ala Ser Phe Lys Met Arg Ser
            580                 585                 590
Ser Thr Gly Ala Gly Ala Asn Thr Val Ser Ala Ala Ala Met Ala Asp
        595                 600                 605
Glu Pro Asp Ile Ser Trp Val Ser Ser Leu Val Lys Asp Val Pro Ser
    610                 615                 620
Ala Glu Asp Ala Met Phe Ala Ala Glu Lys Gly Gln Arg Thr Tyr Gly
625                 630                 635                 640
Lys Asp Ile Arg Glu Arg Ile Thr Pro Trp Val Glu Gln Leu Tyr Arg
                645                 650                 655
Glu Val Pro Arg Met Ala Met
            660
```

<210> 30
<211> 2001
<212> DNA
<213> Triticum aestivum

<220>
<221> misc_feature
<222> (481)..(530)
<223> n is a, c, g, or t

<400> 30

```
cgaattccgg tcgacgattt ctcgatttcc ttctctataa cacaacgctc tcttctcttg      60
caaccaaagt acttgttcca gtgtctactc tactcaaaaa ggatttggga catcatgtgc     120
agtgattcga aaagtaaact ttcttcccca accctcgtcg tcatggagaa tagtaacatt     180
cagaagcaga atctggatgg tctctacaac tcggttttgc ttgaattgtc tgcatctgat     240
gattatgaag ctttcaaaag agaggtggag gaaaaaggct tagatgtgaa cgaggcaggc     300
ttttggtacg gtagaagaat tgggtcaaag aagatgggat ctgaaacgag gacccctctg     360
atgattgctt ctttgtttgg aagcgccaag gtgctcaatt atattcttct tcagaaagga     420
ggaggtgttg atgtgaacag ggtctgtggt tctgataggg ccactgctct ccattgtgct     480
nnnnnnnnnn nnnnnnnnnn nnnnnnnnnn nnnnnnnnnn nnnnnnnnnn tcccgggtcg     540
cgatttcgta ggagattcac agagagaaaa gaagcaatta gtgataataa gaaagaatac     600
cctgttgata tatcactgcc agacataaac aacggtgtat atggaacaga tgattttagg     660
atgtacaact tcaaggtgaa gccttgctca agggcttact cccatgactg gaccgagtgt     720
ccattcgttc acccagggga gaacgctagg aggagagacc cacggaaata cccttacagc     780
tgtgttcctt gccctgagtt ccgcaaaggg acctgccaga agggtgattc ctgtgagtat     840
gctcatggtg tttttgagtc ctggctgcat cctgcccaat accggacaag gctttgcaag     900
gatgagactg gctgcgctag aaaagtctgc ttctttgccc acaaacctga agagctacgc     960
cctgtgtatg cttccactgg gtcggctatg ccatcaccaa aatcatattc agctagtgga    1020
cttgacatga cagcgatgag tccattggct ctaagttcca catctttgcc taatgccccc    1080
ccgtttccag cctcacccta tcgtgcgccc tcgttcttct ctcagagtga agctgtgcag    1140
aacaaaataa accttactcc accatcgttg cagctccctg gtagccgact gaaggctgct    1200
ttgagtgcca gggatctgga gatggagatg gaactgctcg gtctagaaag ccctgctcgc    1260
caacaacagc agcagcagca acaattgatc gaagagattg ccaggatctc ttccccatct    1320

ttccggagca aggaattcaa taggattgtt gatttgaatc ctactaacct tgatgacctg    1380
ttagcatctg ctgacccttc tgtattttct caactacatg gactttctgt gcaaccttca    1440
acacccacac aaagtgggct tcagatgcgc caaaacatga accacctccg tgcgagttat    1500
ccatccaaca tcccttcctc tcctgtgagg aagccctcag cttttgggtt tgactcatca    1560
gctgctgtgg caactgcagt gatgaattct aggtctgctg ccttcgcaaa gcgaagccaa    1620
agtttcattg atcgtggagc tgcaacccac catcttgggc tgtcttcagc ttccaactct    1680
tcttgcaggg tatcctctac cctttcagat tggagttccc ctaccgggaa actggattgg    1740
ggtgtaaacg gagacaagct gaacaagctg aggaaatcta cttcctttgg attcagaaac    1800
agtggggtaa ctgcatcccc catagcacag cctgaatttg gtgctgagcc ggatgtctca    1860
tgggttcatt cattggttaa agatgttccc tccgagaggt ctgagatatt tggtgctgag    1920
aagcaacaat atgatctcag taaagagatg cttccaccat ggatggagca gctgtatata    1980
gagcaggagc agatggtagc a                                               2001
```

<210> 31
<211> 667
<212> PRT
<213> Triticum aestivum

<220>
<221> UNSURE
<222> (161)..(177)
<223> Xaa can be any naturally occurring amino acid

<400> 31

```
Arg Ile Pro Val Asp Asp Phe Ser Ile Ser Phe Ser Ile Thr Gln Arg
1               5                   10                  15
Ser Leu Leu Leu Gln Pro Lys Tyr Leu Phe Gln Cys Leu Leu Tyr Ser
            20                  25                  30
Lys Arg Ile Trp Asp Ile Met Cys Ser Asp Ser Lys Ser Lys Leu Ser
        35                  40                  45
Ser Pro Thr Leu Val Val Met Glu Asn Ser Asn Ile Gln Lys Gln Asn
    50                  55                  60
Leu Asp Gly Leu Tyr Asn Ser Val Leu Leu Glu Leu Ser Ala Ser Asp
65                  70                  75                  80
Asp Tyr Glu Ala Phe Lys Arg Glu Val Glu Glu Lys Gly Leu Asp Val
                85                  90                  95
Asn Glu Ala Gly Phe Trp Tyr Gly Arg Arg Ile Gly Ser Lys Lys Met
            100                 105                 110
Gly Ser Glu Thr Arg Thr Pro Leu Met Ile Ala Ser Leu Phe Gly Ser
        115                 120                 125
Ala Lys Val Leu Asn Tyr Ile Leu Leu Gln Lys Gly Gly Gly Val Asp
    130                 135                 140
Val Asn Arg Val Cys Gly Ser Asp Arg Ala Thr Ala Leu His Cys Ala
145                 150                 155                 160
Xaa Xaa Xaa Xaa Xaa Xaa Xaa Xaa Xaa Xaa Xaa Xaa Xaa Xaa Xaa Xaa
                165                 170                 175
Xaa Pro Gly Ser Arg Phe Arg Arg Arg Phe Thr Glu Arg Lys Glu Ala
            180                 185                 190
Ile Ser Asp Asn Lys Lys Glu Tyr Pro Val Asp Ile Ser Leu Pro Asp
        195                 200                 205
Ile Asn Asn Gly Val Tyr Gly Thr Asp Asp Phe Arg Met Tyr Asn Phe
    210                 215                 220
Lys Val Lys Pro Cys Ser Arg Ala Tyr Ser His Asp Trp Thr Glu Cys
225                 230                 235                 240
Pro Phe Val His Pro Gly Glu Asn Ala Arg Arg Arg Asp Pro Arg Lys
                245                 250                 255
Tyr Pro Tyr Ser Cys Val Pro Cys Pro Glu Phe Arg Lys Gly Thr Cys
            260                 265                 270
Gln Lys Gly Asp Ser Cys Glu Tyr Ala His Gly Val Phe Glu Ser Trp
```

```
        275                 280                 285
Leu His Pro Ala Gln Tyr Arg Thr Arg Leu Cys Lys Asp Glu Thr Gly
    290                 295                 300
Cys Ala Arg Lys Val Cys Phe Phe Ala His Lys Pro Glu Glu Leu Arg
305                 310                 315                 320
Pro Val Tyr Ala Ser Thr Gly Ser Ala Met Pro Ser Pro Lys Ser Tyr
                325                 330                 335
Ser Ala Ser Gly Leu Asp Met Thr Ala Met Ser Pro Leu Ala Leu Ser
            340                 345                 350
Ser Thr Ser Leu Pro Asn Ala Pro Pro Phe Pro Ala Ser Pro Tyr Arg
        355                 360                 365
Ala Pro Ser Phe Phe Ser Gln Ser Glu Ala Val Gln Asn Lys Ile Asn
    370                 375                 380
Leu Thr Pro Pro Ser Leu Gln Leu Pro Gly Ser Arg Leu Lys Ala Ala
385                 390                 395                 400
Leu Ser Ala Arg Asp Leu Glu Met Glu Met Glu Leu Leu Gly Leu Glu
                405                 410                 415
Ser Pro Ala Arg Gln Gln Gln Gln Gln Gln Gln Gln Leu Ile Glu Glu
            420                 425                 430
Ile Ala Arg Ile Ser Ser Pro Ser Phe Arg Ser Lys Glu Phe Asn Arg
        435                 440                 445
Ile Val Asp Leu Asn Pro Thr Asn Leu Asp Asp Leu Leu Ala Ser Ala
    450                 455                 460
Asp Pro Ser Val Phe Ser Gln Leu His Gly Leu Ser Val Gln Pro Ser
465                 470                 475                 480
Thr Pro Thr Gln Ser Gly Leu Gln Met Arg Gln Asn Met Asn His Leu
                485                 490                 495
Arg Ala Ser Tyr Pro Ser Asn Ile Pro Ser Ser Pro Val Arg Lys Pro
            500                 505                 510
Ser Ala Phe Gly Phe Asp Ser Ser Ala Ala Val Ala Thr Ala Val Met
        515                 520                 525
Asn Ser Arg Ser Ala Ala Phe Ala Lys Arg Ser Gln Ser Phe Ile Asp
    530                 535                 540
Arg Gly Ala Ala Thr His His Leu Gly Leu Ser Ser Ala Ser Asn Ser
545                 550                 555                 560
Ser Cys Arg Val Ser Ser Thr Leu Ser Asp Trp Ser Ser Pro Thr Gly
                565                 570                 575
Lys Leu Asp Trp Gly Val Asn Gly Asp Lys Leu Asn Lys Leu Arg Lys
            580                 585                 590
Ser Thr Ser Phe Gly Phe Arg Asn Ser Gly Val Thr Ala Ser Pro Ile
        595                 600                 605
Ala Gln Pro Glu Phe Gly Ala Glu Pro Asp Val Ser Trp Val His Ser
    610                 615                 620
Leu Val Lys Asp Val Pro Ser Glu Arg Ser Glu Ile Phe Gly Ala Glu
625                 630                 635                 640
Lys Gln Gln Tyr Asp Leu Ser Lys Glu Met Leu Pro Pro Trp Met Glu
                645                 650                 655
Gln Leu Tyr Ile Glu Gln Glu Gln Met Val Ala
            660                 665
```

<210> 32
<211> 2683
<212> DNA
<213> Eucalyptus grandis

<400> 32

```
gcaaaggtcg atcacttcct ccctagaaag cgagtgtgga gttgaagctt gataaccaga      60
ggccgcctct cgtctcgtct cgcccgcctg cgcttgctct gctctccgcg tgccaaggga     120
gtgttcctag gtgctgaatc tttccatgtg tagcggttca aaagggaagg gagagtgaat     180
tcgagaagca gaggatgtcg gcccgtcagt tctcgatcct gctcgagtta tctgctgcgg     240
atgatctgac gaactttaag aaagcagttg aggaagacgg ctacgatatt gatgagtcga     300
gcttgtggta tggtaggagg atcgggtcga agaagattgg gcttgaagag agaactcccc     360
tcatgattgc cgcgatgttc ggcagtatgt ccgtgctgga ttatattatc aagtctggcc     420
gggccaatgt aaacaaggcg tgtggttcag atggtgctac cgcgcttcac tgtgctgcgg     480
ctggtggctc ggtacaatct cctgaggtgg tcaagctgtt gcttgattct tcagcgaatg     540
ctaactccat tgatgcgaat gggaaacgag cgggagactt gatttctgag gtctctggtt     600
cgcccttcaa ttcgagaagg aagactttgg atgtcatgtt gactggaggt gggactgttg     660
agtttgttga ggaaacttac aatctgcctg agaatctggg tagtcaaatt gaaggaaacg     720
aacaaagaga gagtccaacg gcccgcgctt ccaaggatgg ttctgaaaag aaagagtatc     780
ctgtcgacct ttctcttccg gacatcaaca atggaatata tagcacagat gagtttagga     840
tgtattcttt caaagtgaag ccttgctcga gagcttactc tcatgactgg actgagtgtc     900
catttgttca ccctggggag aatgcaagac ggcgtgaccc acggaaatat cactacagct     960
gtgtgccttg ccctgagttc cgcaaggggt catgcaggca aggggatggc tgcgagtatg    1020
ctcatggtat atttgagtgc tggcttcacc cagctcaata tcgcacccgt ctctgtaagg    1080
atgagattgg atgcaccaga aaagtctgtt tctttgccca caaacatgaa gagcttcgtc    1140
cattgtatgc atcaactggt tcggcgcttc cttctccaag atcattttcg cccgttgctg    1200
cttctctaga catgggatca ctgagccctc tctctctcgg ttcttcttca gtccggatac    1260
cgccaacttc aacaccacct atgactccat caggggcctc ttctcccctt ggtgggtcga    1320
tgtggaaaag ccaaattaat agcactccgc ctggcttgca gcttccaggt agcaggttga    1380
gaagcgcatt gagtgctaga gacatggatt tagatgttga cttgatcgat ctagaaaata    1440
attatcgttt gcagaagcag ttgctcgaac actttcctga tctgtcctct cctcgtggtt    1500
ggaacaactc ttcatccacc acgtcggctt tccctgagta ttcaggtgac atgactggag    1560
aaataagtag gttaggagta aaaccaaata atctcgagga tagtttcagg tcattggacc    1620
tgaccctctt gtctcagtta caagggctgt cacttgatgg tgcaatatcc cagctgcaat    1680
ctcctactgg aatgaagatt cggcagaaca tgacccagca gctctactca aactatactg    1740
acaagctttc ctcgtcacct agggcaatgc catcatttgg aaccgatcct tccagagctt    1800
cagcagcagc cactctgagt tccaggtcat tggcatttgc aaaaaggagc cacagcttca    1860
ttgagcggag tacagtgaac agtcagtctg gatattcagc aggtgctgct tctccaactg    1920
caaggatgtc ttcccagaat gactggggct cgcccgatgg caaactagac tggggcattc    1980
aaggggagga gctgaacaag ctgaggaaat ctgcatcatt cgggctcagg agcagcagca    2040
accgcttcca tgcgtctgca gattctgcga cagcaactgt aggggaccca gacatgccct    2100
ggattcagtc cttggcaaag gaagccccgt cacaaaaccc tggcaatttt ggagcagagc    2160
atcagcagca gcagcagcag cagcagcagc agcagtatca tcttaattct ggaggtactg    2220
agctgcttcc agcttgggtg gagcagttgt acgcggatca ggagcagatg gtcgcctgag    2280
atcaacattg gcttcttatc taaccactat tagtcatttc gttattgctt taattttttt    2340
tcttctgagt ctagtattaa tgtctaggat tcgaacgaac tggaaaatta aatctagagg    2400
gaagatggga agaaaagagc aggatggaag gtttctgctc ggtccgagat ttctcatagt    2460
ctattataga ctatcgtatt tctcgttctt ttccgtccca atgttcttga tttggttctc    2520
agcatgtttt ctggatgagg cttacaaact atgtaatctt gtcttgctaa aagaatcaga    2580
gctgcacctg caccaaaggt tgtgatacta ccgcttattg atgatgatga taataataat    2640
aattcggaca tttagtacca agtccgatgt ctcaaaaaaa aaa                      2683
```

<210> 33
<211> 694
<212> PRT
<213> Eucalyptus grandis

<400> 33

```
Met Ser Ala Arg Gln Phe Ser Ile Leu Leu Glu Leu Ser Ala Ala Asp
1               5                   10                  15
Asp Leu Thr Asn Phe Lys Lys Ala Val Glu Glu Asp Gly Tyr Asp Ile
            20                  25                  30
Asp Glu Ser Ser Leu Trp Tyr Gly Arg Arg Ile Gly Ser Lys Lys Ile
        35                  40                  45
Gly Leu Glu Glu Arg Thr Pro Leu Met Ile Ala Ala Met Phe Gly Ser
    50                  55                  60
Met Ser Val Leu Asp Tyr Ile Ile Lys Ser Gly Arg Ala Asn Val Asn
65                  70                  75                  80
```

```
Lys Ala Cys Gly Ser Asp Gly Ala Thr Ala Leu His Cys Ala Ala Ala
                85                  90                  95
Gly Gly Ser Val Gln Ser Pro Glu Val Val Lys Leu Leu Leu Asp Ser
            100                 105                 110
Ser Ala Asn Ala Asn Ser Ile Asp Ala Asn Gly Lys Arg Ala Gly Asp
        115                 120                 125
Leu Ile Ser Glu Val Ser Gly Ser Pro Phe Asn Ser Arg Arg Lys Thr
    130                 135                 140
Leu Asp Val Met Leu Thr Gly Gly Gly Thr Val Glu Phe Val Glu Glu
145                 150                 155                 160
Thr Tyr Asn Leu Pro Glu Asn Leu Gly Ser Gln Ile Glu Gly Asn Glu
                165                 170                 175
Gln Arg Glu Ser Pro Thr Ala Arg Ala Ser Lys Asp Gly Ser Glu Lys
            180                 185                 190
Lys Glu Tyr Pro Val Asp Leu Ser Leu Pro Asp Ile Asn Asn Gly Ile
        195                 200                 205
Tyr Ser Thr Asp Glu Phe Arg Met Tyr Ser Phe Lys Val Lys Pro Cys
    210                 215                 220
Ser Arg Ala Tyr Ser His Asp Trp Thr Glu Cys Pro Phe Val His Pro
225                 230                 235                 240
Gly Glu Asn Ala Arg Arg Arg Asp Pro Arg Lys Tyr His Tyr Ser Cys
                245                 250                 255
Val Pro Cys Pro Glu Phe Arg Lys Gly Ser Cys Arg Gln Gly Asp Gly
            260                 265                 270
Cys Glu Tyr Ala His Gly Ile Phe Glu Cys Trp Leu His Pro Ala Gln
        275                 280                 285
Tyr Arg Thr Arg Leu Cys Lys Asp Glu Ile Gly Cys Thr Arg Lys Val
    290                 295                 300
Cys Phe Phe Ala His Lys His Glu Glu Leu Arg Pro Leu Tyr Ala Ser
305                 310                 315                 320
Thr Gly Ser Ala Leu Pro Ser Pro Arg Ser Phe Ser Pro Val Ala Ala
                325                 330                 335
Ser Leu Asp Met Gly Ser Leu Ser Pro Leu Ser Leu Gly Ser Ser Ser
            340                 345                 350
Val Arg Ile Pro Pro Thr Ser Thr Pro Pro Met Thr Pro Ser Gly Ala
        355                 360                 365
Ser Ser Pro Leu Gly Gly Ser Met Trp Lys Ser Gln Ile Asn Ser Thr
    370                 375                 380
Pro Pro Gly Leu Gln Leu Pro Gly Ser Arg Leu Arg Ser Ala Leu Ser
385                 390                 395                 400
Ala Arg Asp Met Asp Leu Asp Val Asp Leu Ile Asp Leu Glu Asn Asn
                405                 410                 415
Tyr Arg Leu Gln Lys Gln Leu Leu Glu His Phe Pro Asp Leu Ser Ser
            420                 425                 430
Pro Arg Gly Trp Asn Asn Ser Ser Ser Thr Thr Ser Ala Phe Pro Glu
        435                 440                 445
Tyr Ser Gly Asp Met Thr Gly Glu Ile Ser Arg Leu Gly Val Lys Pro
    450                 455                 460
Asn Asn Leu Glu Asp Ser Phe Arg Ser Leu Asp Leu Thr Leu Leu Ser
465                 470                 475                 480
Gln Leu Gln Gly Leu Ser Leu Asp Gly Ala Ile Ser Gln Leu Gln Ser
                485                 490                 495
Pro Thr Gly Met Lys Ile Arg Gln Asn Met Thr Gln Gln Leu Tyr Ser
            500                 505                 510
Asn Tyr Thr Asp Lys Leu Ser Ser Ser Pro Arg Ala Met Pro Ser Phe
        515                 520                 525
Gly Thr Asp Pro Ser Arg Ala Ser Ala Ala Ala Thr Leu Ser Ser Arg
    530                 535                 540
Ser Leu Ala Phe Ala Lys Arg Ser His Ser Phe Ile Glu Arg Ser Thr
```

```
545                 550                 555                 560
Val Asn Ser Gln Ser Gly Tyr Ser Ala Gly Ala Ala Ser Pro Thr Ala
                565                 570                 575
Arg Met Ser Ser Gln Asn Asp Trp Gly Ser Pro Asp Gly Lys Leu Asp
            580                 585                 590
Trp Gly Ile Gln Gly Glu Glu Leu Asn Lys Leu Arg Lys Ser Ala Ser
        595                 600                 605
Phe Gly Leu Arg Ser Ser Ser Asn Arg Phe His Ala Ser Ala Asp Ser
    610                 615                 620
Ala Thr Ala Thr Val Gly Asp Pro Asp Met Pro Trp Ile Gln Ser Leu
625                 630                 635                 640
Ala Lys Glu Ala Pro Ser Gln Asn Pro Gly Asn Phe Gly Ala Glu His
                645                 650                 655
Gln Gln Gln Gln Gln Gln Gln Gln Gln Gln Gln Tyr His Leu Asn Ser
            660                 665                 670
Gly Gly Thr Glu Leu Leu Pro Ala Trp Val Glu Gln Leu Tyr Ala Asp
        675                 680                 685
Gln Glu Gln Met Val Ala
    690
```

<210> 34
<211> 2499
<212> DNA
<213> Arabidopsis thaliana

<400> 34

```
atttgacct taagaagaaa gtgacaagga gaggaagaag aagaaaaaaa acataatttg     60
aggaagaaga aaaaaaattc ggatttgttt tttcaataaa ttgactaatt gagtactcgt    120
ttaaaggaag tgaagagcgg ttttttggta gtggtggtcg agaaaagaga gagtttgtct    180
ctgtgactca gagtgaaatc aatagagcgg gaaaagattg ttgctttttt ttgccatggg    240
agttgatgag ctgtctcacc tcaaattctc tcttctgcta gaatcatcag cctgcaatga    300
tttgtccggt tttaagtctc tagttgaaga agaaggtctt gagagcattg atggctctgg    360
tttgtggtat gggaggagat taggatcaaa gaagatgggt tttgaggaga ggacgcctct    420
tatgattgct gccttgtttg gaagcaaaga ggttgttgat tacatcatta gtactggtct    480
tgttgacgtg aaccgctctt gtggctctga tggtgccacg gctcttcact gtgcggtctc    540
tggcttgtct gccaatagcc ttgagattgt tactcttctg ctgaagggct ctgcgaatcc    600
ggattcttgt gatgcttatg gtaacaagcc tggagatgtg attttccctt gtttgagtcc    660
ggtttttagc gcgaggatga aggttttgga gcgtttgttg aaaggaaatg atgatttgaa    720
tgaagttaat gggcaagaag aaagcgagcc agaggttgag gttgaggttg aggtttcgcc    780
tcctcggggg tctgagagga aggagtatcc ggttgatcca acgcttcctg atatcaagaa    840
cggtgtatat gggacggatg agttccggat gtatgctttc aagatcaagc cgtgctctag    900
agcatactct cacgactgga cggaatgtcc ctttgttcat ccgggtgaga acgcaaggag    960
gcgtgatccg aggaagtacc attatagttg tgtcccttgt cctgaattcc ggaaggggtc   1020
ttgttccaga ggtgatactt gcgagtatgc tcatggtatc tttgagtgct ggcttcaccc   1080
ggctcagtac cggactcgtc tctgcaagga cgagacgaat tgctcgagaa gagtttgttt   1140
ctttgcccac aaacccgagg agctgcgtcc tttgtaccct tcaactggat caggtgttcc   1200
gtccccgcgg tcttccttct catcttgcaa ttcctcgacc gctttcgaca tgggaccgat   1260
tagtccgctt cctatcggag caacaaccac acctcctttg agtcctaacg gtgtatcctc   1320
tccaataggt ggaggaaaaa cgtggatgaa ctggcctaac ataacccctc ctgcattgca   1380
gcttccaggg agcagattga aatctgcatt gaatgcaaga gaaatcgatt tctctgaaga   1440
gatgcaaagt cttacttctc caactacatg gaacaacacg ccaatgtcat ctccattctc   1500
cggaaagggc atgaacaggc ttgcaggagg agcaatgagc ccggtgaata gtctcagtga   1560
tatgtttggg acagaggata atacatcggg tttgcagatc cgacgcagcg tcattaaccc   1620
gcagctgcat tccaacagtc tttcttcatc acctgtggga gccaattctc tgttttcgat   1680
ggattcctcc gcagtcttgg cttcaagagc ggctgaattt gctaaacagc gaagccaaag   1740
cttcatagaa cgcaacaacg gactgaatca ccatcccgca atctcttcca tgactacaac   1800
ttgtttaaac gattggggct cattggatgg gaagcttgac tggagcgtcc aaggagacga   1860
gctacagaag ctcagaaaat ccacttcttt ccgtctcaga gccggtggca tggaatcaag   1920
actgcctaac gaagggactg ggctcgaaga gccagatgtc tcatgggtgg agccgctggt   1980

gaaagagcca caggagacaa gactagctcc ggtttggatg gagcaatcat acatggagac   2040
agaacagacc gtggcttgaa tcaaaagttt tgaactttca ttaaccgttc cacaagaagc   2100
aaagtcagaa agattccgag aggtcgatgc taatctattt cattttattt gtttaatgct   2160
ttgttatttt tctttagaat aaaaagaaaa aattcttagg ggacaaaaga gagttcgttt   2220
gtctctctct ctgtctccaa agaaaaacag aggtgaaaaa aggtttcaaa acctaagaaa   2280
ccttgaatta cctcacctca cttccttgat tctttactat tcacaatgag taatcgattt   2340
tttttttct tggtaacact ctcacgctga atatatatgt tttttagtaa taatataatt    2400
ggaatacaga aatgtattta cacttgtgaa gttagggaaa gtgttgtaat tgtttcttct   2460
aagagttgat ctaagatgtt tgagactata tcttcgctt                          2499
```

<210> 35
<211> 607
<212> PRT
<213> Arabidopsis thaliana

<400> 35

```
Met Gly Val Asp Glu Leu Ser His Leu Lys Phe Ser Leu Leu Leu Glu
1               5                   10                  15
Ser Ser Ala Cys Asn Asp Leu Ser Gly Phe Lys Ser Leu Val Glu Glu
            20                  25                  30
Glu Gly Leu Glu Ser Ile Asp Gly Ser Gly Leu Trp Tyr Gly Arg Arg
        35                  40                  45
Leu Gly Ser Lys Lys Met Gly Phe Glu Glu Arg Thr Pro Leu Met Ile
    50                  55                  60
Ala Ala Leu Phe Gly Ser Lys Glu Val Val Asp Tyr Ile Ile Ser Thr
65                  70                  75                  80
Gly Leu Val Asp Val Asn Arg Ser Cys Gly Ser Asp Gly Ala Thr Ala
                85                  90                  95
Leu His Cys Ala Val Ser Gly Leu Ser Ala Asn Ser Leu Glu Ile Val
            100                 105                 110
Thr Leu Leu Leu Lys Gly Ser Ala Asn Pro Asp Ser Cys Asp Ala Tyr
        115                 120                 125
Gly Asn Lys Pro Gly Asp Val Ile Phe Pro Cys Leu Ser Pro Val Phe
    130                 135                 140
Ser Ala Arg Met Lys Val Leu Glu Arg Leu Leu Lys Gly Asn Asp Asp
145                 150                 155                 160
Leu Asn Glu Val Asn Gly Gln Glu Glu Ser Glu Pro Glu Val Glu Val
                165                 170                 175
Glu Val Glu Val Ser Pro Pro Arg Gly Ser Glu Arg Lys Glu Tyr Pro
            180                 185                 190
Val Asp Pro Thr Leu Pro Asp Ile Lys Asn Gly Val Tyr Gly Thr Asp
        195                 200                 205
Glu Phe Arg Met Tyr Ala Phe Lys Ile Lys Pro Cys Ser Arg Ala Tyr
    210                 215                 220
Ser His Asp Trp Thr Glu Cys Pro Phe Val His Pro Gly Glu Asn Ala
225                 230                 235                 240
Arg Arg Arg Asp Pro Arg Lys Tyr His Tyr Ser Cys Val Pro Cys Pro
                245                 250                 255
Glu Phe Arg Lys Gly Ser Cys Ser Arg Gly Asp Thr Cys Glu Tyr Ala
            260                 265                 270
His Gly Ile Phe Glu Cys Trp Leu His Pro Ala Gln Tyr Arg Thr Arg
        275                 280                 285
Leu Cys Lys Asp Glu Thr Asn Cys Ser Arg Arg Val Cys Phe Phe Ala
    290                 295                 300
His Lys Pro Glu Glu Leu Arg Pro Leu Tyr Pro Ser Thr Gly Ser Gly
305                 310                 315                 320
Val Pro Ser Pro Arg Ser Ser Phe Ser Ser Cys Asn Ser Ser Thr Ala
                325                 330                 335
Phe Asp Met Gly Pro Ile Ser Pro Leu Pro Ile Gly Ala Thr Thr Thr
```

```
                340                 345                 350
Pro Pro Leu Ser Pro Asn Gly Val Ser Ser Pro Ile Gly Gly Gly Lys
        355                 360                 365
Thr Trp Met Asn Trp Pro Asn Ile Thr Pro Pro Ala Leu Gln Leu Pro
    370                 375                 380
Gly Ser Arg Leu Lys Ser Ala Leu Asn Ala Arg Glu Ile Asp Phe Ser
385                 390                 395                 400
Glu Glu Met Gln Ser Leu Thr Ser Pro Thr Thr Trp Asn Asn Thr Pro
                405                 410                 415
Met Ser Ser Pro Phe Ser Gly Lys Gly Met Asn Arg Leu Ala Gly Gly
            420                 425                 430
Ala Met Ser Pro Val Asn Ser Leu Ser Asp Met Phe Gly Thr Glu Asp
        435                 440                 445
Asn Thr Ser Gly Leu Gln Ile Arg Arg Ser Val Ile Asn Pro Gln Leu
    450                 455                 460
His Ser Asn Ser Leu Ser Ser Ser Pro Val Gly Ala Asn Ser Leu Phe
465                 470                 475                 480
Ser Met Asp Ser Ser Ala Val Leu Ala Ser Arg Ala Ala Glu Phe Ala
                485                 490                 495
Lys Gln Arg Ser Gln Ser Phe Ile Glu Arg Asn Asn Gly Leu Asn His
            500                 505                 510
His Pro Ala Ile Ser Ser Met Thr Thr Thr Cys Leu Asn Asp Trp Gly
        515                 520                 525
Ser Leu Asp Gly Lys Leu Asp Trp Ser Val Gln Gly Asp Glu Leu Gln
    530                 535                 540
Lys Leu Arg Lys Ser Thr Ser Phe Arg Leu Arg Ala Gly Gly Met Glu
545                 550                 555                 560
Ser Arg Leu Pro Asn Glu Gly Thr Gly Leu Glu Glu Pro Asp Val Ser
                565                 570                 575
Trp Val Glu Pro Leu Val Lys Glu Pro Gln Glu Thr Arg Leu Ala Pro
            580                 585                 590
Val Trp Met Glu Gln Ser Tyr Met Glu Thr Glu Gln Thr Val Ala
        595                 600                 605
```

<210> 36
<211> 1806
<212> DNA
<213> Oryza sativa

<400> 36

```
atgtgctctg ggccgcgcaa gccgtccaca ccgccgctgc cgcagcagca gaaggaggcg     60
acggtgatgg cggcgtcctt gcttcttgag ctggcggcag cggacgacgt ggcggcggtg    120
aggagggtcg tggaggagga gaaggtgtct cttggcgtgg ctgggttgtg gtatgggcct    180
tcggcgagcg gcgtggcgag gctcgggatg gagcggagga cggcggcgat ggtggcggcg    240
ctgtacggga gcacgggggt gcttgggtat gtcgtggcgg cagcgccggc ggaggccgcg    300
cgcgcgtcgg agacggatgg ggccacgccg ctgcacatgg cggctgccgg tggcgcggcg    360
aacgcggtcg cggccacgcg cctgttgctc gccgcggggg cgtcggtcga cgcgctctcg    420
gcttcggggc tccgcgccgg tgacctcctc ccgcgcgcca ccgcggcgga gaaggccatc    480
cggctgctgc tcaagtcgcc ggccgtgtcg ccgtcgtcgt cgccgaagaa gtcggcctcg    540
ccgccgtcgc cgccgccgcc gcaggaggcg aagaaggagt acccgcctga cctgacgctg    600
cccgacctca agagcggact gttcagcacc gacgagttcc gcatgtacag cttcaaggtg    660
aagccgtgct cccgcgccta ctcccatgac tggaccgagt gccccttcgt ccaccccggc    720
gagaacgcgc gccgccgcga ccctcgccgc tactcctaca gctgcgtgcc ttgcccggag    780
ttccgcaagg gcggctcgtg ccgcaagggc gacgcgtgcg agtacgccca tggcgtgttc    840
gagtgctggc tccacccggc gcagtacagg acgcgcctct gcaaggacga ggtcggctgc    900
gcgcgccgca tctgcttctt cgcccacaag cccgacgagc tccgcgccgt caacccctcc    960
gccgtgtccg tcggcatgca gcccaccgta tcgtcgccgc gctcctcgcc gcccaacggg   1020
ctcgacatgg cggcggcggc ggcggcgatg atgagccccg cctggccgtc gtccccagcg   1080
agccgcctca agacggcgct cggcgcgcgg gagctcgact tcgacctcga gatgctcgcg   1140

ctggaccagt accagcagaa gctgttcgac aaggtgtccg gcgcgccgtc gccgagggcg   1200
agctgggggcg ccgcggcgaa cggcctcgcc accgcgtcgc cggcgagggc cgtgccggac   1260
tacaccgacc tgctcggctc cgtcgacccg gccatgctgt cccagctcca cgcgctgtcc   1320
ctcaagcagg ccggcgacat gcccgcgtac agctccatgg cggacaccac gcagatgcac   1380
atgccgacct cgccgatggt gggcggcgcg aacaccgcgt tcgggctgga ccactccatg   1440
gcgaaggcga tcatgagctc ccgcgcctcg gcgttcgcca agcgcagcca gagcttcatc   1500
gaccgcggag gccgcgcccc ggcggcgcgt tcgctcatgt cgccggcgac gaccggcgcg   1560
ccgtccattc tctcggactg ggctcgccg gacggcaagc tggactgggg cgtccagggc   1620
gacgagctgc acaagctccg caagtcggcg tcgttcgcgt tccgcggcca atccgccatg   1680
ccggtggcga cgcacgccgc ggcggcggag ccggacgtgt catgggtgaa ctctcttgtc   1740
aaggacggcc acgccgccgg cgacatattc gcgcagtggc cggagcagga gcagatggtg   1800
gcatga                                                              1806
```

<210> 37

<211> 601

<212> PRT

<213> Oryza sativa

<400> 37

Met Cys Ser Gly Pro Arg Lys Pro Ser Thr Pro Pro Leu Pro Gln Gln
1 5 10 15
Gln Lys Glu Ala Thr Val Met Ala Ala Ser Leu Leu Leu Glu Leu Ala
20 25 30
Ala Ala Asp Asp Val Ala Ala Val Arg Arg Val Val Glu Glu Glu Lys
35 40 45
Val Ser Leu Gly Val Ala Gly Leu Trp Tyr Gly Pro Ser Ala Ser Gly
50 55 60
Val Ala Arg Leu Gly Met Glu Arg Arg Thr Ala Ala Met Val Ala Ala
65 70 75 80
Leu Tyr Gly Ser Thr Gly Val Leu Gly Tyr Val Val Ala Ala Ala Pro
85 90 95
Ala Glu Ala Ala Arg Ala Ser Glu Thr Asp Gly Ala Thr Pro Leu His
100 105 110
Met Ala Ala Ala Gly Gly Ala Ala Asn Ala Val Ala Ala Thr Arg Leu
115 120 125
Leu Leu Ala Ala Gly Ala Ser Val Asp Ala Leu Ser Ala Ser Gly Leu
130 135 140
Arg Ala Gly Asp Leu Leu Pro Arg Ala Thr Ala Ala Glu Lys Ala Ile
145 150 155 160
Arg Leu Leu Leu Lys Ser Pro Ala Val Ser Pro Ser Ser Ser Pro Lys
165 170 175
Lys Ser Ala Ser Pro Pro Ser Pro Pro Pro Pro Gln Glu Ala Lys Lys
180 185 190
Glu Tyr Pro Pro Asp Leu Thr Leu Pro Asp Leu Lys Ser Gly Leu Phe
195 200 205
Ser Thr Asp Glu Phe Arg Met Tyr Ser Phe Lys Val Lys Pro Cys Ser
210 215 220
Arg Ala Tyr Ser His Asp Trp Thr Glu Cys Pro Phe Val His Pro Gly
225 230 235 240
Glu Asn Ala Arg Arg Arg Asp Pro Arg Arg Tyr Ser Tyr Ser Cys Val
245 250 255
Pro Cys Pro Glu Phe Arg Lys Gly Gly Ser Cys Arg Lys Gly Asp Ala
260 265 270
Cys Glu Tyr Ala His Gly Val Phe Glu Cys Trp Leu His Pro Ala Gln
275 280 285
Tyr Arg Thr Arg Leu Cys Lys Asp Glu Val Gly Cys Ala Arg Arg Ile
290 295 300
Cys Phe Phe Ala His Lys Pro Asp Glu Leu Arg Ala Val Asn Pro Ser
305 310 315 320

```
Ala Val Ser Val Gly Met Gln Pro Thr Val Ser Ser Pro Arg Ser Ser
                325                 330                 335
Pro Pro Asn Gly Leu Asp Met Ala Ala Ala Ala Ala Ala Met Met Ser
            340                 345                 350
Pro Ala Trp Pro Ser Ser Pro Ala Ser Arg Leu Lys Thr Ala Leu Gly
        355                 360                 365
Ala Arg Glu Leu Asp Phe Asp Leu Glu Met Leu Ala Leu Asp Gln Tyr
    370                 375                 380
Gln Gln Lys Leu Phe Asp Lys Val Ser Gly Ala Pro Ser Pro Arg Ala
385                 390                 395                 400
Ser Trp Gly Ala Ala Ala Asn Gly Leu Ala Thr Ala Ser Pro Ala Arg
                405                 410                 415
Ala Val Pro Asp Tyr Thr Asp Leu Leu Gly Ser Val Asp Pro Ala Met
            420                 425                 430
Leu Ser Gln Leu His Ala Leu Ser Leu Lys Gln Ala Gly Asp Met Pro
        435                 440                 445
Ala Tyr Ser Ser Met Ala Asp Thr Thr Gln Met His Met Pro Thr Ser
    450                 455                 460
Pro Met Val Gly Gly Ala Asn Thr Ala Phe Gly Leu Asp His Ser Met
465                 470                 475                 480
Ala Lys Ala Ile Met Ser Ser Arg Ala Ser Ala Phe Ala Lys Arg Ser
                485                 490                 495
Gln Ser Phe Ile Asp Arg Gly Gly Arg Ala Pro Ala Ala Arg Ser Leu
            500                 505                 510
Met Ser Pro Ala Thr Thr Gly Ala Pro Ser Ile Leu Ser Asp Trp Gly
        515                 520                 525
Ser Pro Asp Gly Lys Leu Asp Trp Gly Val Gln Gly Asp Glu Leu His
    530                 535                 540
Lys Leu Arg Lys Ser Ala Ser Phe Ala Phe Arg Gly Gln Ser Ala Met
545                 550                 555                 560
Pro Val Ala Thr His Ala Ala Ala Ala Glu Pro Asp Val Ser Trp Val
                565                 570                 575
Asn Ser Leu Val Lys Asp Gly His Ala Ala Gly Asp Ile Phe Ala Gln
            580                 585                 590
Trp Pro Glu Gln Glu Gln Met Val Ala
        595                 600
```

<210> 38
<211> 1692
<212> DNA
<213> Hordeum vulgare

<400> 38

```
cggcacgagg cacatccatc atctaacctc acctctcctc tcctcccctc tcctcctacc     60
aaacccaaaa ccaagcagag caagagcaag agcaagagca agagcaagca agcatgtgcc    120
ctggcctgcg caacctcgcc gccgccatgc caccctccgc ccacgaccac ccctcctcct    180
acctgctcga gctcgccgcc gacgacgacc tccccgcctt ccgccgcgcc gtccaggagg    240
acaacctctc cctcgacgcc gcatccccga ggtacgagcc atcccccaaa tcagaccaac    300
aacaacaaca cgccccagct cgcgctccac ctgcgcaccc ccgccatggt cgccgcgctc    360
tacggcagca ccaccgtcct ctcctacgtc ctctccatcg ccccctccga ggccgcccgc    420
gcctccgcat ccgacggcgc caccccgctc ctcctcgccc accagggccg cgcgccatcc    480
gcgccccacg ccgcacgcct cctcctcacc gacggcgcat catcgtcctc cctactcgcg    540
ccccaagctc accctctcaa ccaccaaaac caaaaccaaa acagccccac caagaaagac    600
tcgccgccgg actccaggag gaccaccacc aagaaggact actcctccgc ctccgactcc    660
cagacggagg acatcaacgc gggcgtcttc gccaccgacg acttccggat gtacagcttc    720
aaggtgaacc cgtgctcccg cgcctacacg cacgactgga ccgagtgccc cttcgcccac    780
cccggcgaga acgcgcgccg ccgcgacccg cgccgcgtgc catactcgtg cgtcccatgc    840
ccggacttcc gccgcgaccc ggccgcatgc cgcaagggcg acgcctgcga gtacgcgcac    900
ggcgtcttcg agtcatggct ccaccccgcg cagtaccgca ccaggctctg caaggacgag    960

gtcggatgcc cgcgccgcat ctgcttcttc gcgcacggcg cccgacagct acgcgccgtc   1020
aacccctccg ccgcatccat ggactcgcca tccccaactt cctcttcgcc gccgcgaacc   1080
tccaggccgg ccgcgctcac cgcgtcgctc agctcgcggg acctcgactt ggacgccgac   1140
aaccaggccc agtacgcgcg caggatgatg atggccaggg ccaactcccc gccggactac   1200
tcgcccgacc tcgtcgccgc ctacgtacag gcgctctcct ccctgcaaca gcagcagcat   1260
cagcagaacc agcaacagca gcatcagcag cagaaccagc accagcagca acatcagcag   1320
aaccagcacc agcagcatca gcagcaacat cagcagagca tggggatggg ggggctgagc   1380
gcccgcgccg ccgccttcac caaccgcagc cagaccttcg tgcaccgctc tccgtccccg   1440
gctccggcgc ggtcgttcaa gtctccggcg ccgtcgtcca tgctcgcgga ctgggggtcg   1500
ccggacggga agctggactg gggcgtgcag gccgcggagc tgcgcaagtc cacgtctttc   1560
ggagtcagaa gcagcagcag gccgcatcat gagacgacga gggcggagga caacatgtac   1620
ccgtcgtgga tgaaggacgg cagcgatatg ctgctggcgg cgcggtggtc ggacctggag   1680
cagatggtcg cc                                                       1692
```

<210> 39
<211> 564
<212> PRT
<213> Hordeum vulgare

<400> 39

```
Arg His Glu Ala His Pro Ser Ser Asn Leu Thr Ser Pro Leu Leu Pro
1               5                   10                  15
Ser Pro Pro Thr Lys Pro Lys Thr Lys Gln Ser Lys Ser Lys Ser Lys
            20                  25                  30
Ser Lys Ser Lys Gln Ala Cys Ala Leu Ala Cys Ala Thr Ser Pro Pro
        35                  40                  45
Pro Cys His Pro Pro Pro Thr Thr Thr Pro Pro Pro Thr Cys Ser Ser
    50                  55                  60
Ser Pro Pro Thr Thr Thr Ser Pro Pro Ser Ala Ala Pro Ser Arg Arg
65                  70                  75                  80
Thr Thr Ser Pro Ser Thr Pro His Pro Arg Gly Thr Ser His Pro Pro
                85                  90                  95
Asn Gln Thr Asn Asn Asn Asn Thr Pro Gln Leu Ala Leu His Leu Arg
            100                 105                 110
Thr Pro Ala Met Val Ala Ala Leu Tyr Gly Ser Thr Thr Val Leu Ser
        115                 120                 125
Tyr Val Leu Ser Ile Ala Pro Ser Glu Ala Ala Arg Ala Ser Ala Ser
    130                 135                 140
Asp Gly Ala Thr Pro Leu Leu Leu Ala His Gln Gly Arg Ala Pro Ser
145                 150                 155                 160
Ala Pro His Ala Ala Arg Leu Leu Leu Thr Asp Gly Ala Ser Ser Ser
                165                 170                 175
Ser Leu Leu Ala Pro Gln Ala His Pro Leu Asn His Gln Asn Gln Asn
            180                 185                 190
Gln Asn Ser Pro Thr Lys Lys Asp Ser Pro Pro Asp Ser Arg Arg Thr
        195                 200                 205
Thr Thr Lys Lys Asp Tyr Ser Ser Ala Ser Asp Ser Gln Thr Glu Asp
    210                 215                 220
Ile Asn Ala Gly Val Phe Ala Thr Asp Asp Phe Arg Met Tyr Ser Phe
225                 230                 235                 240
Lys Val Asn Pro Cys Ser Arg Ala Tyr Thr His Asp Trp Thr Glu Cys
                245                 250                 255
Pro Phe Ala His Pro Gly Glu Asn Ala Arg Arg Arg Asp Pro Arg Arg
            260                 265                 270
Val Pro Tyr Ser Cys Val Pro Cys Pro Asp Phe Arg Arg Asp Pro Ala
        275                 280                 285
Ala Cys Arg Lys Gly Asp Ala Cys Glu Tyr Ala His Gly Val Phe Glu
    290                 295                 300
Ser Trp Leu His Pro Ala Gln Tyr Arg Thr Arg Leu Cys Lys Asp Glu
```

```
305                 310                 315                 320
Val Gly Cys Pro Arg Arg Ile Cys Phe Phe Ala His Gly Ala Arg Gln
                325                 330                 335
Leu Arg Ala Val Asn Pro Ser Ala Ala Ser Met Asp Ser Pro Ser Pro
            340                 345                 350
Thr Ser Ser Ser Pro Pro Arg Thr Ser Arg Pro Ala Ala Leu Thr Ala
        355                 360                 365
Ser Leu Ser Ser Arg Asp Leu Asp Leu Asp Ala Asp Asn Gln Ala Gln
    370                 375                 380
Tyr Ala Arg Arg Met Met Met Ala Arg Ala Asn Ser Pro Pro Asp Tyr
385                 390                 395                 400
Ser Pro Asp Leu Val Ala Ala Tyr Val Gln Ala Leu Ser Ser Leu Gln
                405                 410                 415
Gln Gln Gln His Gln Gln Asn Gln Gln Gln Gln His Gln Gln Gln Asn
            420                 425                 430
Gln His Gln Gln Gln His Gln Gln Asn Gln His Gln Gln His Gln Gln
        435                 440                 445
Gln His Gln Gln Ser Met Gly Met Gly Gly Leu Ser Ala Arg Ala Ala
    450                 455                 460
Ala Phe Thr Asn Arg Ser Gln Thr Phe Val His Arg Ser Pro Ser Pro
465                 470                 475                 480
Ala Pro Ala Arg Ser Phe Lys Ser Pro Ala Pro Ser Ser Met Leu Ala
                485                 490                 495
Asp Trp Gly Ser Pro Asp Gly Lys Leu Asp Trp Gly Val Gln Ala Ala
            500                 505                 510
Glu Leu Arg Lys Ser Thr Ser Phe Gly Val Arg Ser Ser Ser Arg Pro
        515                 520                 525
His His Glu Thr Thr Arg Ala Glu Asp Asn Met Tyr Pro Ser Trp Met
    530                 535                 540
Lys Asp Gly Ser Asp Met Leu Leu Ala Ala Arg Trp Ser Asp Leu Glu
545                 550                 555                 560
Gln Met Val Ala
```

<210> 40
<211> 3610
<212> DNA
<213> Pinus radiata

<400> 40

```
tgtttccagg cgggcactaa agcaagggag ggggtaggct ttactttctg ctctgcgcaa     60
agaacgttga aatcaatcgc cctggctggt ctggcgtgac tactagattc aatttcttca    120
tggccgtctt cacataccca ttctttaccg gttcagagct gtgatcttta tttttaacag    180
ccacaatcat ggtttgtgtt tccagtgtta tgatctgagt gaagttcgtt ctttttctcg    240
tgacccaggc ttgatactag gccggacctt tctgaggtgg aagagatcta tacatttgag    300
gcctattttg tgtagccatg tgtggaggcc cagaacattt gaagcctgcc agcccacacg    360
aaggagaaga taaagtcaaa atggccgaga atcagtctat caaagtgaag gaattgtctg    420
aatcttgttc aagtctacat gaactagctg ctaataatga ccttattggc tttaagaaag    480
caatggagga agaagggtca aagatagatg aggttaactt ttggtacggg aggcagaatg    540
gttctaatca gatggtcctg gagcaaagga ctccattgat ggttgctgca ctttatggca    600
gtgtagatgc gctgagttac atcttatcca tttatgtaac ttgtggagca gatgttaacc    660
aagcctgtgg gtcagataac tccactgcct tgcattgtgc ggctgtggga gggtctgcct    720
gtgcagttga aactgtaaaa ttgttacttc atgcaggcag tgatgtgaat cgcttggatg    780
cttatggcag aagaccagca gatgtgatta tggtttctcc taagctaacc gaaatcaagg    840
ccaagctaga agaaatgtta aacgcagctg gttcatgtca aacttctccg gcaaagttgc    900
ctaacatagt ttcagggcca cctgggtttg agtcaaaggg gatggagtcc atgtccccat    960
tgccattgtt gcctctttca ttgtctttag aagcatccaa taatagatca ggttgtgtga   1020
attctccaac atcttcgcca aagtccatgg aagcattaaa gggtttcggt gatgttaatg   1080
agaagaagga atatcctgtg gacccttctt ttccagacat aaagaatagc atctatacta   1140
```

```
cagatgaatt tcggatgttt tccttcaagg tgcggccatg ttcacgggca tattctcatg 1200
attggactga atgcccattt gtgcatcctg gtgaaaatgc cagaaggcgg gatccaagaa 1260
ggtatcatta tagctgtgtt ccttgcccag attttcggaa agggacttgt aggcgcagtg 1320
atgtttgtga atatgcacac ggtgtttttg agtgctggtt acatcctgct caatatagga 1380
cacggttgtg caaagatggg actaattgtt cacgtagagt ttgcttcttt gctcacacat 1440
ctgaggaact acgccctctc attgtctcta ctgggtctgc tgttccatcc ccaagggcat 1500
catcatctct ggacatgaca tctgtcatga gtcctcttgc cctggttct ccctcttcag 1560
tttcaatgat gtcacccttc ctatcaaatc ctcagcaagg cagtgtgctt actccgccta 1620
tgtctccatc agcgtcctct gtaaatggat atggaggctg gccacagcct aatgtaccaa 1680
ccttacacct tcctggtagc aatgttcaaa ccagccgtct tagagcggaa cttaatgcca 1740
gagacatgcc tgttgaggat tctcctcgaa tttcagacta tgaagggcag caactcctga 1800
atgatttttc tccactgtcc acacaagcca ggctgaatgc tgctgctgct gttatatctg 1860
gtggcgggaa caccacaaca aggtctggaa aatacaagag tcacgggatc aatactgttg 1920
ctccaacgaa tcttgaagac ttgtttgcct ccgaggtaac atctcctaga gtagcagttc 1980
ttgaaccttc catcttttct cagatgagtc cccaaatgca agctcataag actgcccagg 2040
catatatgca gattcaaaac cagatgctgc ctcctataaa tacacaggca ttttcgcagg 2100
gaattacaca gatgcagcag gctgcaatag agcctcagag ccctggacat tctttgatgc 2160
aatcaccttt ccaatcttcc tcgtatgggt tgggatcccc tggtagaatg tcacctcgtt 2220
gtgtggatgt ggaacgtcat aatacatgtg ggtctccctt atcaccggct atggctgcaa 2280
cgataaattc aagaatggct atggctgctt ttgttcagag ggaaaaacgg agccatagtt 2340
cccgtgactt gggagctaat gtgaatccca gttcatggtc tgattggggc tcgcctacag 2400
gtaaagttga ctggggggtt caaggagaag agttgagcaa attaagaaag tcggcttcat 2460
ttggtccccg cagttatgaa gaaccggatt tgtcttgggt tcaaacactg gtaaaggaaa 2520
ctacaccaga gggtaaagat ggaggaaatg taagctgttc tggggaaact ccacacaagg 2580
ggcaaataga aaatgttgat cattcagttt tgggtgcctg gattgaacag atgcagcttg 2640
atcagattgt agcttgagat taggattatt tatttggagt ggtggtaggg ataggctcat 2700
ttaaaattca atttctcatt ttttactatt tcttttataa aaattcccca ttatagttta 2760
ggaaatagtc tggttttcta cctattatca gaattacacc tgcaggaaat tttggaggaa 2820
agcatgcaaa aagtagatag ggatgttatt cctatcagca ggttgacaag ctgaaaatca 2880
cttgggtggt agaccagaga atgacactat tttttgttga catggcaact gaagatgctg 2940
ttttctttac ttatcattaa caaccctata tatatttgtt ttgaaagaac tgagcggaga 3000
aatgttgtca gttggttact ctgcgcaagg ccttggaaga aatccaagat gtggcatctt 3060
ggtgcatttt taatttatca agtgtgaaat ccataacagg tttcagtgag tgacttctga 3120
ggttgtatat ggaaaaacct atgatgttgg ctgtctactg ctatttttct gtgcctaaac 3180
tgtcaactaa agtttgcagg tggcaatttt gtggcagcat atttgcacat tgaagcggat 3240
ggtctgcacc tgctatagaa gttttcgagt ctgtagaatt tgatggtgca agatgatttt 3300
ctagttgata tatttggaag gctttgccaa agtagtggca tgtacatttt gcaaaaattt 3360
aaaggatggc aatccattgt tttgccatgt agcttcactt tattgattag gtggaaagga 3420
attttgagac acttcaattt gtgcatactt ttgttctgaa ctgcaaaatc agtctcttgt 3480
gatgtcctca aggctattat gctcagggat ttgcctaaaa ccataagtgg ccttagataa 3540
ggtaccattg tattaccttt tattgtttgg atattttatt tatgaaagtg aatttatttt 3600
aaaaaaaaaa                                                        3610
```

<210> 41
<211> 779
<212> PRT
<213> Pinus radiata

<400> 41

```
Met Cys Gly Gly Pro Glu His Leu Lys Pro Ala Ser Pro His Glu Gly
1               5                   10                  15
Glu Asp Lys Val Lys Met Ala Glu Asn Gln Ser Ile Lys Val Lys Glu
            20                  25                  30
Leu Ser Glu Ser Cys Ser Ser Leu His Glu Leu Ala Ala Asn Asn Asp
        35                  40                  45
Leu Ile Gly Phe Lys Lys Ala Met Glu Glu Glu Gly Ser Lys Ile Asp
    50                  55                  60
Glu Val Asn Phe Trp Tyr Gly Arg Gln Asn Gly Ser Asn Gln Met Val
65                  70                  75                  80
```

```
Leu Glu Gln Arg Thr Pro Leu Met Val Ala Ala Leu Tyr Gly Ser Val
                85                  90                  95
Asp Ala Leu Ser Tyr Ile Leu Ser Ile Tyr Val Thr Cys Gly Ala Asp
            100                 105                 110
Val Asn Gln Ala Cys Gly Ser Asp Asn Ser Thr Ala Leu His Cys Ala
        115                 120                 125
Ala Val Gly Gly Ser Ala Cys Ala Val Glu Thr Val Lys Leu Leu Leu
    130                 135                 140
His Ala Gly Ser Asp Val Asn Arg Leu Asp Ala Tyr Gly Arg Arg Pro
145                 150                 155                 160
Ala Asp Val Ile Met Val Ser Pro Lys Leu Thr Glu Ile Lys Ala Lys
                165                 170                 175
Leu Glu Glu Met Leu Asn Ala Ala Gly Ser Cys Gln Thr Ser Pro Ala
            180                 185                 190
Lys Leu Pro Asn Ile Val Ser Gly Pro Pro Gly Phe Glu Ser Lys Gly
        195                 200                 205
Met Glu Ser Met Ser Pro Leu Pro Leu Leu Pro Leu Ser Leu Ser Leu
    210                 215                 220
Glu Ala Ser Asn Asn Arg Ser Gly Cys Val Asn Ser Pro Thr Ser Ser
225                 230                 235                 240
Pro Lys Ser Met Glu Ala Leu Lys Gly Phe Gly Asp Val Asn Glu Lys
                245                 250                 255
Lys Glu Tyr Pro Val Asp Pro Ser Phe Pro Asp Ile Lys Asn Ser Ile
            260                 265                 270
Tyr Thr Thr Asp Glu Phe Arg Met Phe Ser Phe Lys Val Arg Pro Cys
        275                 280                 285
Ser Arg Ala Tyr Ser His Asp Trp Thr Glu Cys Pro Phe Val His Pro
    290                 295                 300
Gly Glu Asn Ala Arg Arg Arg Asp Pro Arg Arg Tyr His Tyr Ser Cys
305                 310                 315                 320
Val Pro Cys Pro Asp Phe Arg Lys Gly Thr Cys Arg Arg Ser Asp Val
                325                 330                 335
Cys Glu Tyr Ala His Gly Val Phe Glu Cys Trp Leu His Pro Ala Gln
            340                 345                 350
Tyr Arg Thr Arg Leu Cys Lys Asp Gly Thr Asn Cys Ser Arg Arg Val
        355                 360                 365
Cys Phe Phe Ala His Thr Ser Glu Glu Leu Arg Pro Leu Ile Val Ser
    370                 375                 380
Thr Gly Ser Ala Val Pro Ser Pro Arg Ala Ser Ser Ser Leu Asp Met
385                 390                 395                 400
Thr Ser Val Met Ser Pro Leu Ala Pro Gly Ser Pro Ser Ser Val Ser
                405                 410                 415
Met Met Ser Pro Phe Leu Ser Asn Pro Gln Gln Gly Ser Val Leu Thr
            420                 425                 430
Pro Pro Met Ser Pro Ser Ala Ser Ser Val Asn Gly Tyr Gly Gly Trp
        435                 440                 445
Pro Gln Pro Asn Val Pro Thr Leu His Leu Pro Gly Ser Asn Val Gln
    450                 455                 460
Thr Ser Arg Leu Arg Ala Glu Leu Asn Ala Arg Asp Met Pro Val Glu
465                 470                 475                 480
Asp Ser Pro Arg Ile Ser Asp Tyr Glu Gly Gln Gln Leu Leu Asn Asp
                485                 490                 495
Phe Ser Pro Leu Ser Thr Gln Ala Arg Leu Asn Ala Ala Ala Ala Val
            500                 505                 510
Ile Ser Gly Gly Gly Asn Thr Thr Thr Arg Ser Gly Lys Tyr Lys Ser
        515                 520                 525
His Gly Ile Asn Thr Val Ala Pro Thr Asn Leu Glu Asp Leu Phe Ala
    530                 535                 540
Ser Glu Val Thr Ser Pro Arg Val Ala Val Leu Glu Pro Ser Ile Phe
```

```
545                 550                 555                 560
Ser Gln Met Ser Pro Gln Met Gln Ala His Lys Thr Ala Gln Ala Tyr
                565                 570                 575
Met Gln Ile Gln Asn Gln Met Leu Pro Pro Ile Asn Thr Gln Ala Phe
            580                 585                 590
Ser Gln Gly Ile Thr Gln Met Gln Gln Ala Ala Ile Glu Pro Gln Ser
        595                 600                 605
Pro Gly His Ser Leu Met Gln Ser Pro Phe Gln Ser Ser Ser Tyr Gly
    610                 615                 620
Leu Gly Ser Pro Gly Arg Met Ser Pro Arg Cys Val Asp Val Glu Arg
625                 630                 635                 640
His Asn Thr Cys Gly Ser Pro Leu Ser Pro Ala Met Ala Ala Thr Ile
                645                 650                 655
Asn Ser Arg Met Ala Met Ala Ala Phe Val Gln Arg Glu Lys Arg Ser
            660                 665                 670
His Ser Ser Arg Asp Leu Gly Ala Asn Val Asn Pro Ser Ser Trp Ser
        675                 680                 685
Asp Trp Gly Ser Pro Thr Gly Lys Val Asp Trp Gly Val Gln Gly Glu
    690                 695                 700
Glu Leu Ser Lys Leu Arg Lys Ser Ala Ser Phe Gly Pro Arg Ser Tyr
705                 710                 715                 720
Glu Glu Pro Asp Leu Ser Trp Val Gln Thr Leu Val Lys Glu Thr Thr
                725                 730                 735
Pro Glu Gly Lys Asp Gly Gly Asn Val Ser Cys Ser Gly Glu Thr Pro
            740                 745                 750
His Lys Gly Gln Ile Glu Asn Val Asp His Ser Val Leu Gly Ala Trp
        755                 760                 765
Ile Glu Gln Met Gln Leu Asp Gln Ile Val Ala
    770                 775
```

<210> 42
<211> 3610
<212> DNA
<213> Pinus radiata

<400> 42

```
tgtttccagg cgggcactaa agcaagggag ggggtaggct ttactttctg ctctgcgcaa      60
agaacgttga aatcaatcgc cctggctggt ctggcgtgac tactagattc aatttcttca     120
tggccgtctt cacataccca ttctttaccg gttcagagct gtgatcttta tttttaacag     180
ccacaatcat ggtttgtgtt tccagtgtta tgatctgagt gaagttcgtt ctttttctcg     240
tgacccaggc ttgatactag gccggacctt tctgaggtgg aagagatcta tacatttgag     300
gcctattttg tgtagccatg tgtggaggcc cagaacattt gaagcctgcc agcccacacg     360
aaggagaaga taaagtcaaa atggccgaga atcagtctat caaagtgaag gaattgtctg     420
aatcttgttc aagtctacat gaactagctg ctaataatga ccttattggc tttaagaaag     480
caatggagga agaagggtca aagatagatg aggttaactt ttggtacggg aggcagaatg     540
gttctaatca gatggtcctg gagcaaagga ctccattgat ggttgctgca ctttatggca     600
gtgtagatgc gctgagttac atcttatcca tttatgtaac ttgtggagca gatgttaacc     660
aagcctgtgg gtcagataac tccactgcct tgcattgtgc ggctgtggga gggtctgcct     720
gtgcagttga aactgtaaaa ttgttacttc atgcaggcag tgatgtgaat cgcttggatg     780
cttatggcag aagaccagca gatgtgatta tggtttctcc taagctaacc gaaatcaagg     840
ccaagctaga agaaatgtta aacgcagctg gttcatgtca aacttctccg gcaaagttgc     900
ctaacatagt ttcagggcca cctgggtttg agtcaaaggg gatggagtcc atgtccccat     960
tgccattgtt gcctctttca ttgtctttag aagcatccaa taatagatca ggttgtgtga    1020
attctccaac atcttcgcca aagtccatgg aagcattaaa gggtttcggt gatgttaatg    1080
agaagaagga atatcctgtg gacccttctt ttccagacat aaagaatagc atctatacta    1140
cagatgaatt tcggatgttt tccttcaagg tgcggccatg ttcacgggca tattctcatg    1200
attggactga atgcccattt gtgcatcctg gtgaaaatgc cagaaggcgg gatccaagaa    1260
ggtatcatta tagctgtgtt ccttgcccag attttcggaa aggacttgt aggcgcagtg     1320
atgtttgtga atatgcacac ggtgtttttg agtgctggtt acatcctgct caatatagga    1380
```

```
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc ccctggttct ccctcttcag   1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgatttttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
cgataaattc aagaatggct atggctgctt ttgttcagag ggaaaaacgg agccatagtt   2340
cccgtgactt gggagctaat gtgaatccca gttcatggtc tgattggggc tcgcctacag   2400
gtaaagttga ctgggggtt caaggagaag agttgagcaa attaagaaag tcggcttcat   2460
ttggtccccg cagttatgaa gaaccggatt tgtcttgggt tcaaacactg gtaaaggaaa   2520
ctacaccaga gggtaaagat ggaggaaatg taagctgttc tggggaaact ccacacaagg   2580
ggcaaataga aaatgttgat cattcagttt tgggtgcctg gattgaacag atgcagcttg   2640
atcagattgt agcttgagat taggattatt tatttggagt ggtggtaggg ataggctcat   2700
ttaaaattca atttctcatt ttttactatt tcttttataa aaattcccca ttatagttta   2760
ggaaatagtc tggtttttcta cctattatca gaattacacc tgcaggaaat tttggaggaa   2820
agcatgcaaa aagtagatag ggatgttatt cctatcagca ggttgacaag ctgaaaatca   2880
cttgggtggt agaccagaga atgacactat tttttgttga catggcaact gaagatgctg   2940
ttttctttac ttatcattaa caaccctata tatatttgtt ttgaaagaac tgagcggaga   3000
aatgttgtca gttggttact ctgcgcaagg ccttggaaga aatccaagat gtggcatctt   3060
ggtgcatttt taatttatca agtgtgaaat ccataacagg tttcagtgag tgacttctga   3120
ggttgtatat ggaaaaacct atgatgttgg ctgtctactg ctatttttct gtgcctaaac   3180
tgtcaactaa agtttgcagg tggcaatttt gtggcagcat atttgcacat tgaagcggat   3240
ggtctgcacc tgctatagaa gttttcgagt ctgtagaatt tgatggtgca agatgatttt   3300
ctagttgata tatttggaag gctttgccaa agtagtggca tgtacatttt gcaaaaattt   3360
aaaggatggc aatccattgt tttgccatgt agcttcactt tattgattag gtggaaagga   3420
attttgagac acttcaattt gtgcatactt ttgttctgaa ctgcaaaatc agtctcttgt   3480
gatgtcctca aggctattat gctcagggat ttgcctaaaa ccataagtgg ccttagataa   3540
ggtaccattg tattaccttt tattgtttgg atattttatt tatgaaagtg aatttatttt   3600
aaaaaaaaaa                                                          3610
```

<210> 43
<211> 749
<212> PRT
<213> Pinus radiata

<400> 43

```
Met Lys Glu Met Ala Glu Tyr Cys Ser Pro Ala Leu Leu Glu Leu Ala
1               5                   10                  15
Ala Asn Asn Asp Leu Ser Gly Phe Lys Gln Ala Val Glu Glu Gly Gly
            20                  25                  30
Ser Ser Val Asn Glu Arg Gly Leu Trp Tyr Gly Arg Gln Ile Gly Ser
        35                  40                  45
Gly Gln Lys Met Val Leu Glu Gln Arg Thr Pro Leu Met Val Ala Ala
    50                  55                  60
Leu Tyr Gly Ser Leu Asp Val Leu Ser Tyr Met Leu Ser Gly Gly Arg
65                  70                  75                  80
Val Asp Val Asn Gln Ser Cys Gly Ser Asp Met Ser Thr Ala Leu His
                85                  90                  95
Cys Ala Ala Ala Gly Gly Ser Ile Leu Ala Ile Glu Thr Val Gly Met
            100                 105                 110
```

```
Leu Ile Lys Ala Gly Ala Asp Val Asn Phe Met Asn Ala Gly Gly Arg
        115                 120                 125
Lys Pro Ala Asp Val Ile Met Val Ser Pro Lys Leu Ala His Phe Lys
    130                 135                 140
Asn Val Leu Glu Asp Leu Leu Ile Met Gly Ser Asn Ser Pro Met Lys
145                 150                 155                 160
Ile Pro Cys Arg Val Ser Gly Ser Gly Phe Tyr Leu Pro Glu Gly Gly
                165                 170                 175
Gly Cys Phe Phe Asp Glu His Gly Cys Val Val Ser Val Pro Thr Ser
            180                 185                 190
Ser Pro Leu Phe Ser Ser Pro Asp Ala Thr Ser Pro Ala Thr Val Asn
        195                 200                 205
Ser Pro Leu Ser Ser Pro Pro Thr Ser Leu Asp Thr Pro Lys Asn Leu
    210                 215                 220
Cys Asp Cys Gly Gln Lys Lys Glu Phe Ala Val Asp Ser Ser Leu Pro
225                 230                 235                 240
Asp Ile Lys Asn Ser Ile Tyr Ser Thr Asp Glu Phe Arg Met Tyr Ser
                245                 250                 255
Phe Lys Val Arg Pro Cys Ser Arg Ala Tyr Ser His Asp Trp Thr Glu
            260                 265                 270
Cys Pro Phe Val His Pro Gly Glu Asn Ala Arg Arg Arg Asp Pro Arg
        275                 280                 285
Lys Tyr His Tyr Ser Cys Val Pro Cys Pro Asp Phe Arg Lys Gly Ala
    290                 295                 300
Cys Arg Arg Gly Asp Val Cys Glu Tyr Ala His Gly Val Phe Glu Cys
305                 310                 315                 320
Trp Leu His Pro Ala Gln Tyr Arg Thr Arg Leu Cys Lys Asp Gly Thr
                325                 330                 335
Asn Cys Ser Arg Arg Val Cys Phe Phe Ala His Thr Pro Glu Glu Leu
            340                 345                 350
Arg Pro Leu Tyr Pro Pro Ala Cys Ser Ser Met Leu Ser Gln Arg Thr
        355                 360                 365
Thr Met Thr Ser Ser Asp Lys Met Ala Val Met His Pro Leu Ala Pro
    370                 375                 380
Gly Ser Ala Ser Ser Val Leu Met Met Ser Ser Ser Asn Ser Ser Gln
385                 390                 395                 400
Ser Ser Phe Pro Asn Ser Pro Val Ser Pro Leu Ser Ser Ala Asn Thr
                405                 410                 415
Ser Ser His Ser Ser Phe Gly Gly Gly Ser Trp Ala His Pro Asn Leu
            420                 425                 430
Pro Thr Leu His Leu Ser Asn Gly Ala Leu Gln Ala Ser Arg Leu Arg
        435                 440                 445
Thr Ala Val Asn Ala Arg Asp Met His Pro Asp Cys Ser Ile Glu Ser
    450                 455                 460
Gly Asp Tyr Glu Gly Gln Leu Leu Asn Glu Phe Ala Tyr Leu Ser Thr
465                 470                 475                 480
Gln Ala Arg Gly Asn Gly Pro Met Ala Thr Val Ser Ser Ser Gly Asn
                485                 490                 495
Thr Pro Cys Arg Pro Arg Lys Phe Arg Ala His Asn Val Ala Pro Thr
            500                 505                 510
Asn Leu Glu Asp Leu Phe Ala Ser Glu Val Phe Ser Pro Lys Met Thr
        515                 520                 525
Ala Ser Glu Ser Ala Phe Leu Ser Glu Ile Gln Ser His Lys Ser Ala
    530                 535                 540
Gln Leu Ser Pro Gln Leu Gln Ser Gln Met Leu Ser Ser Phe Asn Thr
545                 550                 555                 560
Gln Val Tyr Pro Gln Gly Ser Thr Gln Gly Gln Met His Met Gln His
                565                 570                 575
Gly Gly Val Asp Cys Gln Ser Pro Ser Val Phe Leu Ser Pro Pro Pro
```

```
                    580                     585                     590
Val Gln Leu Ala Ser Tyr Ser Leu Ser Ser Leu Gly Pro Leu Ser Ser
                595                     600                     605
Leu Thr Gly Glu Leu Glu Arg Gln Asn Ser Asn Gly Ser Pro Leu Ser
        610                     615                     620
Pro Ile Met Ser Thr Ala Ala Asp Ser Arg Ala Val Ala Phe Ser Gln
625                     630                     635                     640
Arg Asp Lys Gly Ser Ser Arg Ser Gly Asp Leu Gly Gly Ala Thr Thr
                        645                     650                     655
Trp Ser Glu Trp Gly Ser Pro Thr Gly Lys Val Asn Trp Gly Ile Arg
                    660                     665                     670
Gly Glu Glu Leu Gln Lys Phe Arg Lys Ser Ala Ser Phe Gly Ile Arg
                675                     680                     685
Ser Ser Asp Glu Pro Asp Leu Ser Trp Val Gln Lys Leu Phe Lys Glu
        690                     695                     700
Ala Pro Met Glu Ser Met Asp Arg Gly Thr Met Gly Arg Ser Met Asp
705                     710                     715                     720
Ile Ala Asn Ser Val Gln Met Glu Ala Thr Asp Leu Gly Gly Trp Ile
                        725                     730                     735
Ser Gln Ile Asn Pro Asp Gln Val Ala Pro Leu Thr Leu
                    740                     745
```

<210> 44
<211> 711
<212> PRT
<213> Glycine max

<400> 44

```
Lys Ser Ala Asn Asp Lys Glu Met Lys Ser Leu Thr Val Asn Thr Glu
1               5                   10                  15
Asp Ser Phe Ser Ser Leu Leu Glu Leu Ala Ser Asn Asn Asp Ile Glu
            20                  25                  30
Gly Phe Lys Val Leu Leu Glu Lys Asp Ser Ser Ser Ile Asn Glu Val
        35                  40                  45
Gly Leu Trp Tyr Gly Arg Gln Asn Gly Ser Lys Gln Phe Val Leu Glu
    50                  55                  60
His Arg Thr Pro Leu Met Val Ala Ala Thr Tyr Gly Ser Ile Asp Val
65                  70                  75                  80
Met Lys Ile Ile Leu Leu Cys Pro Glu Ala Asp Val Asn Phe Ala Cys
                85                  90                  95
Gly Ala Asn Lys Thr Thr Ala Leu His Cys Ala Ala Ser Gly Gly Ser
            100                 105                 110
Ala Asn Ala Val Asp Ala Val Lys Ile Leu Leu Ser Ala Gly Ala Asp
        115                 120                 125
Val Asn Gly Val Asp Ala Asn Gly Asn Arg Pro Ile Asp Val Ile Ala
    130                 135                 140
Val Pro Pro Lys Leu Gln Gly Ala Lys Ala Val Leu Glu Glu Leu Leu
145                 150                 155                 160
Ser Asp Ser Ala Ser Glu Gly Ser Ile Gly Glu Phe Ser Val Pro Val
                165                 170                 175
Ser Val Asn Thr Ser Ser Leu Gly Ser Pro Gly His Ser Ser Asn Gly
            180                 185                 190
Met Pro Tyr Thr Pro Ser Ser Ser Pro Pro Ser Pro Val Val Ala Lys
        195                 200                 205
Phe Thr Asp Ala Ala Val Cys Ser Leu Ser Glu Lys Lys Glu Tyr Pro
    210                 215                 220
Ile Asp Pro Ser Leu Pro Asp Ile Lys Asn Ser Ile Tyr Ala Thr Asp
225                 230                 235                 240
Glu Phe Arg Met Phe Ser Phe Lys Val Arg Pro Cys Ser Arg Ala Tyr
```

```
                245                 250                 255
Ser His Asp Trp Thr Glu Cys Pro Phe Val His Pro Gly Glu Asn Ala
            260                 265                 270
Arg Arg Arg Asp Pro Arg Lys Phe His Tyr Ser Cys Val Pro Cys Pro
        275                 280                 285
Asp Phe Arg Lys Gly Ala Cys Arg Arg Gly Asp Met Cys Glu Tyr Ala
    290                 295                 300
His Gly Val Phe Glu Cys Trp Leu His Pro Ala Gln Tyr Arg Thr Arg
305                 310                 315                 320
Leu Cys Lys Asp Gly Thr Ser Cys Asn Arg Arg Val Cys Phe Phe Ala
                325                 330                 335
His Thr Ala Glu Glu Leu Arg Pro Leu Tyr Val Ser Thr Gly Ser Ala
            340                 345                 350
Val Pro Ser Pro Arg Ser Ser Ala Ser Ala Pro Asn Val Met Asp Met
        355                 360                 365
Ala Ala Ala Met Ser Leu Leu Pro Gly Ser Pro Ser Ser Val Ser Ser
    370                 375                 380
Met Ser Pro Ser His Phe Gly Gln Pro Met Ser Pro Ser Ala Asn Gly
385                 390                 395                 400
Met Ser Leu Ser Ser Ala Trp Ala Gln Pro Asn Val Ser Ala Leu His
                405                 410                 415
Leu Pro Gly Ser Asn Leu Gln Ser Ser Arg Leu Arg Ser Ser Leu Ser
            420                 425                 430
Ala Arg Asp Met Pro Pro Asp Asp Leu Asn Met Met Ser Asp Leu Asp
        435                 440                 445
Gly Gln Gln Gln His Pro Leu Asn Asp Leu Ser Cys Tyr Leu Gln Pro
    450                 455                 460
Arg Pro Gly Ala Gly Ser Val Ser Arg Ser Gly Arg Ser Lys Ile Leu
465                 470                 475                 480
Thr Pro Ser Asn Leu Glu Asp Leu Phe Ser Ala Glu Ile Ser Ser Ser
                485                 490                 495
Pro Arg Tyr Ser Asp Pro Ala Ala Gly Ser Val Phe Ser Pro Thr His
            500                 505                 510
Lys Ser Ala Val Leu Asn Gln Phe Gln Gln Leu Gln Ser Met Leu Ser
        515                 520                 525
Pro Ile Asn Thr Asn Leu Leu Ser Pro Lys Asn Val Glu His Pro Leu
    530                 535                 540
Leu Gln Ala Ser Phe Gly Val Ser Pro Ser Gly Arg Met Ser Pro Arg
545                 550                 555                 560
Ser Val Glu Pro Ile Ser Pro Met Ser Ser Arg Ile Ser Ala Phe Ala
                565                 570                 575
Gln Arg Glu Lys Gln Gln Gln Gln Gln Gln Gln Leu Arg Ser Leu Ser
            580                 585                 590
Ser Arg Asp Leu Gly Ala Asn Ser Pro Ala Ser Leu Val Gly Ser Pro
        595                 600                 605
Ala Asn Pro Trp Ser Lys Trp Gly Ser Pro Asn Gly Lys Ala Asp Trp
    610                 615                 620
Ser Val Asn Gly Asp Thr Leu Gly Arg Gln Met Arg Arg Ser Ser Ser
625                 630                 635                 640
Phe Glu Leu Lys Asn Asn Gly Glu Glu Pro Asp Leu Ser Trp Val Gln
                645                 650                 655
Ser Leu Val Lys Glu Ser Pro Pro Glu Met Ile Lys Glu Lys Phe Ala
            660                 665                 670
Ser Pro Met Pro Thr Ala Ser Ala Asp Gly Pro Asn Ser Asn Ser Gln
        675                 680                 685
Ile Glu Ser Ile Asp His Ser Val Leu Gly Ala Trp Leu Glu Gln Met
    690                 695                 700
Gln Leu Asp Gln Leu Val Val
705                 710
```

<210> 45
<211> 643
<212> PRT
<213> Glycine max

<400> 45

```
Arg Gly Ser Gly Phe Pro Gly Arg Pro Thr Arg Pro Arg Ser Gly Arg
1               5                   10                  15
Thr Arg Gly Arg Thr Arg Gly Val Asn Phe Ala Cys Gly Ala Asn Lys
            20                  25                  30
Thr Thr Ala Leu His Cys Ala Ala Ser Gly Ala Ser Thr Lys Ala Val
        35                  40                  45
Asp Ala Val Lys Leu Leu Leu Ser Ala Gly Ala Asp Val Asn Cys Val
    50                  55                  60
Asp Ala Asn Gly Asn Arg Pro Ile Asp Val Ile Ala Val Pro Pro Lys
65                  70                  75                  80
Leu Gln Gly Ala Lys Ala Val Leu Glu Glu Leu Leu Ser Asp Asn Ala
                85                  90                  95
Ser Asp Val Ser Val Gly Glu Phe Ser Val Pro Val Ser Val Asn Ser
            100                 105                 110
Ser Ser Pro Gly Ser Pro Ala His Ser Ser Asn Gly Met Pro Tyr Thr
        115                 120                 125
Pro Ser Val Ser Pro Pro Ser Pro Val Ala Ala Lys Phe Thr Asp Ala
    130                 135                 140
Ala Ile Cys Ser Leu Ser Glu Lys Ala Arg Glu Tyr Pro Ile Asp Pro
145                 150                 155                 160
Ser Leu Pro Asp Ile Lys Asn Ser Ile Tyr Ala Thr Asp Glu Phe Arg
                165                 170                 175
Met Phe Ser Phe Lys Val Arg Pro Cys Ser Arg Ala Tyr Ser His Asp
            180                 185                 190
Trp Thr Glu Cys Pro Phe Val His Pro Gly Glu Asn Ala Arg Arg Arg
        195                 200                 205
Asp Pro Arg Lys Phe His Tyr Ser Cys Val Pro Cys Pro Asp Phe Arg
    210                 215                 220
Lys Gly Ala Cys Arg Arg Gly Asp Met Cys Glu Tyr Ala His Gly Val
225                 230                 235                 240
Phe Glu Cys Trp Leu His Pro Ala Gln Tyr Arg Thr Arg Leu Cys Lys
                245                 250                 255
Asp Gly Thr Ser Cys Asn Arg Arg Val Cys Phe Phe Ala His Thr Ala
            260                 265                 270
Glu Glu Leu Arg Pro Leu Tyr Val Ser Thr Gly Ser Ala Ala Pro Ser
        275                 280                 285
Pro Arg Ser Ser Ala Ser Gly Pro Asn Val Met Asp Met Ala Ala Ala
    290                 295                 300
Met Ser Leu Phe Pro Gly Ser Pro Ser Ser Gly Ser Ser Ile Ser Leu
305                 310                 315                 320
Ser Ile Ser Phe Ser Leu Asp Pro Met Ser Pro Ser Ala Asn Gly Met
                325                 330                 335
Pro Leu Ser Ser Ala Trp Ala Gln Pro Asn Val Pro Ala Leu His Leu
            340                 345                 350
Pro Gly Ser Asn Leu Gln Ser Ser Arg Leu Arg Ser Ser Leu Ser Ala
        355                 360                 365
Arg Asp Ile Pro Pro Glu Asp Leu Asn Met Met Ser Asp Leu Asp Gly
    370                 375                 380
Gln Gln Gln His His Leu Asn Asp Leu Ser Cys Tyr Ile Gln Pro Arg
385                 390                 395                 400
Pro Gly Ala Ser Ser Val Ser Arg Ser Gly Arg Ser Lys Thr Leu Thr
                405                 410                 415
```

```
Pro Ser Asn Leu Glu Glu Leu Phe Ser Ala Glu Ile Ser Leu Ser Pro
            420                 425                 430
Arg Tyr Ser Asp Pro Ala Ala Gly Ser Val Phe Ser Pro Thr His Lys
        435                 440                 445
Ser Ala Val Leu Asn Gln Phe Gln Gln Leu Gln Ser Met Leu Ser Pro
    450                 455                 460
Ile Asn Thr Asn Leu Leu Ser Pro Lys Asn Val Glu His Pro Leu Phe
465                 470                 475                 480
Gln Ala Ser Phe Gly Val Ser Pro Ser Gly Arg Met Ser Pro Arg Ser
                485                 490                 495
Val Glu Pro Ile Ser Pro Met Ser Ala Arg Leu Ser Ala Phe Ala Gln
            500                 505                 510
Arg Glu Lys Gln Gln Gln Gln Leu Arg Ser Val Ser Ser Arg Asp Leu
        515                 520                 525
Gly Ala Asn Ser Pro Ala Ser Leu Val Gly Ser Pro Ala Asn Pro Trp
    530                 535                 540
Ser Lys Trp Gly Ser Pro Ile Gly Lys Ala Asp Trp Ser Val Asn Gly
545                 550                 555                 560
Asp Ser Leu Gly Arg Gln Met Arg Arg Ser Ser Ser Phe Glu Arg Lys
                565                 570                 575
Asn Asn Gly Glu Glu Pro Asp Leu Ser Trp Val Gln Ser Leu Val Lys
            580                 585                 590
Glu Ser Pro Pro Glu Met Ile Lys Glu Lys Phe Ala Ser Pro Met Pro
        595                 600                 605
Thr Ala Ser Ala Asp Gly Pro Asn Ser Asn Ser Gln Ile Glu Ser Ile
    610                 615                 620
Asp His Ser Val Leu Gly Ala Trp Leu Glu Gln Met Gln Leu Asp Gln
625                 630                 635                 640
Leu Val Val
```

<210> 46
<211> 669
<212> PRT
<213> Glycine max

<400> 46

```
Ser His Glu Met Asn His Leu Ser Leu Asp Thr Glu Asp Ser Leu Ala
1               5                   10                  15
Ser Leu Leu Leu Glu Leu Ala Ala Asn Asn Asp Val Ser Gly Phe Lys
            20                  25                  30
Arg Leu Ile Glu Cys Glu Pro Ser Ser Ile Asp Glu Val Gly Leu Trp
        35                  40                  45
Tyr Gly Arg His Lys Glu Ser Lys Lys Met Val Asn Glu Gln Arg Thr
    50                  55                  60
Pro Leu Met Val Ala Ala Thr Tyr Gly Ser Ile Asp Val Met Thr Leu
65                  70                  75                  80
Ile Leu Ser Leu Ser Glu Ala Asp Val Asn Arg Ser Ser Gly Leu Asp
                85                  90                  95
Lys Ser Thr Ala Leu His Cys Ala Ala Ser Gly Gly Ser Glu Asn Ala
            100                 105                 110
Val Asp Ala Val Lys Leu Leu Leu Glu Ala Gly Ala Asp Arg Asn Ser
        115                 120                 125
Val Asp Ala Asn Gly Arg Arg Pro Gly Asp Val Ile Val Ser Pro Pro
    130                 135                 140
Lys Leu Asp Tyr Val Lys Lys Ser Leu Glu Glu Leu Leu Gly Ser Asp
145                 150                 155                 160
Asp Trp Ser Leu Leu Arg Val Met Arg Ser Thr Cys Asn Gly Cys Ser
                165                 170                 175
```

```
Ala Glu Asp Leu Lys Met Lys Thr Asn Glu Val Ser Glu Lys Lys Glu
            180                 185                 190
Tyr Pro Val Asp Leu Ser Leu Pro Asp Ile Lys Asn Ser Ile Tyr Ser
        195                 200                 205
Ser Asp Glu Phe Arg Met Tyr Ser Phe Lys Val Arg Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Phe His Tyr Ser Cys Val Pro
                245                 250                 255
Cys Pro Glu Phe Arg Lys Gly Ala Cys Arg Arg Gly Asp Met Cys Glu
            260                 265                 270
Tyr Ala His Gly Val Phe Glu Cys Trp Leu His Pro Ala Gln Tyr Arg
        275                 280                 285
Thr Arg Leu Cys Lys Asp Gly Thr Asn Cys Ala Arg Arg Val Cys Phe
    290                 295                 300
Phe Ala His Thr Asn Glu Glu Leu Arg Pro Leu Tyr Val Ser Thr Gly
305                 310                 315                 320
Ser Ala Val Pro Ser Pro Arg Ser Ser Ala Ser Ser Ala Met Asp Phe
                325                 330                 335
Val Ala Ala Ile Ser Pro Ser Ser Met Ser Val Met Ser Pro Ser Pro
            340                 345                 350
Phe Thr Pro Pro Met Ser Pro Ser Ser Ala Ser Ile Ala Trp Pro Gln
        355                 360                 365
Pro Asn Ile Pro Ala Leu His Leu Pro Gly Ser Asn Phe His Ser Ser
    370                 375                 380
Arg Leu Arg Ser Ser Leu Asn Ala Arg Asp Phe Ser Val Asp Asp Phe
385                 390                 395                 400
Asp Leu Leu Leu Pro Asp Tyr Asp His His His His Gln Gln Gln Gln
                405                 410                 415
Gln Gln Phe Leu Asn Glu Leu Ser Cys Leu Ser Pro His Ala Met Asn
            420                 425                 430
Cys Asn Thr Met Asn Arg Ser Gly Arg Met Lys Pro Leu Thr Pro Ser
        435                 440                 445
Asn Leu Asp Asp Leu Phe Ser Ala Glu Ser Ser Ser Pro Arg Tyr Ala
    450                 455                 460
Asp Pro Ala Leu Ala Ser Ala Val Phe Ser Pro Thr His Lys Ser Ala
465                 470                 475                 480
Val Phe Asn Gln Phe Gln His Gln Gln Ser Met Leu Ala Pro Leu Asn
                485                 490                 495
Thr Asn Phe Ala Ser Lys Asn Phe Glu His Pro Leu Leu Gln Ala Ser
            500                 505                 510
Leu Gly Met Ser Pro Arg Asn Val Glu Pro Ile Ser Pro Met Gly Ser
        515                 520                 525
Arg Ile Ser Met Leu Ala Gln Arg Glu Lys Gln Gln Phe Arg Ser Leu
    530                 535                 540
Ser Phe Arg Glu Leu Gly Ser Asn Ser Ala Ala Ala Ser Ala Asp Ser
545                 550                 555                 560
Trp Ser Lys Trp Gly Ser Pro Asn Val Lys Leu Asp Trp Pro Val Gly
                565                 570                 575
Ala Gly Glu Val Gly Lys Leu Arg Arg Ser Ser Ser Phe Glu Leu Gly
            580                 585                 590
Asn Asn Gly Glu Glu Pro Asp Leu Ser Trp Val Gln Ser Leu Val Lys
        595                 600                 605
Glu Ser Pro Ala Glu Val Lys Asp Lys Leu Ala Thr Thr Val Ser Tyr
    610                 615                 620
Val Ala Ala Ala Ala Ala Gly Ser Ser Ser Glu Gly Ser Asn Ile Ser
625                 630                 635                 640
Thr Gln Met Glu Ser Val Val Asp His Ala Val Leu Gly Ala Trp Leu
```

645 650 655

Glu Gln Met Gln Leu Asp His Leu Val Ala Gln Gln Asn

660 665

<210> 47
<211> 580
<212> PRT
<213> Arabidopsis thaliana

<400> 47

Met Cys Ser Gly Pro Lys Ser Asn Leu Cys Ser Ser Arg Thr Leu Thr
1 5 10 15
Glu Ile Glu Ser Arg Gln Lys Glu Glu Glu Thr Met Leu Leu Leu Glu
20 25 30
Phe Ala Ala Cys Asp Asp Leu Asp Ser Phe Lys Arg Glu Val Glu Glu
35 40 45
Lys Gly Leu Asp Leu Asp Glu Ser Gly Leu Trp Tyr Cys Arg Arg Val
50 55 60
Gly Ser Lys Lys Met Gly Leu Glu Glu Arg Thr Pro Leu Met Val Ala
65 70 75 80
Ala Met Tyr Gly Ser Ile Lys Val Leu Thr Phe Ile Val Ser Thr Gly
85 90 95
Lys Ser Asp Val Asn Arg Ala Cys Gly Glu Glu Arg Val Thr Pro Leu
100 105 110
His Cys Ala Val Ala Gly Cys Ser Val Asn Met Ile Glu Val Ile Asn
115 120 125
Val Leu Leu Asp Ala Ser Ala Leu Val Asn Ser Val Asp Ala Asn Gly
130 135 140
Asn Gln Pro Leu Asp Val Phe Val Arg Val Ser Arg Phe Val Ala Ser
145 150 155 160
Pro Arg Arg Lys Ala Val Glu Leu Leu Leu Arg Gly Gly Gly Val Gly
165 170 175
Gly Leu Ile Asp Glu Ala Val Glu Glu Glu Ile Lys Ile Val Ser Lys
180 185 190
Tyr Pro Ala Asp Ala Ser Leu Pro Asp Ile Asn Glu Gly Val Tyr Gly
195 200 205
Ser Asp Glu Phe Arg Met Tyr Ser Phe Lys Val Lys Pro Cys Ser Arg
210 215 220
Ala Tyr Ser His Asp Trp Thr Glu Cys Ala Phe Val His Pro Gly Glu
225 230 235 240
Asn Ala Arg Arg Arg Asp Pro Arg Lys Tyr Pro Tyr Thr Cys Val Pro
245 250 255
Cys Pro Glu Phe Arg Lys Gly Ser Cys Pro Lys Gly Asp Ser Cys Glu
260 265 270
Tyr Ala His Gly Val Phe Glu Ser Trp Leu His Pro Ala Gln Tyr Lys
275 280 285
Thr Arg Leu Cys Lys Asp Glu Thr Gly Cys Ala Arg Lys Val Cys Phe
290 295 300
Phe Ala His Lys Arg Glu Glu Met Arg Pro Val Asn Ala Ser Thr Gly
305 310 315 320
Ser Ala Val Ala Gln Ser Pro Phe Ser Ser Leu Glu Met Met Pro Gly
325 330 335
Leu Ser Pro Leu Ala Tyr Ser Ser Gly Val Ser Thr Pro Pro Val Ser
340 345 350
Pro Met Ala Asn Gly Val Pro Ser Ser Pro Arg Asn Gly Gly Ser Trp
355 360 365
Gln Asn Arg Val Asn Thr Leu Thr Pro Pro Ala Leu Gln Leu Asn Gly
370 375 380
Gly Ser Arg Leu Lys Ser Thr Leu Ser Ala Arg Asp Ile Asp Met Glu

```
385                 390                 395                 400
Met Glu Met Glu Leu Arg Leu Arg Gly Phe Gly Asn Asn Val Glu Glu
                405                 410                 415
Thr Phe Gly Ser Tyr Val Ser Ser Pro Ser Arg Asn Ser Gln Met Gly
            420                 425                 430
Gln Asn Met Asn Gln His Tyr Pro Ser Ser Pro Val Arg Gln Pro Pro
        435                 440                 445
Ser Gln His Gly Phe Glu Ser Ser Ala Ala Ala Ala Val Ala Val Met
    450                 455                 460
Lys Ala Arg Ser Thr Ala Phe Ala Lys Arg Ser Leu Ser Phe Lys Pro
465                 470                 475                 480
Ala Thr Gln Ala Ala Pro Gln Ser Asn Leu Ser Asp Trp Gly Ser Pro
                485                 490                 495
Asn Gly Lys Leu Glu Trp Gly Met Lys Gly Glu Glu Leu Asn Lys Met
            500                 505                 510
Arg Arg Ser Val Ser Phe Gly Ile His Gly Asn Asn Asn Asn Asn Ala
        515                 520                 525
Ala Arg Asp Tyr Arg Asp Glu Pro Asp Val Ser Trp Val Asn Ser Leu
    530                 535                 540
Val Lys Asp Ser Thr Val Val Ser Glu Arg Ser Phe Gly Met Asn Glu
545                 550                 555                 560
Arg Val Arg Ile Met Ser Trp Ala Glu Gln Met Tyr Arg Glu Lys Glu
                565                 570                 575
Gln Thr Val Val
            580
```

<210> 48
<211> 719
<212> PRT
<213> Arabidopsis thaliana

<400> 48

```
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
            20                  25                  30
Asp Met Asn His Ile Thr Val Glu Thr Glu Asp Thr Phe Ala Ser Leu
        35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
```

```
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu
    450                 455                 460
Asn Glu Tyr Ser Asn Ala Leu Ser Arg Ser Gly Arg Met Lys Ser Met
465                 470                 475                 480
Pro Pro Ser Asn.Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Gln Gln Ser Met Leu Ser Pro Ile Asn Thr Ser Phe Ser Ser Pro
    530                 535                 540
Lys Ser Val Asp His Ser Leu Phe Ser Gly Gly Gly Arg Met Ser Pro
545                 550                 555                 560
Arg Asn Val Val Glu Pro Ile Ser Pro Met Ser Ala Arg Val Ser Met
                565                 570                 575
Leu Ala Gln Cys Val Lys Gln Gln Gln Gln Gln Gln Gln Gln Gln Gln
            580                 585                 590
Gln Gln Gln His Gln Phe Arg Ser Leu Ser Ser Arg Glu Leu Arg Thr
        595                 600                 605
Asn Ser Ser Pro Ile Val Gly Ser Pro Val Asn Asn Asn Thr Trp Ser
    610                 615                 620
Ser Lys Trp Gly Ser Ser Asn Gly Gln Pro Asp Trp Gly Met Ser Ser
625                 630                 635                 640
Glu Ala Leu Gly Lys Leu Arg Ser Ser Ser Ser Phe Asp Gly Asp Glu
                645                 650                 655
Pro Asp Val Ser Trp Val Gln Ser Leu Val Lys Glu Thr Pro Ala Glu
            660                 665                 670
Ala Lys Glu Lys Ala Ala Thr Ser Ser Ser Gly Glu His Val Met Lys
        675                 680                 685
```

```
Gln Pro Asn Pro Val Glu Pro Val Met Asp His Ala Gly Leu Glu Ala
    690                 695                 700
Trp Ile Glu Gln Met Gln Leu Asp Gln Leu Val Ala Gln Gln Asn
705                 710                 715
```

<210> 49
<211> 686
<212> PRT
<213> Arabidopsis thaliana

<400> 49

```
Met Asn His Ile Thr Val Glu Thr Glu Asp Thr Phe Ala Ser Leu Leu
1               5                   10                  15
Glu Leu Ala Ala Asn Asn Asp Val Glu Gly Val Arg Leu Ser Ile Glu
            20                  25                  30
Arg Asp Pro Ser Cys Val Asp Glu Ala Gly Leu Trp Tyr Gly Arg Gln
        35                  40                  45
Lys Gly Ser Lys Ala Met Val Asn Asp Tyr Arg Thr Pro Leu Met Val
    50                  55                  60
Ala Ala Thr Tyr Gly Ser Ile Asp Val Ile Lys Leu Ile Val Ser Leu
65                  70                  75                  80
Thr Asp Ala Asp Val Asn Arg Ala Cys Gly Asn Asp Gln Thr Thr Ala
                85                  90                  95
Leu His Cys Ala Ala Ser Gly Gly Ala Val Asn Ala Ile Gln Val Val
            100                 105                 110
Lys Leu Leu Leu Ala Ala Gly Ala Asp Leu Asn Leu Leu Asp Ala Glu
        115                 120                 125
Gly Gln Arg Ala Gly Asp Val Ile Val Val Pro Pro Lys Leu Glu Gly
    130                 135                 140
Val Lys Leu Met Leu Gln Glu Leu Leu Ser Ala Asp Gly Ser Ser Thr
145                 150                 155                 160
Ala Glu Arg Asn Leu Arg Val Val Thr Asn Val Pro Asn Arg Ser Ser
                165                 170                 175
Ser Pro Cys His Ser Pro Thr Gly Glu Asn Gly Gly Ser Gly Ser Gly
            180                 185                 190
Ser Pro Leu Gly Ser Pro Phe Lys Leu Lys Ser Thr Glu Phe Lys Lys
        195                 200                 205
Glu Tyr Pro Val Asp Pro Ser Leu Pro Asp Ile Lys Asn Ser Ile Tyr
    210                 215                 220
Ala Thr Asp Glu Phe Arg Met Tyr Ser Phe Lys Val Arg Pro Cys Ser
225                 230                 235                 240
Arg Ala Tyr Ser His Asp Trp Thr Glu Cys Pro Phe Val His Pro Gly
                245                 250                 255
Glu Asn Ala Arg Arg Arg Asp Pro Arg Lys Phe His Tyr Ser Cys Val
            260                 265                 270
Pro Cys Pro Asp Phe Arg Lys Gly Ala Cys Arg Arg Gly Asp Met Cys
        275                 280                 285
Glu Tyr Ala His Gly Val Phe Glu Cys Trp Leu His Pro Ala Gln Tyr
    290                 295                 300
Arg Thr Arg Leu Cys Lys Asp Gly Thr Gly Cys Ala Arg Arg Val Cys
305                 310                 315                 320
Phe Phe Ala His Thr Pro Glu Glu Leu Arg Pro Leu Tyr Ala Ser Thr
                325                 330                 335
Gly Ser Ala Val Pro Ser Pro Arg Ser Asn Ala Asp Tyr Ala Ala Ala
            340                 345                 350
Leu Ser Leu Leu Pro Gly Ser Pro Ser Gly Val Ser Val Met Ser Pro
        355                 360                 365
Leu Ser Pro Ser Ala Ala Gly Asn Gly Met Ser His Ser Asn Met Ala
    370                 375                 380
```

```
Trp Pro Gln Pro Asn Val Pro Ala Leu His Leu Pro Gly Ser Asn Leu
385                 390                 395                 400
Gln Ser Ser Arg Leu Arg Ser Ser Leu Asn Ala Arg Asp Ile Pro Thr
                405                 410                 415
Asp Glu Phe Asn Met Leu Ala Asp Tyr Glu Gln Gln Gln Leu Leu Asn
            420                 425                 430
Glu Tyr Ser Asn Ala Leu Ser Arg Ser Gly Arg Met Lys Ser Met Pro
        435                 440                 445
Pro Ser Asn Leu Glu Asp Leu Phe Ser Ala Glu Gly Ser Ser Ser Pro
    450                 455                 460
Arg Phe Thr Asp Ser Ala Leu Ala Ser Ala Val Phe Ser Pro Thr His
465                 470                 475                 480
Lys Ser Ala Val Phe Asn Gln Phe Gln Gln Gln Gln Gln Gln Gln Gln
                485                 490                 495
Gln Gln Ser Met Leu Ser Pro Ile Asn Thr Ser Phe Ser Ser Pro Lys
            500                 505                 510
Ser Val Asp His Ser Leu Phe Ser Gly Gly Gly Arg Met Ser Pro Arg
        515                 520                 525
Asn Val Val Glu Pro Ile Ser Pro Met Ser Ala Arg Val Ser Met Leu
    530                 535                 540
Ala Gln Cys Val Lys Gln Gln Gln Gln Gln Gln Gln Gln Gln Gln Gln
545                 550                 555                 560
Gln Gln His Gln Phe Arg Ser Leu Ser Ser Arg Glu Leu Arg Thr Asn
                565                 570                 575
Ser Ser Pro Ile Val Gly Ser Pro Val Asn Asn Asn Thr Trp Ser Ser
            580                 585                 590
Lys Trp Gly Ser Ser Asn Gly Gln Pro Asp Trp Gly Met Ser Ser Glu
        595                 600                 605
Ala Leu Gly Lys Leu Arg Ser Ser Ser Ser Phe Asp Gly Asp Glu Pro
    610                 615                 620
Asp Val Ser Trp Val Gln Ser Leu Val Lys Glu Thr Pro Ala Glu Ala
625                 630                 635                 640
Lys Glu Lys Ala Ala Thr Ser Ser Ser Gly Glu His Val Met Lys Gln
                645                 650                 655
Pro Asn Pro Val Glu Pro Val Met Asp His Ala Gly Leu Glu Ala Trp
            660                 665                 670
Ile Glu Gln Met Gln Leu Asp Gln Leu Val Ala Gln Gln Asn
        675                 680                 685
```

<210> 50
<211> 633
<212> PRT
<213> Arabidopsis thaliana

<400> 50

```
Met Val Asn Asp Tyr Arg Thr Pro Leu Met Val Ala Ala Thr Tyr Gly
1               5                   10                  15
Ser Ile Asp Val Ile Lys Leu Ile Val Ser Leu Thr Asp Ala Asp Val
            20                  25                  30
Asn Arg Ala Cys Gly Asn Asp Gln Thr Thr Ala Leu His Cys Ala Ala
        35                  40                  45
Ser Gly Gly Ala Val Asn Ala Ile Gln Val Val Lys Leu Leu Leu Ala
    50                  55                  60
Ala Gly Ala Asp Leu Asn Leu Leu Asp Ala Glu Gly Gln Arg Ala Gly
65                  70                  75                  80
Asp Val Ile Val Val Pro Pro Lys Leu Glu Gly Val Lys Leu Met Leu
                85                  90                  95
Gln Glu Leu Leu Ser Ala Asp Gly Ser Ser Thr Ala Glu Arg Asn Leu
            100                 105                 110
```

```
Arg Val Val Thr Asn Val Pro Asn Arg Ser Ser Ser Pro Cys His Ser
        115                 120                 125
Pro Thr Gly Glu Asn Gly Gly Ser Gly Ser Gly Ser Pro Leu Gly Ser
    130                 135                 140
Pro Phe Lys Leu Lys Ser Thr Glu Phe Lys Lys Glu Tyr Pro Val Asp
145                 150                 155                 160
Pro Ser Leu Pro Asp Ile Lys Asn Ser Ile Tyr Ala Thr Asp Glu Phe
                165                 170                 175
Arg Met Tyr Ser Phe Lys Val Arg Pro Cys Ser Arg Ala Tyr Ser His
            180                 185                 190
Asp Trp Thr Glu Cys Pro Phe Val His Pro Gly Glu Asn Ala Arg Arg
        195                 200                 205
Arg Asp Pro Arg Lys Phe His Tyr Ser Cys Val Pro Cys Pro Asp Phe
    210                 215                 220
Arg Lys Gly Ala Cys Arg Arg Gly Asp Met Cys Glu Tyr Ala His Gly
225                 230                 235                 240
Val Phe Glu Cys Trp Leu His Pro Ala Gln Tyr Arg Thr Arg Leu Cys
                245                 250                 255
Lys Asp Gly Thr Gly Cys Ala Arg Arg Val Cys Phe Phe Ala His Thr
            260                 265                 270
Pro Glu Glu Leu Arg Pro Leu Tyr Ala Ser Thr Gly Ser Ala Val Pro
        275                 280                 285
Ser Pro Arg Ser Asn Ala Asp Tyr Ala Ala Ala Leu Ser Leu Leu Pro
    290                 295                 300
Gly Ser Pro Ser Gly Val Ser Val Met Ser Pro Leu Ser Pro Ser Ala
305                 310                 315                 320
Ala Gly Asn Gly Met Ser His Ser Asn Met Ala Trp Pro Gln Pro Asn
                325                 330                 335
Val Pro Ala Leu His Leu Pro Gly Ser Asn Leu Gln Ser Ser Arg Leu
            340                 345                 350
Arg Ser Ser Leu Asn Ala Arg Asp Ile Pro Thr Asp Glu Phe Asn Met
        355                 360                 365
Leu Ala Asp Tyr Glu Gln Gln Gln Leu Leu Asn Glu Tyr Ser Asn Ala
    370                 375                 380
Leu Ser Arg Ser Gly Arg Met Lys Ser Met Pro Pro Ser Asn Leu Glu
385                 390                 395                 400
Asp Leu Phe Ser Ala Glu Gly Ser Ser Ser Pro Arg Phe Thr Asp Ser
                405                 410                 415
Ala Leu Ala Ser Ala Val Phe Ser Pro Thr His Lys Ser Ala Val Phe
            420                 425                 430
Asn Gln Phe Gln Gln Gln Gln Gln Gln Gln Gln Gln Gln Ser Met Leu
        435                 440                 445
Ser Pro Ile Asn Thr Ser Phe Ser Ser Pro Lys Ser Val Asp His Ser
    450                 455                 460
Leu Phe Ser Gly Gly Gly Arg Met Ser Pro Arg Asn Val Val Glu Pro
465                 470                 475                 480
Ile Ser Pro Met Ser Ala Arg Val Ser Met Leu Ala Gln Cys Val Lys
                485                 490                 495
Gln Gln Gln Gln Gln Gln Gln Gln Gln Gln Gln Gln Gln His Gln Phe
            500                 505                 510
Arg Ser Leu Ser Ser Arg Glu Leu Arg Thr Asn Ser Ser Pro Ile Val
        515                 520                 525
Gly Ser Pro Val Asn Asn Asn Thr Trp Ser Ser Lys Trp Gly Ser Ser
    530                 535                 540
Asn Gly Gln Pro Asp Trp Gly Met Ser Ser Glu Ala Leu Gly Lys Leu
545                 550                 555                 560
Arg Ser Ser Ser Ser Phe Asp Gly Asp Glu Pro Asp Val Ser Trp Val
                565                 570                 575
Gln Ser Leu Val Lys Glu Thr Pro Ala Glu Ala Lys Glu Lys Ala Ala
```

```
            580                     585                     590
Thr Ser Ser Ser Gly Glu His Val Met Lys Gln Pro Asn Pro Val Glu
        595                     600                     605
Pro Val Met Asp His Ala Gly Leu Glu Ala Trp Ile Glu Gln Met Gln
    610                     615                     620
Leu Asp Gln Leu Val Ala Gln Gln Asn
625                 630
```

<210> 51
<211> 678
<212> PRT
<213> Arabidopsis thaliana

<400> 51

```
Met Asn Asp Ala Ala Glu Trp Glu His Ser Phe Ser Ala Leu Leu Glu
1               5                   10                  15
Phe Ala Ala Asp Asn Asp Val Glu Gly Phe Arg Arg Gln Leu Ser Asp
            20                  25                  30
Val Ser Cys Ile Asn Gln Met Gly Leu Trp Tyr Arg Arg Gln Arg Phe
        35                  40                  45
Val Arg Arg Met Val Leu Glu Gln Arg Thr Pro Leu Met Val Ala Ser
    50                  55                  60
Leu Tyr Gly Ser Leu Asp Val Val Lys Phe Ile Leu Ser Phe Pro Glu
65                  70                  75                  80
Ala Glu Leu Asn Leu Ser Cys Gly Pro Asp Lys Ser Thr Ala Leu His
                85                  90                  95
Cys Ala Ala Ser Gly Ala Ser Val Asn Ser Leu Asp Val Val Lys Leu
            100                 105                 110
Leu Leu Ser Val Gly Ala Asp Pro Asn Ile Pro Asp Ala His Gly Asn
        115                 120                 125
Arg Pro Val Asp Val Leu Val Val Ser Pro His Ala Pro Gly Leu Arg
    130                 135                 140
Thr Ile Leu Glu Glu Ile Leu Lys Lys Asp Glu Ile Ile Ser Glu Asp
145                 150                 155                 160
Leu His Ala Ser Ser Ser Ser Leu Gly Ser Ser Phe Arg Ser Leu Ser
                165                 170                 175
Ser Ser Pro Asp Asn Gly Ser Ser Leu Leu Ser Leu Asp Ser Val Ser
            180                 185                 190
Ser Pro Thr Lys Pro His Gly Thr Asp Val Thr Phe Ala Ser Glu Lys
        195                 200                 205
Lys Glu Tyr Pro Ile Asp Pro Ser Leu Pro Asp Ile Lys Ser Gly Ile
    210                 215                 220
Tyr Ser Thr Asp Glu Phe Arg Met Phe Ser Phe Lys Ile Arg Pro Cys
225                 230                 235                 240
Ser Arg Ala Tyr Ser His Asp Trp Thr Glu Cys Pro Phe Ala His Pro
                245                 250                 255
Gly Glu Asn Ala Arg Arg Arg Asp Pro Arg Lys Phe His Tyr Thr Cys
            260                 265                 270
Val Pro Cys Pro Asp Phe Lys Lys Gly Ser Cys Lys Gln Gly Asp Met
        275                 280                 285
Cys Glu Tyr Ala His Gly Val Phe Glu Cys Trp Leu His Pro Ala Gln
    290                 295                 300
Tyr Arg Thr Arg Leu Cys Lys Asp Gly Met Gly Cys Asn Arg Arg Val
305                 310                 315                 320
Cys Phe Phe Ala His Ala Asn Glu Glu Leu Arg Pro Leu Tyr Pro Ser
                325                 330                 335
Thr Gly Ser Gly Leu Pro Ser Pro Arg Ala Ser Ser Ala Val Ser Ala
            340                 345                 350
Ser Thr Met Asp Met Ala Ser Val Leu Asn Met Leu Pro Gly Ser Pro
```

```
        355                 360                 365
Ser Ala Ala Gln His Ser Phe Thr Pro Pro Ile Ser Pro Ser Gly Asn
    370                 375                 380
Gly Ser Met Pro His Ser Ser Met Gly Trp Pro Gln Gln Asn Ile Pro
385                 390                 395                 400
Ala Leu Asn Leu Pro Gly Ser Asn Ile Gln Leu Ser Arg Leu Arg Ser
                405                 410                 415
Ser Leu Asn Ala Arg Asp Ile Pro Ser Glu Gln Leu Ser Met Leu His
            420                 425                 430
Glu Phe Glu Met Gln Arg Gln Leu Ala Gly Asp Met His Ser Pro Arg
        435                 440                 445
Phe Met Asn His Ser Ala Arg Pro Lys Thr Leu Asn Pro Ser Asn Leu
    450                 455                 460
Glu Glu Leu Phe Ser Ala Glu Val Ala Ser Pro Arg Phe Ser Asp Gln
465                 470                 475                 480
Leu Ala Val Ser Ser Val Leu Ser Pro Ser His Lys Ser Ala Leu Leu
                485                 490                 495
Asn Gln Leu Gln Asn Asn Lys Gln Ser Met Leu Ser Pro Ile Lys Thr
            500                 505                 510
Asn Leu Met Ser Ser Pro Lys Asn Val Glu Gln His Ser Leu Leu Gln
        515                 520                 525
Gln Ala Ser Ser Pro Arg Gly Gly Glu Pro Ile Ser Pro Met Asn Ala
    530                 535                 540
Arg Met Lys Gln Gln Leu His Ser Arg Ser Leu Ser Ser Arg Asp Phe
545                 550                 555                 560
Gly Ser Ser Leu Pro Arg Asp Leu Met Pro Thr Asp Ser Gly Ser Pro
                565                 570                 575
Leu Ser Pro Trp Ser Ser Trp Asp Gln Thr His Gly Ser Lys Val Asp
            580                 585                 590
Trp Ser Val Gln Ser Asp Glu Leu Gly Arg Leu Arg Lys Ser His Ser
        595                 600                 605
Leu Ala Asn Asn Pro Asn Arg Glu Ala Asp Val Ser Trp Ala Gln Gln
    610                 615                 620
Met Leu Lys Asp Ser Ser Ser Pro Arg Asn Gly Asn Arg Val Val Asn
625                 630                 635                 640
Met Asn Gly Ala Arg Pro Leu Thr Gln Gly Gly Ser Ser Val Asn Pro
                645                 650                 655
His Asn Ser Asp Thr Arg Glu Ser Asp Ile Leu Asp Ala Trp Leu Glu
            660                 665                 670
Gln Leu His Leu Asp Arg
        675
```

<210> 52
<211> 640
<212> PRT
<213> Arabidopsis thaliana

<400> 52

```
Met Gly Leu Trp Tyr Arg Arg Gln Arg Phe Val Arg Arg Met Val Leu
1               5                   10                  15
Glu Gln Arg Thr Pro Leu Met Val Ala Ser Leu Tyr Gly Ser Leu Asp
            20                  25                  30
Val Val Lys Phe Ile Leu Ser Phe Pro Glu Ala Glu Leu Asn Leu Ser
        35                  40                  45
Cys Gly Pro Asp Lys Ser Thr Ala Leu His Cys Ala Ala Ser Gly Ala
    50                  55                  60
Ser Val Asn Ser Leu Asp Val Val Lys Leu Leu Leu Ser Val Gly Ala
65                  70                  75                  80
Asp Pro Asn Ile Pro Asp Ala His Gly Asn Arg Pro Val Asp Val Leu
```

```
                    85                  90                  95
Val Val Ser Pro His Ala Pro Gly Leu Arg Thr Ile Leu Glu Glu Ile
                100                 105                 110
Leu Lys Lys Asp Glu Ile Ile Ser Glu Asp Leu His Ala Ser Ser Ser
            115                 120                 125
Ser Leu Gly Ser Ser Phe Arg Ser Leu Ser Ser Ser Pro Asp Asn Gly
    130                 135                 140
Ser Ser Leu Leu Ser Leu Asp Ser Val Ser Ser Pro Thr Lys Pro His
145                 150                 155                 160
Gly Thr Asp Val Thr Phe Ala Ser Glu Lys Lys Glu Tyr Pro Ile Asp
                165                 170                 175
Pro Ser Leu Pro Asp Ile Lys Ser Gly Ile Tyr Ser Thr Asp Glu Phe
            180                 185                 190
Arg Met Phe Ser Phe Lys Ile Arg Pro Cys Ser Arg Ala Tyr Ser His
        195                 200                 205
Asp Trp Thr Glu Cys Pro Phe Ala His Pro Gly Glu Asn Ala Arg Arg
    210                 215                 220
Arg Asp Pro Arg Lys Phe His Tyr Thr Cys Val Pro Cys Pro Asp Phe
225                 230                 235                 240
Lys Lys Gly Ser Cys Lys Gln Gly Asp Met Cys Glu Tyr Ala His Gly
                245                 250                 255
Val Phe Glu Cys Trp Leu His Pro Ala Gln Tyr Arg Thr Arg Leu Cys
            260                 265                 270
Lys Asp Gly Met Gly Cys Asn Arg Arg Val Cys Phe Phe Ala His Ala
        275                 280                 285
Asn Glu Glu Leu Arg Pro Leu Tyr Pro Ser Thr Gly Ser Gly Leu Pro
    290                 295                 300
Ser Pro Arg Ala Ser Ser Ala Val Ser Ala Ser Thr Met Asp Met Ala
305                 310                 315                 320
Ser Val Leu Asn Met Leu Pro Gly Ser Pro Ser Ala Ala Gln His Ser
                325                 330                 335
Phe Thr Pro Pro Ile Ser Pro Ser Gly Asn Gly Ser Met Pro His Ser
            340                 345                 350
Ser Met Gly Trp Pro Gln Gln Asn Ile Pro Ala Leu Asn Leu Pro Gly
        355                 360                 365
Ser Asn Ile Gln Leu Ser Arg Leu Arg Ser Ser Leu Asn Ala Arg Asp
    370                 375                 380
Ile Pro Ser Glu Gln Leu Ser Met Leu His Glu Phe Glu Met Gln Arg
385                 390                 395                 400
Gln Leu Ala Gly Asp Met His Ser Pro Arg Phe Met Asn His Ser Ala
                405                 410                 415
Arg Pro Lys Thr Leu Asn Pro Ser Asn Leu Glu Glu Leu Phe Ser Ala
            420                 425                 430
Glu Val Ala Ser Pro Arg Phe Ser Asp Gln Leu Ala Val Ser Ser Val
        435                 440                 445
Leu Ser Pro Ser His Lys Ser Ala Leu Leu Asn Gln Leu Gln Asn Asn
    450                 455                 460
Lys Gln Ser Met Leu Ser Pro Ile Lys Thr Asn Leu Met Ser Ser Pro
465                 470                 475                 480
Lys Asn Val Glu Gln His Ser Leu Leu Gln Gln Ala Ser Ser Pro Arg
                485                 490                 495
Gly Gly Glu Pro Ile Ser Pro Met Asn Ala Arg Met Lys Gln Gln Leu
            500                 505                 510
His Ser Arg Ser Leu Ser Ser Arg Asp Phe Gly Ser Ser Leu Pro Arg
        515                 520                 525
Asp Leu Met Pro Thr Asp Ser Gly Ser Pro Leu Ser Pro Trp Ser Ser
    530                 535                 540
Trp Asp Gln Thr His Gly Ser Lys Val Asp Trp Ser Val Gln Ser Asp
545                 550                 555                 560
```

```
Glu Leu Gly Arg Leu Arg Lys Ser His Ser Leu Ala Asn Asn Pro Asn
                565                 570                 575
Arg Glu Ala Asp Val Ser Trp Ala Gln Gln Met Leu Lys Asp Ser Ser
            580                 585                 590
Ser Pro Arg Asn Gly Asn Arg Val Val Asn Met Asn Gly Ala Arg Pro
        595                 600                 605
Leu Thr Gln Gly Gly Ser Ser Val Asn Pro His Asn Ser Asp Thr Arg
    610                 615                 620
Glu Ser Asp Ile Leu Asp Ala Trp Leu Glu Gln Leu His Leu Asp Arg
625                 630                 635                 640
```

<210> 53
<211> 2158
<212> DNA
<213> Oryza sativa

<400> 53

```
tgctccttct tcattgcaaa gaaagcacca ccttttaaag aatcctcctc acactccatt      60
cttcttaaaa aacccaccac aacacaattc ccacttgttt cttcatcatc acctacttca     120
atcaaaaaac attccaactt tcttctcaat ttcattccag gatagtatta tgtgcagtgg     180
accaaagagc aatctctgct cttcaagaac cttaacagaa atcgaatcaa ggcaaaagga     240
agaagaaaca atgcttctcc tcgaattcgc tgcttgtgat gatcttgact cgttcaagag     300
agaggttgaa gagaaagggc ttgatttgga tgagtcaggg ttatggtatt gcagacgtgt     360
cggttctaag aagatgggtc ttgaagaaag aacacccttta atggttgcag ctatgtatgg     420
aagcataaag gttttgactt tcatcgtttc cactggaaaa tctgatgtga acagagcttg     480
tggtgaagag agagttactc cgcttcactg tgctgttgct ggctgttctg tgaatatgat     540
tgaagtcatc aatgtcttgc ttgatgcttc tgctttggtt aactctgttg atgctaatgg     600
gaatcaacct ttggatgtgt ttgttcgagt ttcgaggttt gtggctagtc cgaggaggaa     660
agcggttgag ttgttgctga gaggaggagg tgttggagga ttgatcgatg aggcggttga     720
agaagagatc aagattgtct ctaagtatcc agctgatgct tctttaccgg atataaacga     780
aggggtttat ggaagtgatg agtttaggat gtatagcttt aaggttaagc catgttctag     840
ggcttattct catgattgga ccgagtgtgc ttttgttcat ccgggagaaa atgcgaggag     900
gagagatccg aggaagtatc cttacacttg tgtcccctgt cccgagttcc gtaaaggatc     960
atgcccgaaa ggagattctt gcgagtatgc tcacggggtt ttcgagtcgt ggcttcaccc    1020
cgcgcagtat aaaacccggc tttgtaaaga tgaaacgggt tgtgcaagga aagtttgttt    1080
ctttgctcat aaacgcgaag agatgagacc tgttaatgct tcaactggct ctgccgtggc    1140
tcagtctccg tttagcagct tggagatgat gccagggttg tctcctcttg cttattcttc    1200
aggagtttcg actcctccgg tttctccaat ggctaatggt gttccttcct ctccaagaaa    1260
cggcggatca tggcagaaca gagtcaatac ccttactcca ccggctttgc agctcaatgg    1320
tggaagcaga ttgaagtcca cactgagcgc tagagatatc gatatggaga tggagatgga    1380
attgagactc cgcggttttg gcaacaatgt ggaagagacg ttcgggtctt atgtttcctc    1440
tccaagtagg aattctcaaa tgggtcaaaa catgaaccaa cattatccat cttccccggt    1500
gagacaaccg ccatctcaac acgggttcga atcttcagca gctgcagcgg ttgcagtgat    1560
gaaagcgaga tcaaccgcct ttgcgaaacg tagcttgagc ttcaaaccag ctactcaagc    1620
agcaccacag tcgaatctct cggattgggg atctccaaac gggaagctgg aatggggaat    1680
gaaaggagaa gagctgaata agatgagaag aagtgtttcc tttggaatcc atggaaacaa    1740
caacaataac gcagctagag actacaggga cgagccagat gtgtcatggg ttaactcttt    1800
agttaaagac agtactgtgg tgtctgagag aagctttgga atgaatgaga gggttcggat    1860
aatgtcgtgg gctgagcaaa tgtacagaga gaaggagcag actgtggtgt aaacacacac    1920
aaagatggtt tcttatatat attgcttttg ggccatctct gcaaatttga ttctttaatt    1980
tttgtgactt tctttagttg ttactgttat tagtagtata tggtttgttg tcactacgag    2040
tctacgtgat gaaaagatag aagttaattg cattagtttc tatattcgtt tctcatcctc    2100
ttgtaattta tcaaaccatg aaatggctaa gcaatccaaa ccgaaaaaaa aaaaaaa       2158
```

<210> 54
<211> 2193
<212> DNA
<213> Oryza sativa

<400> 54

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agagggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtacctttttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga atttttttc ccaaatatct taaaaagtca   1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcacccccttc attattctga gctgaaagtc tggcatgaac   1980
tgtcctcaat tttgttttca aattcacatc gattatctat gcattatcct cttgtatcta   2040
cctgtagaag tttcttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                 2193
```

<210> 55

<211> 1827

<212> DNA

<213> Oryza sativa

<400> 55

```
gcttgagtca tagggagaaa acaaatcgat catatttgac tcttttccct ccatctctct     60
taccggcaaa aaaagtagta ctggtttata tgtaaagtaa gattctttaa ttatgtgaga    120
tccggcttaa tgcttttctt ttgtcacata tactgcattg caacaattgc catatattca    180
cttctgccat cccattatat agcaactcaa gaatggattg atatatcccc tattactaat    240
ctagacatgt taaggctgag ttgggcagtc catcttccca acccaccacc ttcgtttttc    300
gcgcacatac ttttcaaact actaaatggt gtgtttttta aaaatatttt caatacaaaa    360
gttgctttaa aaaattatat tgatccattt ttttaaaaaa aatagctaat acttaattaa    420
tcacgtgtta aaagaccgct ccgttttgcg tgcaggaggg ataggttcac atcctgcatt    480
accgaacaca gcctaaatct tgttgtctag attcgtagta ctggatatat taaatcatgt    540
tctaagttac tatatactga gatgaataga ataagtaaaa ttagacccac cttaagtctt    600
gatgaagtta ctactagctg cgtttgggag gacttcccaa aaaaaaaagt attagccatt    660
agcacgtgat taattaagta ctagtttaaa aaacttaaaa aataaattaa tatgattctc    720
ttaagtaact ctcctataga aaacttttac aaaattacac cgtttaatag tttggaaaat    780
atgtcagtaa aaaataagag agtagaagtt atgaaagtta gaaaaagaat tgttttagta    840

gtatacagtt ataaactatt ccctctgttc taaaacataa gggattatgg atggattcga    900
catgtaccag taccatgaat cgaatccaga caagtttttt atgcatattt attctactat    960
aatatatcac atctgctcta aatatcttat atttcgaggt ggagactgtc gctatgtttt   1020
tctgcccgtt gctaagcaca cgccaccccc gatgcgggga cgcctctggc cttcttgcca   1080
cgataattga atggaacttc cacattcaga ttcgataggt gaccgtcgac tccaagtgct   1140
ttgcacaaaa caactccggc ctcccggcca ccagtcacac gactcacggc actaccaccc   1200
ctgactccct gaggcggacc tgccactgtt ctgcatgcga agctatctaa aattctgaag   1260
caaagaaagc acagcacatg ctccgggaca cgcgccaccc ggcggaaaag ggctcggtgt   1320
ggcgatctca cagccgcata tcgcatttca caagccgccc atctccaccg gcttcacgag   1380
gctcatcgcg gcacgaccgc gcacggaacg cacgcggccg acccgcgcgc ctcgatgcgc   1440
gagcccatcc gccgcgtcct ccctttgcct ttgccgctat cctctcggtc gtatcccgtt   1500
tctctgtctt ttgctccccg gcgcgcgcca gttcggagta ccagcgaaac ccggacacct   1560
ggtacacctc cgccggccac aacgcgtgtc cccctacgtg gccgcgcagc acatgcccat   1620
gcgcgacacg tgcacctcct catccaaact ctcaagtctc aacggtccta taaatgcacg   1680
gatagcctca agctgctcgt cacaaggcaa gaggcaagag gcaagagcat ccgtattaac   1740
cagccttttg agacttgaga gtgtgtgtga ctcgatccag cgtagtttca gttcgtgtgt   1800
tggtgagtga ttccagccaa gtttgcg                                       1827
```

<210> 56
<211> 2194
<212> DNA
<213> Oryza sativa

<400> 56

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa cctttttaaca gcaggctttg cggccaggag agaggaggag aggcaaagaa   960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aattttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100

aagctgtaat cgggatagtt atactgcttg ttcttatgat tcatttcctt tgtgcagttc   2160
ttggtgtagc ttgccacttt caccagcaaa gttc                                2194
```